# EUROPEAN PATENT SPECIFICATION

(11) **EP 2 279 004 B1**
(45) Date of publication and mention of the grant of the patent: **14.01.2015**
(21) Application number: 09747765.7
(22) Date of filing: 18.05.2009
(51) Int. Cl.: A61K 39/00, G01N 33/569, G01N 33/68, C07K 16/28

(54) **USE OF BIOMARKERS FOR ASSESSING TREATMENT OF GASTROINTESTINAL INFLAMMATORY DISORDERS WITH BETA7INTEGRIN ANTAGONISTS**
VERWENDUNG VON BIOMARKERN ZUR BEURTEILUNG DER BEHANDLUNG VON ENTZÜNDLICHEN MAGEN-DARM-ERKRANKUNGEN MIT BETA7-INTEGRIN-ANTAGONISTEN
UTILISATION DE BIOMARQUEURS POUR ÉVALUER LE TRAITEMENT DE TROUBLES INFLAMMATOIRES GASTRO-INTESTINAUX AVEC DES ANTAGONISTES DE L'INTÉGRINE BÊTA7

(30) Priority: 16.05.2008 US 54115 P
(43) Date of publication of application: 02.02.2011
(73) Proprietor: F. Hoffmann-La Roche AG, 4070 Basel (CH)
(72) Inventor: GELZLEICHTER, Thomas, Richard, Brisbane, CA 94005 (US); HIRARAGI, Hajime, San Francisco, CA 94107 (US); STEFANICH, Eric, Gary, Emerald Hills, CA 94062 (US); WANG, Hong, San Carlos, CA 94070 (US); WILLIAMS, Marna Bromberg, Palo Alto, CA 94306 (US)
(74) Representative: Heiroth, Ulrike Hildegard
(86) International application number: PCT/US2009/044375
(87) International publication number: WO 2009/140684

(56) References cited:
- WO-A1-96/24673
- WO-A2-2005/067620
- WO-A2-2006/026759
- WO-A2-2008/036600
- JP-A- 2007 302 676
- US-A1- 2001 046 496
- US-A1- 2006 093 601
- OHMAN ET AL: "A Controlled Study of Colonic Immune Activity and beta7<+> Blood T Lymphocytes in Patients With Irritable Bowel Syndrome" CLINICAL GASTROENTEROLOGY AND HEPATOLOGY, AMERICAN GASTROENTEROLOGICAL ASSOCIATION, US LNKD- DOI:10.1016/S1542-3565(05)00410-6, vol. 3, no. 10, 1 October 2005 (2005-10-01), pages 980-986, XP005120665 ISSN: 1542-3565
- MEENAN J ET AL: "Altered expression of alpha 4 beta 7, a gut homing integrin, by circulating and mucosal T cells in colonic mucosal inflammation." February 1997 (1997-02), GUT FEB 1997 LNKD- PUBMED:9071939, VOL. 40, NR. 2, PAGE(S) 241 - 246 , XP002608822 ISSN: 0017-5749 the whole document * abstract; figures 1-4
- HESTERBERG ET AL: "Rapid resolution of chronic colitis in the cotton-top tamarin with an antibody to a gut-homing integrin alpha 4 beta 7" GASTROENTEROLOGY, ELSEVIER, PHILADELPHIA, PA LNKD- DOI:10.1053/GAST.1996.V111.PM8898653, vol. 111, no. 5, 1 November 1996 (1996-11-01), pages 1373-1380, XP005688626 ISSN: 0016-5085
- PICARELLA D ET AL: "MONOCLONAL ANTIBODIES SPECIFIC FOR BETA7 INTEGRIN AND MUCOSAL ADDRESSIN CELL ADHESION MOLECULE-1 (MADCAM-1) REDUCE INFLAMMATION IN THE COLON OF SCID MICE RECONSTITUTED WITH CD45RBHIGH CD4+T CELLS" JOURNAL OF IMMUNOLOGY, AMERICAN ASSOCIATION OF IMMUNOLOGISTS, US, vol. 158, 1 January 1997 (1997-01-01), pages 2099-2106, XP001030596 ISSN: 0022-1767
- FEAGAN B G ET AL: "TREATMENT OF ULCERATIVE COLITIS WITH A HUMANIZED ANTIBODY TO THE ALPHA4BETA7 INTEGRIN" NEW ENGLAND JOURNAL OF MEDICINE, MASSACHUSETTS MEDICAL SOCIETY, BOSTON, MA, US LNKD- DOI:10.1056/NEJMOA042982, vol. 352, no. 24, 1 June 2005 (2005-06-01) , pages 2499-2507, XP009067914 ISSN: 1533-4406
- MCKENZIE B S ET AL: "Targeting lymphocyte Peyer's patch adhesion molecule-1: A relay approach to gut immunization" VACCINE, ELSEVIER LTD, GB LNKD- DOI:10.1016/J.VACCINE.2005.02.016, vol. 23, no. 28, 25 May 2005 (2005-05-25), pages 3668-3678, XP004887026 ISSN: 0264-410X
- FUH F ET AL: "703 Gut-Homing CD4+ Lymphocytes Are Specifically Targeted in Cynomolgus Monkeys Dosed with Anti-Beta7 Antibodies" GASTROENTEROLOGY, ELSEVIER, PHILADELPHIA, PA LNKD- DOI:10.1016/S0016-5085(08)60465-5, vol. 134, no. 4, 1 April 2008 (2008-04-01) , pages A-99, XP023432262 ISSN: 0016-5085 [retrieved on 2008-04-01]
- LUSIJAH ROTT ET AL: 'Expression of Mucosal Homing Receptor a4 b7 by Circulating CD4 1 Cells with Memory for Intestinal Rotavirus', [Online] 05 September 1997, pages 1204 - 1208, XP055034062 Retrieved from the Internet: <URL:http://www.ncbi.nlm.nih.gov/pmc/articl es/PMC508297/pdf/1001204.pdf> [retrieved on 2012-07-27]
- MARNA WILLIAMS ET AL: 'The Memory B Cell Subset Responsible for the Secretory IgA Response and Protective Humoral Immunity to Rotavirus Expresses the Intestinal Homing Receptor, a4b7', [Online] 01 January 1998, pages 4227 - 4235;, XP055034070 Retrieved from the Internet: <URL:http://www.jimmunol.org/content/161/8/ 4227.full.pdf> [retrieved on 2012-07-27]

## Description

The present invention concerns methods of assessing the effect, efficacy, safety, and/or dosing of therapeutic agents (or drug), such as integrin beta7 antagonists, for the treatment of gastrointestinal inflammatory disorders. In particular, the present invention concerns methods of using the level of gut-homing lymphocytes in a patient's peripheral blood, the level of occupancy of a drug on gut-homing lymphocytes, and/or the level of beta7 integrin receptors on gut-homing lymphocytes as indicators ("biomarkers") of the effect, efficacy, safety, and/or dosing of therapeutic agents such as beta7 integrin antagonists for the treatment of gastrointestinal inflammatory disorders. Such methods include, for example, assessing the responsiveness of a patient to treatment of a gastrointestinal disorder with an integrin beta7 antagonist, using one or more of these biomarkers. Additionally, the present invention provides methods of using such biomarkers to design a drug treatment and/or dosing regimen, or for prognosis.

### Background of the Invention

In order to protect the body against foreign substances, cells of the immune system circulate through the body via the peripheral blood and home into lymph nodes and tissues where antigen is concentrated. In general, there are two distinct systems of homing, peripheral homing and mucosal homing, which provide immunity either to systemic or mucosal antigens. Cells initially activated in gastrointestinal sites will home preferentially to mucosal tissues and nodes. In contrast, cells activated in peripheral lymph nodes traffic preferentially to peripheral sites. (Butcher et al., Adv Immunol (1999)) Cells in circulation enter intestinal lymph nodes and tissues by extravasating across high endothelial venules (HEV). This occurs through interactions between adhesion molecules on the surface of lymphocytes and receptors (addressins) on the HEV. (Butcher et al., Adv Immunol (1999))

The integrins are alpha/beta heterodimeric cell surface receptors involved in numerous cellular processes from cell adhesion to gene regulation (Hynes, R. O., Cell, 1992, 69:11-25; and Hemler, M. E., Annu. Rev. Immunol., 1990, 8:365-368). In the immune system, integrins are involved in leukocyte trafficking, adhesion and infiltration during inflammatory processes (Nakajima, H. et al., J. Exp. Med., 1994, 179:1145-1154). Differential expression of integrins regulates the adhesive properties of cells and different integrins are involved in different inflammatory responses. (Butcher, E. C. et al., Science, 1996, 272:60-66). The beta7 containing integrins (*i.e.,* alpha4beta7 and alphaEbeta7) are expressed primarily on monocytes, lymphocytes, eosinophils, basophils, and macrophages but not on neutrophils (Elices, M. J. et al., Cell, 1990, 60:577-584). The primary ligands for alpha4beta7 integrin are the endothelial surface proteins mucosal addressin cell adhesion molecule (MAdCAM-1) and vascular cell adhesion molecule (VCAM-1) (Makarem, R. et al., J. Biol. Chem., 1994, 269:4005-4011). The binding of the alpha4beta7 integrin to MAdCAM-1 and/or VCAM-1 expressed on high endothelial venules (HEVs) at sites of inflammation reportedly results in firm adhesion of the leukocyte to the endothelium; this firm adhesion step is followed by extravasation of the cells into the inflamed tissue (Chuluyan, H. E. et al., Springer Semin. Immunopathol., 1995, 16:391404). A primary ligand for alphaEbeta7 integrin (which is expressed on intra-epithelial lymphocytes (IEL)) is E-cadherein, which reportedly facilitate adherence of the alphaEbeta7-bearing cells to epithelial cells. While E-cadherin reportedly does not play a role in homing into the gut, interactions between E-cadherin and alphaEbeta7 are believed to play a role in tethering lymphocytes to the gut epithelium.

IBD is a group of inflammatory, autoimmune conditions of large intestine, and in some cases, small intestine. The main forms of IBD are Crohn's disease (CD) and ulcerative colitis (UC). As an autoimmune disease, IBD is characterized by increased infiltration of leukocytes into the gastrointestinal tract, which leads to thickening of the intestine and cellular destruction. Inhibiting this infiltration by blocking homing of leukocytes can downmodulate the inflammation and cellular destruction associated with the disease. Circulating mucosal homing lymphocytes are reportedly altered in patients with colonic inflammation (Meenan et al. Gut 1997; 40:241-246). Monoclonal antibodies directed against alpha.4beta.7, MAdCAM-1, or VCAM-1 are reportedly effective modulators in animal models of chronic inflammatory diseases such as colitis (Viney et al., J. Immunol., 1996, 157: 2488-2497) and inflammatory bowel diseases (IBD; Podolsky, D. K., N. Eng. J. Med., 1991, 325:928-937; Powrie, F. et al., Ther. Immunol., 1995, 2:115-123).

Administration of monoclonal antibodies against alphaE beta7 reportedly prevents and ameliorates immunization induced colitis in IL-2 ^{-/-} mice, suggesting that the onset and maintenance of inflammatory bowel disease depends on colonic localization of lamina propria CD4⁺ lymphocytes expressing alpha Ebeta7 (Ludviksson et al., J Immunol. 1999, 162(8):4975-82). An anti-α4 antibody (natalizumab) reportedly has efficacy in treatment of patients with CD (Sandborn et al., N Engl J Med 2005;353:1912-25) and an anti-α4β7 antibody (MLN-02) reportedly is effective in patients with UC (Feagan et al., N Engl J Med 2005;352:2499-507). These findings validate α4β7 as a therapeutic target and support the idea that the interaction between α4β7 and MAdCAM-1 mediates the pathogenesis of IBD. Thus, antagonists of beta7 integrin are of great potential as a therapeutic agent in treating IBD.

US 2001/046496 discloses a monoclonal humanized antibody targeting α4β7, named ActlmAB (LDP-02). In a clinical trial, LDP-02 is administered to patients. Blood samples are analysed for a "α4β7 signal" by measuring the binding of labeled ACT-1 (the murine homologue of LDP-02) to α4β7 in a FACS analysis (see e.g. paragraph [0070]. The "α4β7 signal" is lost after administration of LDP-02 and this is interpreted as saturation of α4β7 binding sites and/or inhibition of α4β7 integrin expression on the surface of circulating lymphocytes (paragraph [0006]).

In order to design therapies with a desired effect, safety, and/or efficacy, it is important to assess a patient's responsiveness and prognosis in response to treatment with a therapeutic agent, such as a beta7 integrin antagonist. Therefore, it is desirable to develop a biomarker that can accurately predict and/or track the responsiveness of a patient to treatment with a therapeutic agent. Such prediction is essential for designing effective treatment regimens for human patients in clinical trial studies and disease treatment.

### Summary of the Invention

The present specification discloses methods of assessing the effect, efficacy, safety, prognosis, and/or dosing of therapeutic agents (or drug), such as integrin beta7 antagonists, for the treatment of a patient having a gastrointestinal inflammatory disorder. In particular, the present specification discloses methods of using the level of gut-homing lymphocytes in the patient's peripheral blood, the level of occupancy of a drug on gut-homing lymphocytes, and/or the level of beta7 integrin receptors on gut-homing lymphocytes as indicators (or biomarkers) of the effect, efficacy, safety, prognosis, and/or dosing of therapeutic agents such as beta7 integrin antagonists for the treatment of gastrointestinal inflammatory disorders. Such methods include, for example, assessing the responsiveness of a patient to treatment of a gastrointestinal disorder with an integrin beta7 antagonist, using one or more of these biomarkers. Additionally, the present specification discloses methods of using such biomarkers to design a drug treatment and/or dosing regimen, or for prognosis.

In some aspects, the level of a biomarker before treatment of the patient with a therapeutic agent is compared to the level of the same biomarker during and/or after treatment of the patient, and a change (*e.g.,* increase or decrease) in the level of this biomarker in the patient is indicative of the effect, efficacy, safety, and/or prognosis of the therapeutic agent, *e.g.,* a beta7 integrin antagonist, for the treatment of a gastrointestinal inflammatory disorder in a patient. Additionally, such methods can be used to design a drug treatment or dosing regimen for treatment of a gastrointestinal inflammatory disorder in a patient.

In one aspect, the specification discloses a method of determining the efficacy of an integrin beta7 antagonist for treatment of a gastrointestinal inflammatory disorder in a patient, the method comprising comparing the amount of a biomarker in a sample obtained from the patient after or during treatment with the integrin beta7 antagonist, to an amount of the biomarker in a sample obtained from the patient before the treatment, wherein a change in the amount of the biomarker after or during the treatment, as compared to before the treatment, is indicative of the efficacy of the antagonist for treatment of the gastrointestinal disorder in the patient.

In another aspect, the specification discloses a method of predicting the responsiveness of a patient having a gastrointestinal inflammatory disorder to treatment with an integrin beta7 antagonist, the method comprising comparing the amount of a biomarker in a sample obtained from the patient after or during treatment with the integrin beta7 antagonist, to the amount of the biomarker in a sample obtained from the patient before the treatment, wherein a change in the amount of the biomarker after or during the treatment, as compared to before the treatment is indicative of the responsiveness of said patient to treatment with said antagonist.

In yet another aspect, the specification discloses a method of determining the dosing of an integrin beta7 antagonist for treatment of a gastrointestinal inflammatory disorder in a patient, the method comprising adjusting the dose of the integrin beta7 antagonist based on a comparison of the amount of a biomarker in a sample obtained from the patient after or during treatment with a dose of the integrin beta7 antagonist, to an amount of the biomarker in a sample obtained from the patient before the treatment, wherein a change in the amount of the biomarker after or during the treatment, as compared to before the treatment, is indicative of the efficacy of or responsiveness to the dose of the integrin beta7 antagonist for treatment of the gastrointestinal disorder in the patient.

In another aspect, the specification discloses a method of determining the dosing regimen of an integrin beta7 antagonist for treatment of a gastrointestinal inflammatory disorder in a patient, the method comprising adjusting the dose regimen of the integrin beta7 antagonist based on a comparison of the amount of a biomarker in a sample obtained from the patient after or during treatment with a dosing regimen of the integrin beta7 antagonist, to an amount of the biomarker in a sample obtained from the patient before the treatment, wherein a change in the amount of the biomarker after or during the treatment, as compared to before the treatment, is indicative of the efficacy of or responsiveness to the dosing regimen of the integrin beta7 antagonist for treatment of the gastrointestinal disorder in the patient.

In one aspect, the change of the amount of biomarker is an increase.

In another aspectt, the change of the amount of biomarker is a decrease.

In yet another aspect, the sample is a peripheral blood sample of the patient.

In one aspect, the amount of biomarker in a blood sample of said patient after receiving the integrin beta7 antagonist is measured within 100 days after receiving a first dose of the integrin beta7 antagonist.

In another aspect, the amount of biomarker in a blood sample of said patient after receiving the integrin beta7 antagonist is measured within about 50 days administering the integrin beta7 antagonist.In yet another aspect, the amount of biomarker in a blood sample of said patient after receiving the integrin beta7 antagonist is measured within about 24 hours after administering the integrin beta7 antagonist.

The present invention provides a method of determining the dosing of an integrin beta7 antagonist for treatment of a gastrointestinal inflammatory disorder in a patient, wherein said integrin beta7 antagonist is an anti-beta7 antibody, the method comprising adjusting the dose of the integrin beta7 antagonist based on a comparison of the amount of a biomarker in a sample obtained from the patient after or during treatment with a dose or dosing regimen of the integrin beta7 antagonist, to an amount of the biomarker in a sample obtained from the patient before the treatment, wherein a change in the amount of the biomarker after or during the treatment, as compared to before the treatment, is indicative of the efficacy of or responsiveness to the dose or dosing regimen of the integrin beta7 antagonist for treatment of the gastrointestinal disorder in the patient, and wherein the biomarker is selected from a group consisting of gut-homing lymphocytes in the patient's peripheral blood, integrin beta7 antagonist occupancy on gut-homing lymphocytes, and beta7 integrin receptors on gut-homing lymphocytes, wherein the gut-homing lymphocytes are a distinctive subgroup of lymphocytes identified as CD45RA⁻β7^{high}CD4⁺.

In one embodiment, the gastrointestinal inflammatory disorder is an inflammatory bowel disease.

In a further embodiment, the inflammatory bowel disease is Crohn's disease (CD)" or "ulcerative colitis (UC).

In another embodiment, said patient is a human.

In one aspect, said changed amount is at least about 30%, about 50%, about one fold, or about three fold increase in the amount of said biomarkers.

In one aspect, the anti-beta7 integrin antibody is administered in an amount of about 1 mg/kg to 100 mg/kg.

In a further aspect, said amount is 5mg/kg to 50mg//kg.

In one embodiment, the treatment further comprises administering an effective amount of one or more further medicaments.

In a further aspect, the further medicament is an immunosuppressive agent, a pain-control agent, an antidiarrheal agent, an antibiotic, or a combination of one or more of said agents.

In yet a further aspect, said immunosuppressive agent is sulfasalazine, 5-aminosalisylic acid (5-ASA), Metroidazole, ciprofloxacin, Azathioprine or 6-mercaptopurine.

In another aspect, said further medication is another anti-beta7 integrin antibody.

In one aspect, the anti-beta7 integrin antibody is administered into said patient parentally.

In a further aspect, the anti-beta7 integrin antibody is administered into said patient intravenously and/or subcutaneously.

In one aspect, said anti-beta7 integrin antibody is administered at a frequency of at least once every 1, 2, 3, 4, 5, 6, 8, or 12 weeks.

In a further aspect, said anti-beta7 integrin antibody is administered weekly.

In another aspect, said anti-beta7 antibody is administered for at least 1, 2, 4, 6, 8, 12, 24, 36, or 52 weeks.

In one embodiment, antibody is monoclonal.

In another embodiment, said antibody is a chimeric, human or humanized antibody.

In another embodiment, said antibody is an antibody fragment.

In one aspect, said antibody inhibits the interaction of a human beta7 integrin subunit with a second integrin subunit and/or an integrin ligand.

In one aspect, the second integrin subunit is an alpha4 integrin subunit, and wherein the ligand is MAdCAM, VCAM or fibronectin.

In one aspect, the alpha4 integrin subunit is from a human.

In one aspect, the second integrin subunit is alphaE integrin subunit, and wherein the ligand is E-cadherein.

In one aspect, the ligand is from a human.

In one aspect, the alphaE integrin subunit is from human.

In another aspect, the anti-beta7 integrin antibody comprises three heavy chain hypervariable region (HVR-H1-H3) sequences and three light chain hypervariable region (HVR-L1-L3) sequences, wherein-one, two, three, four, five or six hypervariable region (HVR) are selected from the group consisting of:
(i) HVR-L1 comprising sequence A1-A11, wherein A1-A11 is RASESVDTYLH (SEQ ID NO: 1);
(ii) HVR-L2 comprising sequence B1-B8, wherein B1-B8 is KYASQSIS (SEQ ID NO: 2);
(iii) HVR-L3 comprising sequence C1-C9, wherein C1-C9 is QQGNSLPNT (SEQ ID NO: 3);
(iv) HVR-H1 comprising sequence D1-D10, wherein D1-D10 is GFFITNNYWG (SEQ ID NO: 4);
(v) HVR-H2 comprising sequence E1-E17, wherein E1-E17 is GYISYSGSTSYNPSLKS (SEQ ID NO: 5); and
(vi) HVR-H3 comprising sequence F1-F11, wherein F1-F11 is MTGSSGYFDF (SEQ ID NO: 6).

In yet another embodiment, the antibody comprises six hypervariable regions (HVRs) selected from the group consisting of HVR-L1, HVR-L2, HVR-L3, HVR-H1, HVR-H2, and HVR-H3, wherein:
(i) HVR-L1 comprises amino acid sequence A1-A11, wherein A1-A11 is RASESVDTYLH (SEQ ID NO: 1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21), where Xaa represents any amino acid, or a variant of SEQ ID NOs:2, 20 or 21 wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 wherein amino acid C8 is selected from the group consisting of N, V, W, Y, R, S, T, A, F, H, I L, M, and Y;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E 17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 wherein amino acid F2 is R, M, A, E, G, Q, S, and/or amino acid F11 is selected from the group consisting of F and Y.

In another aspect, the antibody further comprises a framework, wherein the amino acid at framework position 71 is R or A, and the amino acid at framework position 73 is N or T, and the amino acid at framework position 78 is F or A or L.

In another aspect, the antibody further comprises a heavy chain human subgroup III heavy chain consensus framework sequence comprising a substitution at position 71, 73 and/or 78.

In one aspect, the substitution is R71A, N73T, ,L78A or L78F.

In another aspect, a framework sequence between sequence HVR-H2 positions E1-E1 and HVR-H3 positions F1-F11 is HFR3-1-HFR3-31 and wherein HFR3-6 is A or R, HFR3-8 is N or T, and HFR3-13 is L or A or F.

In yet another aspect, heavy chain framework position 71 comprises amino acid R or A, and/or heavy chain framework position 73 comprises T or N, and/or heavy chain framework position 78 comprises F or A or L. sequence comprises at heavy chain framework position 71.

In an exemplary embodiment, the antibody comprises three heavy chain hypervariable region (HVR-H1-H3) sequences and three light chain hypervariable region (HVR-L1-L3) sequences, wherein:
(i) HVR-L1 comprises SEQ ID NO: 7, SEQ ID NO:8 or SEQ ID NO:9;
(ii) HVR-L2 comprises SEQ ID NO: 2;
(iii) HVR-L3 comprises SEQ ID NO:3;
(iv) HVR-H1 comprises SEQ ID NO:4;
(v) HVR-H2 comprises SEQ ID NO:5; and
(vi) HVR-H3 comprises SEQ ID NO:6 or SEQ ID NO:16 or SEQ ID NO:17 or SEQ ID NO:19.

In yet another exemplary aspect, said humanized antibody comprises a light chain variable region sequence of SEQ ID NO:24, and a heavy chain variable region sequence of SEQ ID NO:25.

In one aspect, said antibody bind to the same epitope of an antibody comprising light chain and heavy chain variable sequences SEQ ID NO:10 andSEQ ID NO:11, respectively.

In one aspect, the specification discloses a method of designing a treatment with a candidate agent for a human patient diagnosed with a gastrointestinal inflammatory disorder, comprising determining an effective dosage for the human patient based on a dosage that effectively increases the amount of a biomarker in peripheral blood of a non-human subject in response to a treatment with said candidate agent.

In one aspect, said non-human subject is a monkey.

In another aspect, the specification discloses a method predicting prognosis of an inflammatory bowel disease for a patient comprising comparing a ratio between amount of gut-homing lymphocytes and amount of peripheral-homing lymphocytes in a blood sample of said patient with a ratio between amount of gut-homing lymphocytes and amount of peripheral homing lymphocytes in a blood sample of a healthy individual, wherein a decreased ratio of said patient as compared to that of the healthy individual is indicative of the prognosis of the disease.

In another aspect, the specification discloses a method of identifying a population of lymphocytes comprising lymphocytes expressing alphaEbeta7 integrin and lymphocytes expressing alpha4beta7 integrin, comprising binding said lymphocytes with an isolated antibody that bind to the same epitope of an antibody comprising a light chain variable region sequence of SEQ ID NO:10, and a heavy chain variable region sequence of SEQ ID NO:11.

In one embodiment, said lymphocytes are in peripheral blood of a patient diagnosed with an inflammatory bowel disease.

In another aspect, said lymphocytes are in lymph node and tissues of the intestine of a patient diagnosed with an inflammatory bowel disease.

### Brief Description of the Drawings

Figure 1 shows CD4 + lymphocyte subsets in cynomolgus monkey peripheral blood.
Figure 2 shows Group Mean (± SD) CD45RA - beta7high CD4 + lymphocytes in peripheral blood following 12 intravenous doses of 5, 15, or 50 mg/kg rhuMAb Beta7 or 12 intravenous and subcutaneous doses of vehicle to cynomolgus monkeys (Absolute Counts, % BL).
Figure 3 shows Group Mean (± SD) CD45RA - beta7high CD4+ lymphocytes in peripheral blood following 12 subcutaneous doses of 15 or 50 mg/kg rhuMAb Beta7 or 12 intravenous and subcutaneous doses of vehicle to cynomolgus monkeys (Absolute Counts, % BL).
Figure 4 shows Group Mean (± SD) CD45RA- beta7low CD4+ lymphocytes in peripheral blood following 12 intravenous doses of 5, 15, or 50 mg/kg rhuMAb Beta7 or 12 intravenous and subcutaneous doses of vehicle to cynomolgus monkeys (Absolute Counts, % BL).
Figure 5 shows Group Mean (± SD) CD45RA- beta7low CD4+ lymphocytes in peripheral blood following 12 subcutaneous doses of 15 or 50 mg/kg rhuMAb Beta7 or 12 intravenous and subcutaneous doses of vehicle to cynomolgus monkeys (Absolute Counts, % BL).
Figure 6 shows Group Mean (± SD) CD45RA + beta7intermediate CD4 + Lymphocytes in peripheral blood following 12 intravenous doses of 5, 15, or 50 mg/kg rhuMAb Beta7 or 12 intravenous and subcutaneous doses of vehicle to cynomolgus monkeys (Absolute Counts, % BL).
Figure 7 shows Group Mean (± SD) CD45RA + beta7intermediate CD4 + lymphocytes in peripheral blood following 12 subcutaneous doses of 15 or 50 mg/kg rhuMAb Beta7 or 12 intravenous and subcutaneous doses of vehicle to cynomolgus monkeys (Absolute Counts, % BL).
Figure 8 shows transient increase in peripheral blood memory/effector 'Gut Homing' CD45RA- beta7 hi CD4+ Lymphocyte counts following administration of rhuMAb Beta7 -- 4 weekly IV doses of 25 mg/kg rhuMAb Beta7 or vehicle (Absolute Counts, %BL).

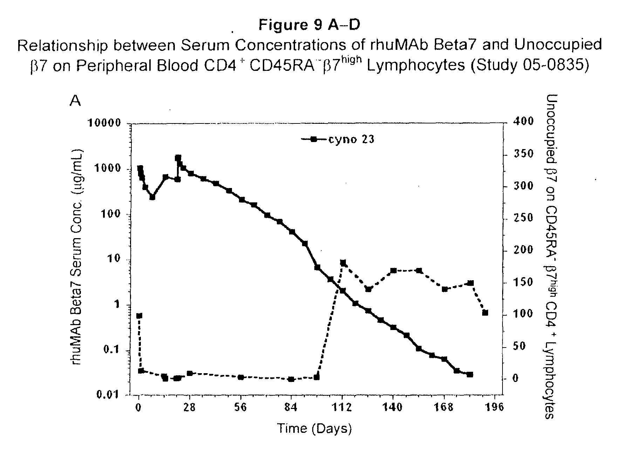

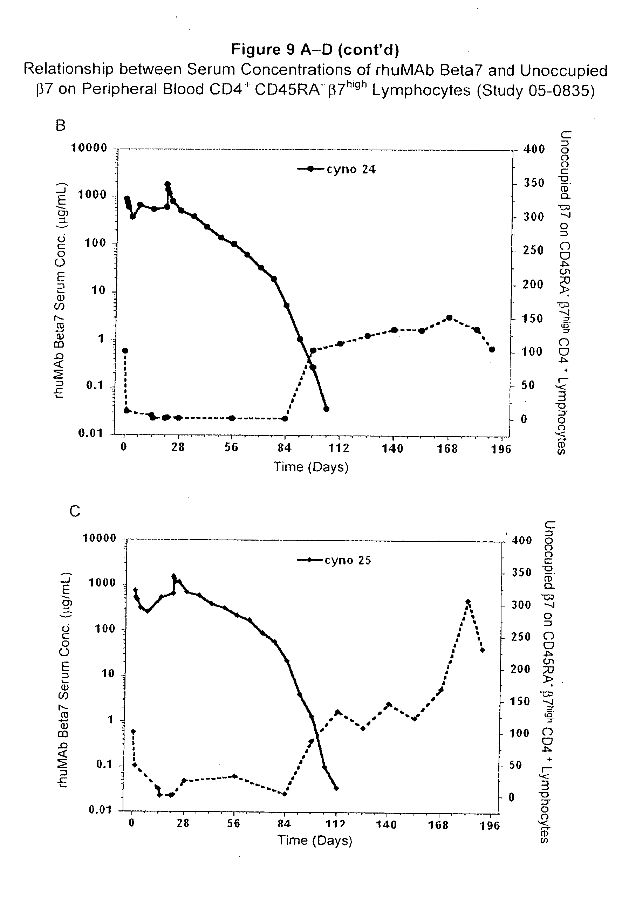

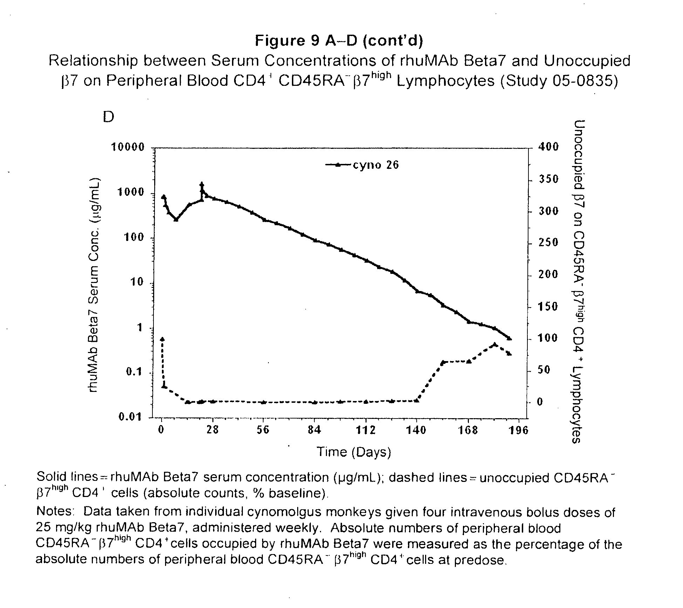

Figure 9 (A-D) shows serum rhuMAb Beta7 Concentration (micrograms/microliter) and occupancy of β7 receptors on CD45RA- beta7hi Memory/Effector CD45RA- CD4+ peripheral blood lymphocytes.

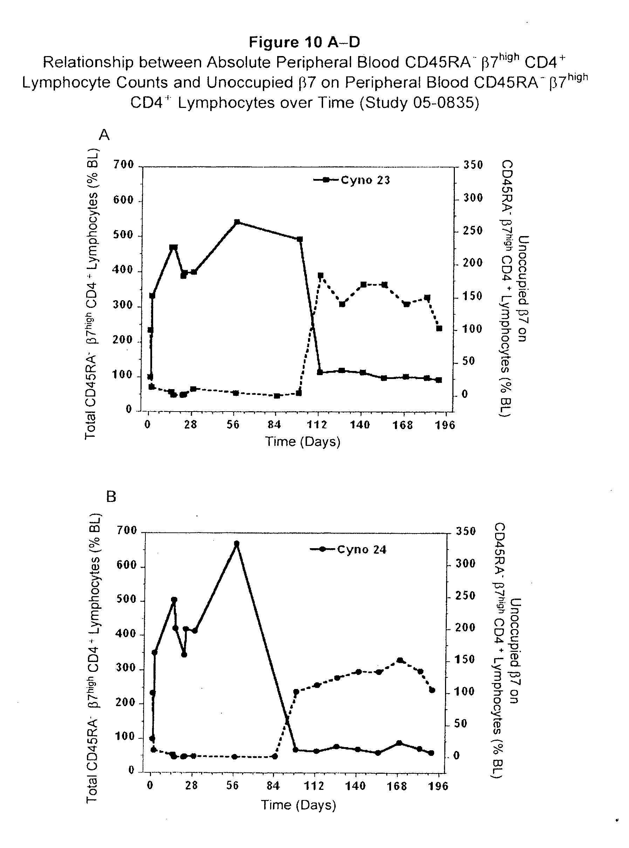

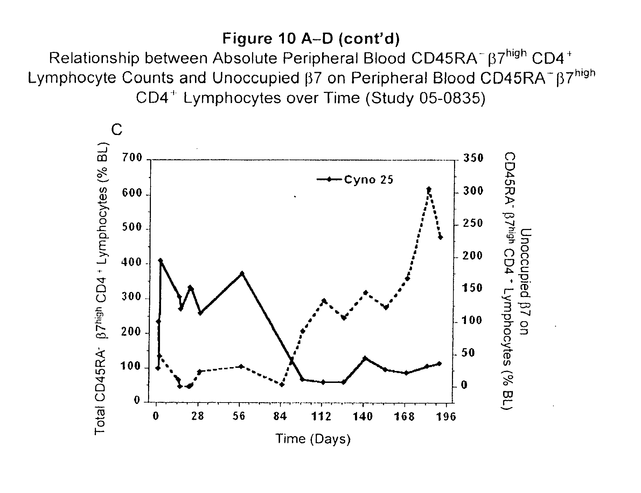

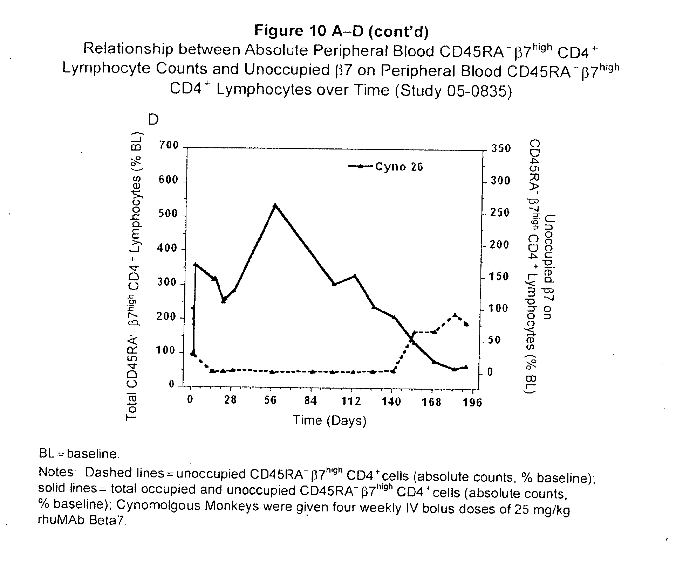

Figure 10(A-D) shows transient increase in 'Gut-Homing' CD45RA- β7hi Memory/Effector CD4+ lymphocytes in peripheral blood correlates with occupancy of β7 Receptor on CD45RA- β7hi Memory/Effector CD4+ lymphocytes.
Figure 11 shows rhuMAb Beta7 inhibits *In vivo* homing of lymphocytes to the inflamed colon but not the spleen of CD45RBhigh-Reconstituted SCID mice (mouse colitis model).

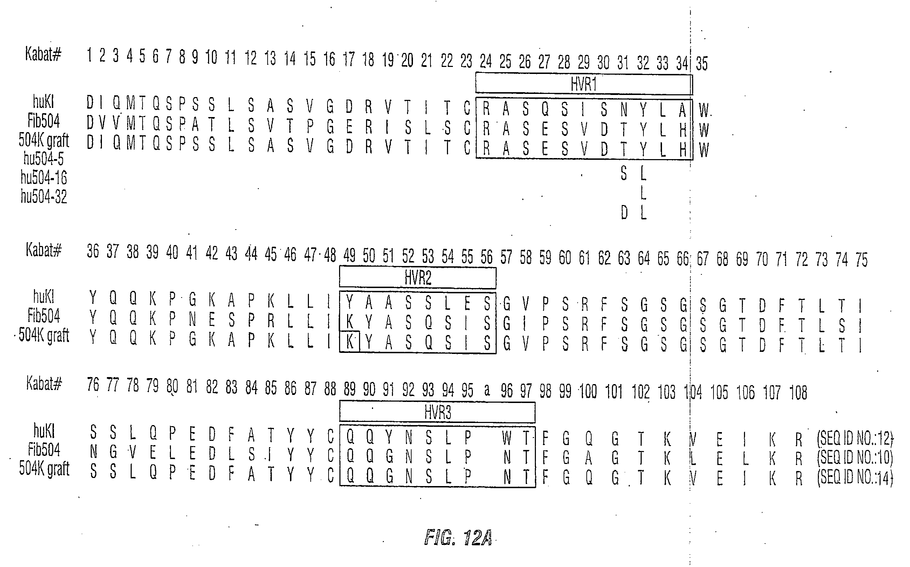

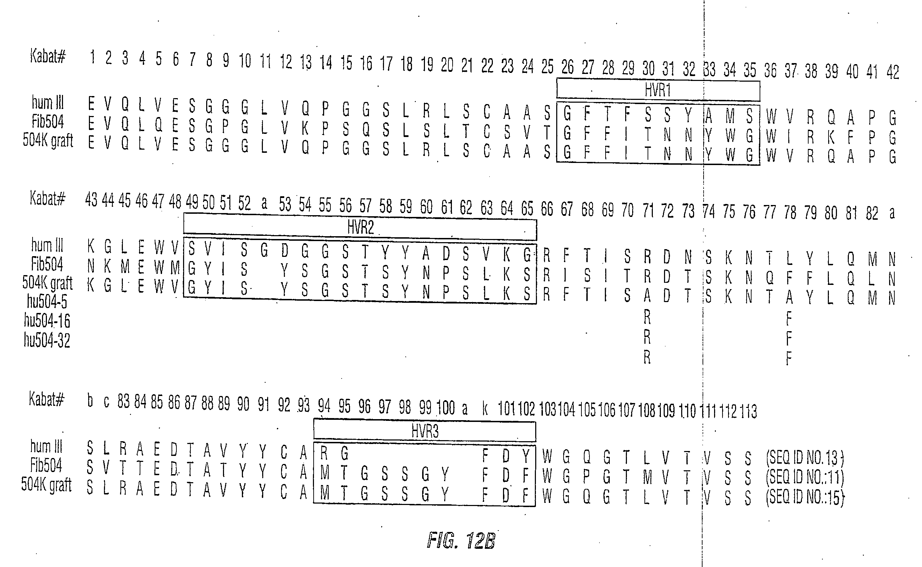

Figure. 12A and 12B shows alignment of sequences of the variable light and heavy chains for the following: light chain human subgroup kappa I consensus sequence (FIG. 12A, SEQ ID NO:12), heavy chain human subgroup III consensus sequence (FIG. 12B, SEQ ID NO:13), rat anti-mouse beta7 antibody (Fib504) variable light chain (FIG. 12A, SEQ ID NO:10), rat anti-mouse beta7 antibody (Fib504) variable heavy chain (FIG. 12B, SEQ ID NO:11),and humanized antibody variants: Humanized hu504Kgraft variable light chain (FIG. 12A, SEQ ID NO:14), humanized hu504K graft variable heavy chain (FIG. 12B, SEQ ID NO:15), variants hu504-5, hu504-16, and hu504-32 (amino acid variations from humanized hu504K graft are indicated in FIG. 12A) (light chain) (SEQ ID NOS:22-24, respectively, in order of appearance) and FIG. 12B (heavy chain) for variants hu504-5, hu504-16, and hu504-32 (SEQ ID NO:25).
Figure 13 shows CD45RA- Beta7hi Memory/Effector CD4+ Lymphocytes (Absolute Counts, %BL) in cynomolgus monkeys following 12 intravenous or subcutaneous doses of vehicle.
Figure 14 shows CD45RA- Beta7hi Memory/Effector CD4+ Lymphocytes (Absolute Counts, %BL) in cynomolgus monkeys following 12 intravenous doses of 5 mg/kg rhuMAb Beta7.
Figure 15 shows CD45RA- Beta7hi Memory/Effector CD4+ Lymphocytes (Absolute Counts, %BL) in cynomolgus monkeys following 12 intravenous doses of 15 mg/kg rhuMAb Beta7.
Figure 16 shows CD45RA- Beta7hi Memory/Effector CD4+ Lymphocytes (Absolute Counts, %BL) in cynomolgus monkeys following 12 intravenous doses of 50 mg/kg rhuMAb Beta7.
Figure 17 shows CD45RA- Beta7hi Memory/Effector CD4+ Lymphocytes (Absolute Counts, %BL) in cynomolgus monkeys following 12 subcutaneous doses of 15 mg/kg rhuMAb Beta7.
Figure 18 shows CD45RA- Beta7hi Memory/Effector CD4+ Lymphocytes (Absolute Counts, %BL) in cynomolgus monkeys following 12 subcutaneous doses of 50 mg/kg rhuMAb Beta7.

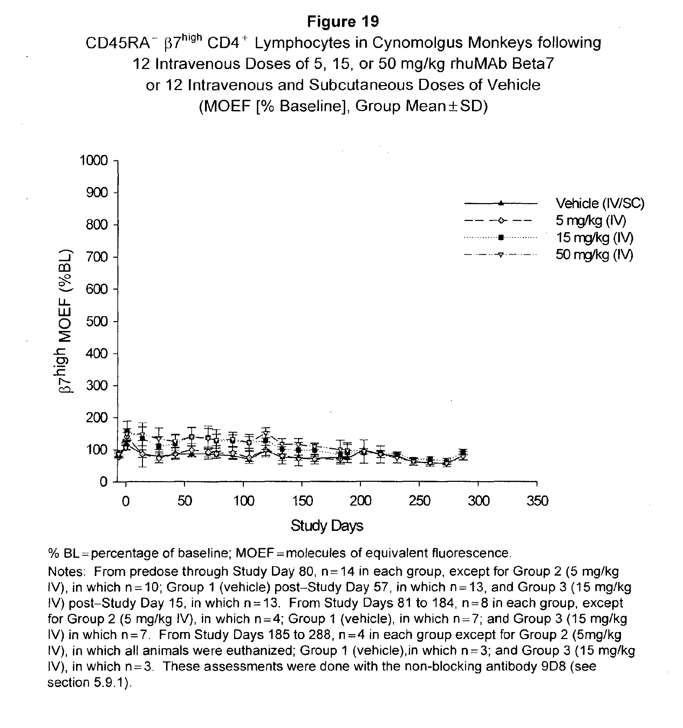

Figure 19 shows CD45RA⁻ β7^{high} CD4⁺ lymphocytes in cynomolgus monkeys following 12 intravenous doses of 5, 15, or 50 mg/kg rhuMAb Beta7 or 12 intravenous and subcutaneous doses of vehicle (MOEF [% Baseline], Group Mean ± SD); MOEF = molecules of equivalent fluorescence.

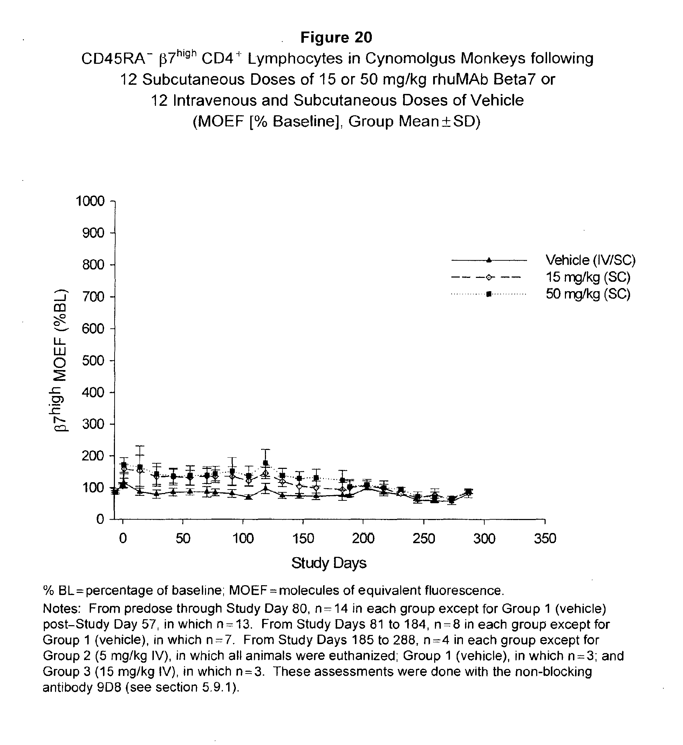

Figure 20 shows CD45RA⁻ β7^{high} CD4 ⁺ lymphocytes in cynomolgus monkeys following 12 subcutaneous doses of 15 or 50 mg/kg rhuMAb Beta7 or 12 intravenous and subcutaneous doses of vehicle (MOEF [% Baseline], Group Mean ± SD) MOEF = molecules of equivalent fluorescence.

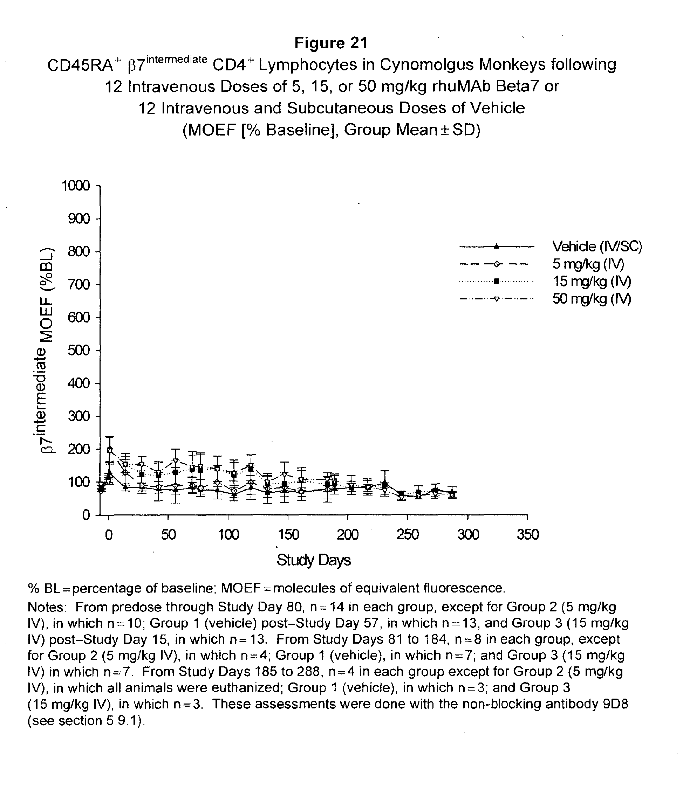

Figure 21 shows CD45RA⁺ β7^{intermediate} CD4⁺ Lymphocytes in cynomolgus monkeys following 12 intravenous doses of 5, 15, or 50 mg/kg rhuMAb Beta7 or 12 intravenous and subcutaneous doses of vehicle (MOEF [% Baseline], Group Mean ± SD); MOEF = molecules of equivalent fluorescence.

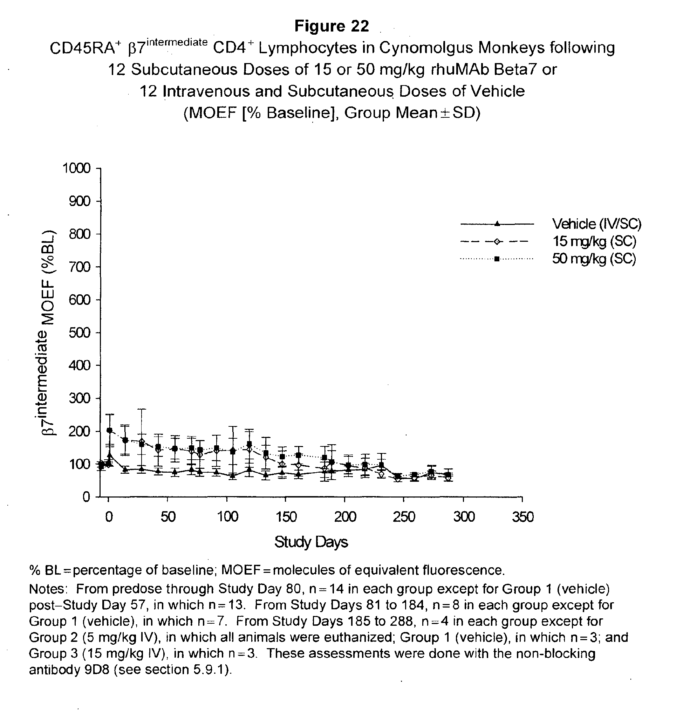

Figure 22 shows CD45RA⁺ β7^{intermediate} CD4⁺ Lymphocytes in cynomolgus monkeys following 12 subcutaneous doses of 15 or 50 mg/kg rhuMAb Beta7 or 12 intravenous and subcutaneous doses of vehicle (MOEF [% Baseline], Group Mean ± SD); MOEF = molecules of equivalent fluorescence.
Figure 23 shows mean (± SD) serum concentrations of rhuMAb β7 following four weekly IV bolus doses of 5 or 25 mg/kg rhuMAb β7 to cynomolgus monkeys.
Figure 24 shows actual mean (± SD) serum concentrations from 4 recovery animals and model-predicted serum concentrations of rhuMAb β7 following four weekly IV bolus doses of 25 mg/kg rhuMAb β7 to cynomolgus monkeys.
Figure 25 shows CD4 T-cell subsets in cynomolgus monkey peripheral blood.
Figure 26 shows mean (±SD) absolute peripheral blood CD45RA⁻β7^{high} CD4⁺ T-cell counts (expressed as percentage of predose baseline) following four weekly IV bolus doses of vehicle or 5 or 25 mg/kg rhuMAb β7 to cynomolgus monkeys.
Figure 27 shows mean (±SD) absolute peripheral blood CD45RA⁺β7^{intermediate} CD4⁺ T-cell counts (expressed as percentage of predose baseline) following four weekly IV bolus doses of vehicle or 5 or 25 mg/kg rhuMAb β7 to cynomolgus monkeys.
Figure 28 shows Individual absolute peripheral blood CD45RA⁻β7^{high} CD4⁺ T-cell counts (expressed as percentage of predose baseline) following four weekly IV bolus doses of vehicle or 25 mg/kg rhuMAb β7 to cynomolgus monkeys.
Figure 29 shows mean (±SD) absolute peripheral blood lymphocyte counts (expressed as percentage of predose baseline) following four weekly IV bolus doses of vehicle, 5 or 25 mg/kg rhuMAb β7 to cynomolgus monkeys.
Figure 30 shows mean (±SD) absolute peripheral blood CD3⁺ T-cell counts (expressed as percentage of predose baseline) following four weekly IV bolus doses of 5 or 25 mg/kg rhuMAb β7 to each group of cynomolgus monkeys.
Figure 31 shows mean (±SD) absolute peripheral blood CD20⁺ B-cell counts (expressed as percentage of predose baseline) following four weekly IV bolus doses of 5 or 25 mg/kg rhuMAb β7 to each group of cynomolgus monkeys.
Figure 32A shows the relationship between serum concentrations of rhuMAb β7 and unoccupied peripheral-blood CD4⁺ β7^{high} CD45RA⁻ T cells over time in Individual cynomolgus monkeys following four IV bolus doses of 25 mg/kg rhuMAb β7 administered weekly (Animal 23).
Figure 32B shows the relationship between serum concentrations of rhuMAb β7 and unoccupied peripheral-blood CD4⁺ β7^{high} CD45RA⁻ T cells over time in individual cynomolgus monkeys following four IV bolus doses of 25 mg/kg rhuMAb β7 administered weekly (Animal 24).
Figure 32C shows the relationship between serum concentrations of rhuMAb β7 and unoccupied peripheral-blood CD4⁺ β7^{high} CD45RA⁻ T cells over time in individual cynomolgus monkeys following four IV bolus doses of 25 mg/kg rhuMAb β7 administered weekly (Animal 25).
Figure 32D shows the relationship between serum concentrations of rhuMAb β7 and unoccupied peripheral-blood CD4⁺ β7^{high} CD45RA⁻ T cells over time in individual cynomolgus monkeys following four IV bolus doses of 25 mg/kg rhuMAb β7 administered weekly (Animal 26).
Figure 33 shows the relationship between serum concentrations of rhuMAb β7 and unoccupied peripheral-blood CD4⁺ β7^{high} CD45RA⁻ T cells over time in individual cynomolgus monkeys following four IV bolus doses of 25 mg/kg rhuMAb β7 administered weekly.
Figure 34A shows the relationship between absolute β7^{high}-expressing memory/effector (CD45RA⁻) CD4⁺ T-cells and unoccupied peripheral-blood CD4⁺ β7^{high} CD45RA⁻ T-cells over time in individual cynomolgus monkeys following four weekly IV bolus doses of 25 mg/kg rhuMAb β7 (Animal 23).
Figure 34B shows the relationship between absolute β7^{high}-expressing memory/effector (CD45RA⁻) CD4⁺ T-cells and unoccupied peripheral-blood CD4⁺ β7^{high} CD45RA- T-cells over time in individual cynomolgus monkeys following four weekly IV bolus doses of 25 mg/kg rhuMAb β7 (Animal 24).
Figure 34C shows the relationship between absolute β7^{high}-expressing memory/effector (CD45RA⁻) CD4⁺ T-cells and unoccupied peripheral-blood CD4⁺ β7^{high} CD45RA⁻ T-cells over time in individual cynomolgus monkeys following four weekly IV bolus doses of 25 mg/kg rhuMAb β7 (Animal 25).
Figure 34D shows the relationship between absolute β7^{high}-expressing memory/effector (CD45RA⁻) CD4⁺ T-cells and unoccupied peripheral-blood CD4⁺ β7^{high} CD45RA- T-cells over time in individual cynomolgus monkeys following four weekly IV bolus doses of 25 mg/kg rhuMAb β7 (Animal 26).
Figure 35 shows mean absolute peripheral-blood CD4⁺ β7^{high} CD45RA⁻ T cell counts.
Figure 36 shows mean absolute peripheral-blood CD8⁺ β7^{high} CD45RA- T cell counts.
Figure 37 shows mean absolute peripheral-blood CD4⁺ β7^{intermediate} CD45RA⁻ T cell counts.
Figure 38 shows mean absolute peripheral-blood CD8⁺ β7^{intermediate} CD45RA⁻ T cell counts.
Figure 39 shows relationship between serum concentrations of rhuMab β7 and available β7-expressing peripheral-blood CD4⁺ β7^{high} CD45RA⁻ T cells.
Figure 40 shows relationship between serum concentrations of rhuMab β7 and available β7-expressing peripheral-blood CD8⁺ β7^{high} CD45RA- T cells.

### Detailed Description of the Preferred Embodiment

### I. Definitions

As used herein, with respect to patient response or patient responsiveness, the term "prediction" or "predicting" is used herein to refer to the likelihood that a patient will respond either favorably or unfavorably to a drug or set of drugs. In one aspect, the prediction relates to the extent of those responses. In one aspect, the prediction relates to whether and/or the probability that a patient will survive or improve following treatment, for example treatment with a particular therapeutic agent, for instance, anti-beta7 integrin antibody, and for a certain period of time without disease recurrence. For example, the predictive methods disclosed can be used clinically to make treatment decisions by choosing the most appropriate treatment modalities for any particular patient. The predictive methods disclosed are valuable tools in predicting whether a patient is likely to respond favorably to a treatment regimen, including for example, administration of a given therapeutic agent or combination, surgical intervention, steroid treatment, etc., or whether long-term survival of the patient, following a therapeutic regimen is likely.

"Treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis.

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Modifying a treatment" refers to changing the treatment regimen including, changing dosage, frequency of administration, or duration of treatment, and/or addition of a second medication.

"Patient response" or "patient responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (*i.e.,* reduction, slowing down or complete stopping) of disease cell infiltration into adjacent peripheral organs and/or tissues; (5) inhibition (*i.e.,* reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment. The term "responsiveness" refers to a measurable response, including complete response (CR) and partial response (PR).

By "complete response" or "CR" is intended the disappearance of all signs of inflammation or remission in response to treatment. This does not always mean the disease has been cured.

"Partial response" or "PR" refers to a decrease of at least 50% in the severity of inflammation, in response to treatment.

An "beneficial response" of a patient to treatment with an integrin beta7 antagonist and similar wording refers to the clinical or therapeutic benefit imparted to a patient at risk for or suffering from a gastrointestinal inflammatory disorder from or as a result of the treatment with the antagonist, such as an anti-beta7 integrin antibody. Such benefit includes cellular or biological responses, a complete response, a partial response, a stable disease (without progression or relapse), or a response with a later relapse of the patient from or as a result of the treatment with the antagonist.

"A patient maintains responsiveness to a treatment" when the patient' responsiveness does not decrease with time during the course of a treatment.

The term "diagnosis" is used herein to refer to the identification or classification of a molecular or pathological state, disease or condition. For example, "diagnosis" may refer to identification of a particular type of gastrointestinal inflammatory disorder, and more particularly, the classification of a particular sub-type of gastrointestinal inflammatory disorder, by tissue/organ involvement (*e.g.,* inflammatory bowel disease), or by other features (*e.g.,* a patient subpopulation characterized by responsiveness to a treatment, such as to a treatment with an integrin beta7 antagonist).

The term "prognosis" is used herein to refer to the prediction of the likelihood of disease symptoms, including, for example, recurrence, flaring, and drug resistance, of a gastrointestinal inflammatory disorder.

The term "sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. The sample can be obtained from a tissue for the subject of interest or from peripheral blood of the subject.

"A beta7 integrin antagonist" or "beta7 antagonist" refers to any molecule that inhibits one or more biological activities or blocking binding of beta7 integrin with one or more of its associated molecules. Antagonists of the invention can be used to modulate one or more aspects of beta7 associated effects, including but not limited to association with alpha4 integrin subunit, association with alphaE integrin subunit, binding of alpha4beta7 integrin to MAdCAM, VCAM-1 or fibronectin and binding of alphaEbeta7 integrin to E-cadherin. These effects can be modulated by any biologically relevant mechanism, including disruption of ligand binding to beta7 subunit or to the alpha4beta7 or alphaEbeta dimeric integrin, and/or by disrupting association between the alpha and beta integrin subunits such that formation of the dimeric integrin is inhibited. In one aspect, the beta7 antagonist is an anti-beta7 integrin antibody (or anti-beta7 antibody). In one embodiment, the anti-beta7 integrin antibody is a humanized anti-beta7 integrin antibody and more particularly a recombinant humanized monoclonal anti-beta7 antibody (or rhuMAb beta7). In some embodiments, the anti-beta7 antibodies of the present invention are anti-integrin beta7 antagonistic antibodies that inhibit or block the binding of beta7 subunit with alpha4 integrin subunit, association with alphaE integrin subunit, binding of alpha4beta7 integrin to MAdCAM, VCAM-1 or fibronectin and binding of alphaEbeta7 integrin to E-cadherin.

By "beta7 subunit" or ".beta. 7 subunit" is meant the human .beta.7 integrin subunit (Erle et al., (1991) J. Biol. Chem. 266:11009-11016). The beta7 subunit associates with alpha4 integrin subunit, such as the human .alpha.4 subunit (Kilger and Holzmann (1995) J. Mol. Biol. 73:347-354). The alpha4beta7 integrin is reportedly expressed on a majority of mature lymphocytes, as well as a small population of thymocytes, bone marrow cells and mast cells. (Kilshaw and Murant (1991) Eur. J. Immunol. 21:2591-2597; Gurish *et al.,* (1992) 149: 1964-1972; and Shaw, S. K. and Brenner, M. B. (1995) Semin. Immunol. 7:335). The beta7 subunit also associates with the alphaE subunit, such as the human alphaE integrin subunit (Cepek, K. L, et al. (1993) J. Immunol. 150:3459). The alphaEbeta7 integrin is expressed on intra-intestinal epithelial lymphocytes (iIELs) (Cepek, K. L. (1993) *supra*).

By "alphaE subunit" or "alphaE integrin subunit" or ".alpha.E subunit" or ".alpha.E integrin subunit" or "CD103" is meant an integrin subunit found to be associated with beta7 integrin on intra-epithelial lymphocytes, which alphaEbeta7 integrin mediates binding of the iELs to intestinal epithelium expressing E-cadherin (Cepek, K. L. et al. (1993) J. Immunol. 150:3459; Shaw, S. K. and Brenner, M. B. (1995) Semin. Immunol. 7:335).

"MAdCAM" or "MAdCAM-1" are used interchangeably in the context of the present invention and refer to the protein mucosal addressin cell adhesion molecule-1, which is a single chain polypeptide comprising a short cytoplasmic tail, a transmembrane region and an extracellular sequence composed of three immunoglobulin-like domains. The cDNAs for murine, human and macaque MAdCAM-1 have been cloned (Briskin, et al., (1993) Nature, 363:461-464; Shyjan et al., (1996) J. Immunol. 156:2851-2857).

"VCAM-1" or "vascular cell adhesion molecule-1" "CD106" refers to a ligand of alpha4beta7 and alpha4beta1, expressed on activated endothelium and important in endothelial-leukocyte interactions such as binding and transmigration of leukocytes during inflammation.

"CD45" refers to a protein of the protein tyrosine phosphatase (PTP) family. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. This gene is specifically expressed in hematopoietic cells. This PTP has been shown to be an essential regulator of T-and B-cell antigen receptor signaling. It functions through either direct interaction with components of the antigen receptor complexes, or by activating various Src family kinases required for the antigen receptor signaling. This PTP also suppresses JAK kinases, and thus functions as a regulator of cytokine receptor signaling. Four alternatively spliced transcripts variants of this gene, which encode distinct isoforms, have been reported. (Tchilian EZ, Beverley PC (2002). "CD45 in memory and disease." Arch. Immunol. Ther. Exp. (Warsz.) 50 (2): 85-93. Ishikawa H, Tsuyama N, Abroun S, et al. (2004). "Interleukin-6, CD45 and the src-kinases in myeloma cell proliferation." Leuk. Lymphoma 44 (9):1477-81.

Various isoforms of CD45 exist: CD45RA, CD45RB, CD45RC, CD45RAB, CD45RAC, CD45RBC, CD45RO, CD45R (ABC). CD45 is also highly glycosylated. CD45R is the longest protein and migrates at 200 kDa when isolated from T cells. B cells also express CD45R with heavier glycosylation, bringing the molecular weight to 220 kDa, hence the name B220; B cell isoform of 220 kDa. B220 expression is not restricted to B cells and can also be expressed on activated T cells, on a subset of dendritic cells and other antigen presenting cells. Stanton T, Boxall S, Bennett A, et al. (2004). "CD45 variant alleles: possibly increased frequency of a novel exon 4 CD45 polymorphism in HIV seropositive Ugandans." Immunogenetics 56 (2): 107-10.

"Gut-homing lymphocytes" refer to a subgroup of lymphocytes having the characteristic of selectively homing to intestinal lymph nodes and tissues but not homing to peripheral lymph nodes and tissues. This subgroup of lymphocytes are characterized by an unique expression pattern of a combination of multiples cell surface molecules, including, but not limited to, the combination of CD4, CD45RA and Beta7. Typically, at least two subsets of peripheral blood CD4⁺ lymphocytes can be subdivided based on the markers of CD45RA and Beta7, CD45RA⁻ β7_{□}^{high}, and CD45RA⁻β7_{□}^{low} CD4⁺ cells. CD45RA⁻β7_{□}^{high} CD4⁺ cells home preferentially to intestinal lymph nodes and tissues, whereas CD45RA-β7_{□}^{low} CD4⁺ cells home preferentially to peripheral lymph nodes and tissues (Rott *et al.* 1996; Rott *et al.* 1997; Williams *et al.* 1998; Rose *et al.* 1998; Williams and Butcher 1997; Butcher *et al.* 1999). Gut-homing lymphocytes are therefore a distinctive subgroup of lymphocytes identified as CD45RA⁻ β7 ^{high} CD4⁺ in a flow cytometry assay. The methods of identifying this group of lymphocytes are well-known in the art and also disclosed in detail in Examples of the present application.

As used herein with respect to a cell surface marker, the symbol "+" indicates a positive expression of a cell surface marker. For instance, CD4⁺ lymphocytes are a group of lymphocytes having CD4 expressed on their cell surfaces.

As used herein with respect to a cell surface marker, the symbol "-" indicates a negative expression of a cell surface marker. For instance, CD45RA⁻ lymphocytes are a group of lymphocytes having no CD45RA expressed on their cell surfaces.

As used herein with respect to the expression of a cell surface marker, the symbol "low" indicates a relatively low level of expression of a cell surface marker on lymphocytes, while "high" indicates a relatively high level of expression of a cell surface marker on lymphocytes. In a flow cytometry, the intensity of β7 ^{high} is at least about 10 or 100 fold higher than that of β7_{□}^{low}. Thus, as provided herein in exemplary embodiments, the CD45RA⁻ β7_{□}^{low} CD4⁺ and CD45RA⁻ β7_{□}^{high} CD4⁺ lymphocytes locate in distinct portions of a dot plot or histogram of a flow cytometry analysis where X-axis is the intensity of expression of CD45AR and Y-axis is the intensity of the expression of Beta7.

"Peripheral-homing lymphocytes" refer to a subgroup of lymphocytes having the characteristic of homing to peripheral lymph nodes and tissues and not homing to intestinal lymph nodes and tissues. In an exemplary embodiment, as explained above, Peripheral-homing lymphocytes are a distinctive group of lymphocytes identified as CD45RA⁻ β7_{□}^{low} CD4⁺ cells in a flow cytometry assay. The methods of identifying this group of lymphocytes are known in the art and disclosed in detail in the present application.

An "amount" or "level" of biomarker can be determined using methods known in the art. For example, in exemplary embodiments, the "amount" or "level" of gut-homing lymphocytes, which is associated with the responsiveness of a patient to a treatment with integrin beta7 antagonist is a detectable level in a biological sample, preferably in a peripheral blood sample. The amount of the gut-homing lymphocytes can be quantified by methods known to the expert skilled in the art and disclosed by this invention, such as flow cytometry analysis.

In some embodiments, an "elevated" or "increased" amount or level of a biomarker, is as compared to a reference/comparator amount of the biomarker. The increase is preferably greater than about 10%, preferably greater than about 30%, preferably greater than about 50%, preferably greater than about 100%, preferably greater than about 300% as a function of the value for the reference or comparator amount. For example, a reference or comparator amount can be the amount of a biomarker before treatment and more particularly, can be the baseline or pre-dose amount.

For example, in one embodiment, an "amount" or "level" of gut-homing lymphocytes denotes a sufficient increase in the number of lymphocytes such that one of skill in the art would consider the increase to be of statistical significance within the context of the biological characteristic measured by said values. The increase is preferably greater than about 10%, preferably greater than about 30%, preferably greater than about 50%, preferably greater than about 100%, preferably greater than about 300% as a function of the value for the reference/comparator amount of lymphocytes.

In some embodiments, a "decreased" amount or level of a biomarker, is as compared to a reference/comparator amount of the biomarker. The decrease is preferably less than about 10%, preferably less than about 30%, preferably less than about 50%, preferably less than about 100%, preferably less than about 300% as a function of the value for the reference or comparator amount. For example, a reference or comparator amount can be the amount of a biomarker before treatment and more particularly, can be the baseline or pre-dose amount.

For example, a "treatment that effectively increases the amount of the gut-homing lymphocytes" refers to a treatment that sufficiently increases in the number of lymphocytes such that one of skill in the art would consider the increase to be of statistical significance within the context of the biological characteristic measured by said values. The increase is preferably greater than about 10%, preferably greater than about 30%, preferably greater than about 50%, preferably greater than about 100%, preferably greater than about 300% as a function of the value for the reference/comparator amount of lymphocytes prior to the treatment.

The phrase "does not substantially change" as used herein, denotes a insignificant degree of change such that one of skill in the art would not consider the change to be of statistical significance within the context of the biological characteristic measured by said values (*e.g.,* the amount of lymphocytes). The change is preferably less than about 10%, preferably less than about 5%, preferably less than about 1%.

"Gastrointestinal inflammatory disorders" are a group of chronic disorders that cause inflammation and/or ulceration in the mucous membrane. These disorders include, for example, inflammatory bowel disease (*e.g.,* Crohn's disease, ulcerative colitis, indeterminate colitis and infectious colitis), mucositis (*e.g.,* oral mucositis, gastrointestinal mucositis, nasal mucositis and proctitis), necrotizing enterocolitis and esophagitis. In a preferred embodiment, the gastrointestinal inflammatory disorder is a inflammatory bowel disease.

"Inflammatory Bowel Disease" or "IBD" is used interchangeably herein to refer to diseases of the bowel that cause inflammation and/or ulceration and includes without limitation Crohn's disease and ulcerative colitis.

"Crohn's disease (CD)" or "ulcerative colitis (UC)" are chronic inflammatory bowel diseases of unknown etiology. Crohn's disease, unlike ulcerative colitis, can affect any part of the bowel. The most prominent feature Crohn's disease is the granular, reddish-purple edmatous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual.

Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

Anti-inflammatory drugs sulfasalazine and 5-aminosalisylic acid (5-ASA) are useful for treating mildly active colonic Crohn's disease and are commonly prescribed to maintain remission of the disease. Metroidazole and ciprofloxacin are similar in efficacy to sulfasalazine and appear to be particularly useful for treating perianal disease. In more severe cases, corticosteroids are effective in treating active exacerbations and can even maintain remission. Azathioprine and 6-mercaptopurine have also shown success in patients who require chronic administration of cortico steroids. It is also possible that these drugs may play a role in the long-term prophylaxis. Unfortunately, there can be a very long delay (up to six months) before onset of action in some patients. Antidiarrheal drugs can also provide symptomatic relief in some patients. Nutritional therapy or elemental diet can improve the nutritional status of patients and induce symptomatic improvement of acute disease, but it does not induce sustained clinical remissions. Antibiotics are used in treating secondary small bowel bacterial overgrowth and in treatment of pyogenic complications.

"Ulcerative colitis (UC)" afflicts the large intestine. The course of the disease may be continuous or relapsing, mild or severe. The earliest lesion is an inflammatory infiltration with abscess formation at the base of the crypts of Lieberkuhn. Coalescence of these distended and ruptured crypts tends to separate the overlying mucosa from its blood supply, leading to ulceration. Symptoms of the disease include cramping, lower abdominal pain, rectal bleeding, and frequent, loose discharges consisting mainly of blood, pus and mucus with scanty fecal particles. A total colectomy may be required for acute, severe or chronic, unremitting ulcerative colitis.

The clinical features of UC are highly variable, and the onset may be insidious or abrupt, and may include diarrhea, tenesmus and relapsing rectal bleeding. With fulminant involvement of the entire colon, toxic megacolon, a life-threatening emergency, may occur. Extraintestinal manifestations include arthritis, pyoderma gangrenoum, uveitis, and erythema nodosum.

Treatment for UC includes sulfasalazine and related salicylate-containing drugs for mild cases and corticosteroid drugs in severe cases. Topical administration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

An "effective dosage" refers to an amount effective, at dosages and for periods of time necessary, to achieve the desired therapeutic or prophylactic result.

As used herein, the term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

The term "non-human subject" refers to any single non-human animal, more preferably a mammal (including such non-human animals as, for example, dogs, cats, horses, rabbits, zoo animals, cows, pigs, sheep, and non-human primates).

The terms "antibody" and "immunoglobulin" are used interchangeably in the broadest sense and include monoclonal antibodies (for example, full length or intact monoclonal antibodies), polyclonal antibodies, multivalent antibodies, multispecific antibodies (*e.g.,* bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, *e.g.,* Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, *e.g.,* Kozbor J. Immunol., 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (*e.g.,* mice) that are capable of producing a full repertoire of human antibodies in the absence of endogenous immunoglobulin production (see, *e.g.,* Jakobovits et al., Proc. Natl. Acad. Sci USA, 90: 2551 (1993); Jakobovits et al., Nature, 362: 255 (1993); Bruggermann et al.,Year in Immunol., 7: 33 (1993)). This definition of a human antibody specifically excludes a humanized antibody comprising antigen-binding residues from a non-human animal.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The term "hypervariable region," "HVR," or "HV," when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these HVRs are noted below.

| Loop Kabat | AbM | Chothia | Contact |
|---|---|---|---|
| L1 L24-L34 | L24-L34 | L26-L32 | L30-L36 |
| L2 L50-L56 | L50-L56 | L50-L52 | L46-L55 |
| L3 L89-L97 | L89-L97 | L91-L96 | L89-L96 |
| H1 H31-H35B | H26-H35B | H26-H32 | H30-H35B (Kabat Numbering) |
| H1 H31-H35 | H26-H35 | H26-H32 | H30-H35 (Chothia Numbering) |
| H2 H50-H65 | H50-H58 | H53-H55 | H47-H58 |
| H3 H95-H102 | H95-H102 | H96-H101 | H93-H101 |

Hypervariable regions may comprise "extended hypervariable regions" as follows: 24-36 or 24-34 (L1), 46-56 or 49-56 or 50-56 or 52-56 (L2) and 89-97 (L3) in the VL and 26-35 (H1), 50-65 or 49-65 (H2) and 93-102, 94-102 or 95-102 (H3) in the VH. The variable domain residues are numbered according to Kabat *et al., supra* for each of these definitions.

"Framework" or "FR" residues are those variable domain residues other than the hypervariable region residues as herein defined.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat *et al.* In one embodiment, for the VL, the subgroup is subgroup kappa I as in Kabat *et al.* In one embodiment, for the VH, the subgroup is subgroup III as in Kabat *et al.*

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1996); Yelton et al. J. Immunol. 155:1994-2004 (1995); Jackson et al., J. Immunol. 154(7):3310-9 (1995); and Hawkins et al. J. Mol. Biol. 226:889-896 (1992).

The phrase "substantially similar," or "substantially the same," as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristic measured by said values (*e.g.,* Kd values). The difference between said two values is preferably less than about 50%, preferably less than about 40%, preferably less than about 30%, preferably less than about 20%, preferably less than about 10% as a function of the value for the reference/comparator antibody.

"Binding affinity" generally refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheep configuration, connected by three hypervariable regions, which form loops connecting, and in some cases forming part of, the β-sheep structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab= fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), *e.g.,* IgG₁, IgG₂, IgG₃, IgG₄, IgA₁, and IgA₂. The heavy-chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W. B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors (*e.g.,* B cell receptor; BCR), etc. Such effector functions generally require the Fc region to be combined with a binding domain (*e.g.,* an antibody variable domain) and can be assessed using various assays as herein disclosed, for example.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, *e.g.,* from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g.,* IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, e.g., from blood or PBMCs as described herein.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)), and regulates homeostasis of immunoglobulins. Antibodies with improved binding to the neonatal Fc receptor (FcRn), and increased half-lives, are described in WO00/42072 (Presta, L.) and US2005/0014934A1 (Hinton et al.)*.* These antibodies comprise an Fc region with one or more substitutions therein which improve binding of the Fc region to FcRn. For example, the Fc region may have substitutions at one or more of positions 238, 250, 256, 265, 272, 286, 303, 305, 307, 311, 312, 314, 317, 340, 356, 360, 362, 376, 378, 380, 382, 413, 424, 428 or 434 (Eu numbering of residues). The preferred Fc region-comprising antibody variant with improved FcRn binding comprises amino acid substitutions at one, two or three of positions 307, 380 and 434 of the Fc region thereof (Eu numbering of residues).

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994). HER2 antibody scFv fragments are described in WO93/16185; U.S. Patent No. 5,571,894; and U.S. Patent No. 5,587,458.

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "affinity matured" antibody is one with one or more alterations in one or more hypervariable regions thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. Ordinarily, amino acid sequence variants will possess at least about 70% homology with the main species antibody, and preferably, they will be at least about 80%, more preferably at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (*e.g.,* deamidated antibody variant), a basic variant, an antibody with an amino-terminal leader extension (*e.g.* VHS-) on one or two light chains thereof, an antibody with a C-terminal lysine residue on one or two heavy chains thereof, etc, and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieties attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc, and combinations of glycosylation alterations. Where the antibody has an Fc region, an oligosaccharide structure may be attached to one or two heavy chains of the antibody, *e.g.* at residue 299 (298, Eu numbering of residues).

The term "cytotoxic agent" as used herein refers to a substance that inhibits or prevents the function of cells and/or causes destruction of cells. The term is intended to include radioactive isotopes (*e.g*. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "immunosuppressive agent" as used herein for adjunct therapy refers to substances that act to suppress or mask the immune system of the subject being treated herein. This would include substances that suppress cytokine production, down-regulate or suppress self-antigen expression, or mask the MHC antigens. Examples of such agents include 2-amino-6-aryl-5-substituted pyrimidines (see U.S. Patent No. 4,665,077); non-steroidal anti-inflammatory drugs (NSAIDs); ganciclovir; tacrolimus; glucocorticoids such as cortisol or aldosterone; anti-inflammatory agents such as a cyclooxygenase inhibitor; a 5-lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6 mercaptopurine; steroids such as corticosteroids or glucocorticosteroids or glucocorticoid analogs, *e.g.,* prednisone, methylprednisolone, including SOLU-MEDROL.RTM. methylprednisolone sodium succinate, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or antagonists including anti-interferon-alpha, -beta, or -gamma antibodies, anti-tumor necrosis factor(TNF)-alpha antibodies (infliximab (REMICADE.RTM.) or adalimumab), anti-TNF-alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CD11a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor *(*Cohen et al., U.S. Patent No. 5,114,721); T-cell receptor fragments (Offner et al., Science, 251: 430-432 (1991); WO 90/11294; Ianeway, Nature, 341: 482 (1989); and WO 91/01133); BAFF antagonists such as BAFF or BR3 antibodies or immunoadhesins and zTNF4 antagonists (for review, see Mackay and Mackay, Trends Immunol., 23:113-5 (2002) and see also definition below); biologic agents that interfere with T cell helper signals, such as anti-CD40 receptor or anti-CD40 ligand (CD154), including blocking antibodies to CD40-CD40 ligand.(*e.g*., Durie etal., Science, 261: 1328-30 (1993); Mohan et al., J. Immunol., 154: 1470-80 (1995)) and CTLA4-Ig (Finck et al., Science, 265: 1225-7 (1994)); and T-cell receptor antibodies (EP 340,109) such as T10B9.

### II. Detailed Description

### A. Beta7 integrin Antagonists

The present specification discloses methods of predicting the responsiveness of a patient to the treatment of beta7 integrin antagonists. Examples of potential antagonists include an oligonucleotide that binds to the fusions of immunoglobulin with beta7 integrin, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the beta7 integrin that recognizes the ligand but imparts no effect, thereby competitively inhibiting the action of the beta7 integrin.

Another potential beta7 integrin antagonist is an antisense RNA or DNA construct prepared using antisense technology, where, *e.g.,* an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the beta7 integrin herein, is used to design an antisense RNA oligonucleotide of from about 10 to 40 base pairs in length. A DNA oligonucleotide is designed to be complementary to a region of the gene involved in transcription (triple helix--see Lee et al., Nucl. Acids Res., 6:3073 (1979); Cooney et al., Science, 241: 456 (1988); Dervan et al., Science, 251:1360 (1991)), thereby preventing transcription and the production of the beta7 integrin. The antisense RNA oligonucleotide hybridizes to the mRNA *in vivo* and blocks translation of the mRNA molecule into beta7 integrin protein (antisense--Okano, Neurochem., 56:560 (1991); Oligodeoxynucleotides as Antisense Inhibitors of Gene Expression (CRC Press: Boca Raton, Fla., 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, *e.g.,* between about -10 and +10 positions of the target gene nucleotide sequence, are preferred.

Other potential antagonists include small molecules that bind to the active site, the ligand or binding molecule binding site, thereby blocking the normal biological activity of the beta7 integrin. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can-be identified by known techniques. For further details see, *e.g.,* Rossi, Current Biology, 4:469-471 (1994), and PCT Publication No. WO 97/33551 (published Sep. 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.,* PCT Publication No. WO 97/33551. These small molecules can be identified by any one or more of the screening assays discussed hereinabove and/or by any other screening techniques well known for those skilled in the art.

Screening assays for antagonists are designed to identify compounds that bind or complex with the beta7 integrin encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

### B. Anti-Beta7 integrin Antibodies

In one aspect, the beta7 integrin antagonists are anti-beta7 antibodies. Exemplary antibodies include polyclonal, monoclonal, humanized, human, bispecific, and heteroconjugate antibodies, etc., as described below.

### 1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g.,* keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g.,* 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, Calif. USA, and SP-2 and derivatives *e.g.,* X63-Ag8-653 cells available from the American Type Culture Collection, Manassas, Va., USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980). Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal *e.g.,* by i.p. injection of the cells into mice. The monoclonal antibodies secreted by the subclones are suitably separated from the culture medium, ascites fluid, or serum by conventional antibody purification procedures such as, for example, affinity chromatography (*e.g.,* using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (*e.g.,* by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs. 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (CH and CL) sequences for the homologous murine sequences (U.S. Patent No. 4,816,567; and Morrison, et al., Proc. Natl. Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Exemplary anti-beta7 antibodies are Fib504, Fib 21, 22, 27, 30 (Tidswell, M. J Immunol. 1997 Aug 1;159(3):1497-505) or humanized derivatives thereof. Humanized antibodies of Fib504 was disclosed in detail in U.S. Patent Publication No. 20060093601 (issued as U.S. Patent No. 7,528,236), the content of which is incorporated by reference in its entirety (also see discussion below).

### 3. Human and Humanized Antibodies

The anti-beta7 integrin antibodies of the invention may further comprise humanized antibodies or human antibodies. Humanized forms of non-human (*e.g.,* murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)). It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized Anti-beta7 integrin antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

Exemplary humanized anti-beta7 antibodies include, but are not limited to rhuMAb Beta7, which is a humanized monoclonal antibody against the integrin subunit β7 and was derived from the rat anti-mouse/human monoclonal antibody FIB504 (Andrew et al., 1994 J Immunol 1994;153:3847-61.). It has been engineered to include human immunoglobulin (Ig)G1 heavy chain and κ1 light chain frameworks and is produced by Chinese hamster ovary cells. This antibody binds to two integrins, α4β7 (Holzmann et al. 1989 Cell, 1989;56:37-46; Hu et al., 1992, Proc Natl Acad Sci USA 1992;89:8254-8) and αEβ7 (Cepek et al., 1993 J Immunol 1993;150:3459-70), which regulate trafficking and retention of lymphocyte subsets in the gastrointestinal tract and are involved in inflammatory bowel diseases (IBD) such as ulcerative colitis (UC) and Crohn's disease (CD). rhuMAb Beta7 is a potent *in vitro* blocker of the cellular interaction between α4β7 and its ligands (mucosal addressin cell adhesion molecule-1 [MAdCAM]-1, vascular cell adhesion molecule [VCAM]-1, and fibronectin) as well as the interaction between α4β7 and its ligand (E-cadherin). rhuMAb Beta7 binds reversibly, with similar high affinity, to β7 on lymphocytes from rabbits, cynomolgus monkeys, and humans. It also binds to mouse β7 with high affinity. The amino acid sequence and the make and use of rhuMAb Beta7 and its variants are disclosed in detail in U.S. Patent Application Publication No. 20060093601 (issued as U.S. Patent No. 7,528,236), the content of which is incorporated in its entirety.

FIGS. 12A and 12B depict alignment of sequences of the variable light and heavy chains for the following: light chain human subgroup kappa I consensus sequence (FIG. 12A, SEQ ID NO:12), heavy chain human subgroup III consensus sequence (FIG. 12B, SEQ ID NO:13), rat anti-mouse beta7 antibody (Fib504) variable light chain (FIG. 12A, SEQ ID NO:10), rat anti-mouse beta7 antibody (Fib504) variable heavy chain (FIG. 12B, SEQ ID NO:11), and humanized antibody variants: Humanized hu504Kgraft variable light chain (FIG. 12A, SEQ ID NO:14), humanized hu504K graft variable heavy chain (FIG. 12B, SEQ ID NO:15), variants hu504-5, hu504-16, and hu504-32 (amino acid variations from humanized hu504K graft are indicated in FIG. 12A) (light chain) (SEQ ID NOS:22-24, respectively, in order of appearance) and FIG. 12B (heavy chain) for variants hu504-5, hu504-16, and hu504-32 (SEQ ID NO:25).

### 4. Human Antibodies

As an alternative to humanization, human antibodies can be generated. For example, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy-chain joining region (J.sub.H) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all of GenPharm); U.S. Patent No. 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, *e.g.,* Johnson, Kevin S. and Chiswell, David J., Current Opinion in Structural Biology 3:564-571 (1993). Several sources of V-gene segments can be used for phage display. Clackson et al., Nature, 352:624-628(1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al, J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patent Nos. 5,567,610 and 5,229,275).

### 5. Antibody Fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," e.g., as described in US Patent No. 5,641,870 for example. Such linear antibody fragments may be monospecific or bispecific.

### 6. Bispecific Antibodies

Bispecific antibodies are antibodies that have binding specificities for at least two different epitopes. Exemplary bispecific antibodies may bind to two different epitopes of beta7 integrin as described herein. Other such antibodies may combine a TAT binding site with a binding site for another protein. Alternatively, an anti-Beta7 integrin arm may be combined with an arm which binds to a triggering molecule on a leukocyte such as a T-cell receptor molecule (*e.g.,* CD3), or Fc receptors for IgG (Fc.γ.R), such as Fc.γRI (CD64), Fc.γRII (CD32) and Fc.γ.RIII (CD16), so as to focus and localize cellular defense mechanisms to the TAT-expressing cell. Bispecific antibodies may also be used to localize cytotoxic agents to cells which express TAT. These antibodies possess a TAT-binding arm and an arm which binds the cytotoxic agent (*e.g.,* saporin, anti-interferon-.alpha., vinca alkaloid, ricin A chain, methotrexate or radioactive isotope hapten). Bispecific antibodies can be prepared as full length antibodies or antibody fragments (*e.g.,* F(ab')₂ bispecific antibodies).

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificities (antibody-antigen combining sites) are fused to immunoglobulin constant domain sequences. Preferably, the fusion is with an Ig heavy chain constant domain, comprising at least part of the hinge, C_{H2}, and C_{H3} regions. It is preferred to have the first heavy-chain constant region (C_{H1}) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C.sub.H3 domain. In this method, one or more small amino acid side chains from the interface of the first antibody molecule are replaced with larger side chains (*e.g.,* tyrosine or tryptophan). Compensatory "cavities" of identical or similar size to the large side chain(s) are created on the interface of the second antibody molecule by replacing large amino acid side chains with smaller ones (*e.g.,* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab').sub.2 fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives: One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med. 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V.sub.H connected to a V.sub.L by a linker which is too short to allow pairing between the two domains on the same chain. Accordingly, the V.sub.H and V.sub.L domains of one fragment are forced to pair with the complementary V.sub.L and V.sub.H domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 7. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 8. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (*e.g.,* tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1).sub.n-VD2-(X2).sub.n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 9. Effector Function Engineering

It may be desirable to modify the antibody of the invention with respect to effector function, *e.g.,* so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., AntiCancer Drug Design 3:219-230 (1989). To increase the serum half life of the antibody, one may incorporate a salvage receptor binding epitope into the antibody (especially an antibody fragment) as described in U.S. Patent No. 5,739,277, for example. As used herein, the term "salvage receptor binding epitope" refers to an epitope of the Fc region of an IgG molecule (*e.g.,* IgG₁, IgG₂, IgG₃, or IgG₄) that is responsible for increasing the *in vivo* serum half-life of the IgG molecule.

### 10. Immunoconjugates

The antagonist or antibody used in the methods herein is optionally conjugated to another agent, such as a cytotoxic agent, or cytokine.

Conjugation will ordinarily be achieved through a covalent linkage, the precise nature of which will be determined by the targeting molecule and the linking site on the integrin beta7 antagonist or antibody polypeptide. Typically, a non-peptidic agent is modified by the addition of a linker that allows conjugation to anti-beta7 integrin antibody through its amino acid side chains, carbohydrate chains, or reactive groups introduced on antibody by chemical modification. For example, a drug may be attached through the .epsilon.-amino group of a lysine residue, through a free .alpha.-amino group, by disulfide exchange to a cysteine residue, or by oxidation of the 1,2- diols in a carbohydrate chain with periodic acid to allow attachment of drugs containing various nucleophiles through a Schiff-base linkage. See, for example, U.S. Patent No. 4,256,833. Protein modifying agents include amine-reactive reagents (*e.g.,* reactive esters, isothiocyanates, aldehydes, and sulfonyl halides), thiol-reactive reagents (*e.g.,* haloacetyl derivatives and maleimides), and carboxylic acid- and aldehyde-reactive reagents. Integrin beta7 antagonist or antibody polypeptides can be covalently joined to peptidic agents through the use of bifunctional cross-linking reagents. Heterobifunctional reagents are more commonly used and permit the controlled coupling of two different proteins through the use of two different reactive moieties (*e.g.,* amine-reactive plus thiol, iodoacetamide, or maleimide). The use of such linking agents is well known in the art. See, for example, Brinkley, *supra,* and U.S. Patent No. 4,671,958. Peptidic linkers can also be employed. In the alternative, an anti-beta7 integrin antibody polypeptide can be linked to a peptidic moiety through preparation of a fusion polypeptide.

Examples of further bifunctional protein coupling agents include N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### 11. Immunoliposomes

The anti-beta7 integrin antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Patent Nos. 4,485,045 and 4,544,545; and WO97/38731 published Oct. 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 12. Vectors, Host Cells and Recombinant Methods For Antibody Production

The invention also provides isolated nucleic acids encoding the anti-beta7 antibodies or polypeptide agents described herein, vectors and host cells comprising the nucleic acids and recombinant techniques for the production of the antibodies.

For recombinant production of the antibody, the nucleic acid encoding it may be isolated and inserted into a replicable vector for further cloning (amplification of the DNA) or for expression. In another embodiment, the antibody may be produced by homologous recombination, e.g., as described in U.S. Patent No. 5,204,244, specifically incorporated herein by reference. DNA encoding the monoclonal antibody is readily isolated and sequenced using conventional procedures (*e.g.,* by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence, *e.g.,* as described in U.S. Patent No. 5,534,615 issued Jul. 9, 1996 and specifically incorporated herein by reference.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, *e.g., E. coli,* Enterobacter, Erwinia, Klebsiella, Proteus, Salmonella, *e.g.,* Salmonella typhimurium, Serratia, *e.g.,* Serratia marcescans, and Shigella, as well as Bacilli such as B. subtilis and B. licheniformis (*e.g.,* B. licheniformis 41P disclosed in DD 266,710 published 12 Apr. 1989), Pseudomonas such as P. aeruginosa, and Streptomyces. One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for anti-beta7 integrin antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.,* Neurospora, Penicillium, Tolypocladium, and Aspergillus hosts such as A. nidulans and A. niger.

Suitable host cells for the expression of glycosylated anti-Beta7 antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR(CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for anti-beta7 integrin antibody production and cultured in conventional nutrient media modified as appropriate for inducing promoters, selecting transformants, or amplifying the genes encoding the desired sequences.

The host cells used to produce the anti-beta7 integrin antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 1 02:255 (1980), U.S. Patent Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN.TM.drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human .gamma. 1, .gamma.2, or .gamma.4 heavy chains (Lindmark et al., J. Immunol. Meth. 62:1-13 (1983)). Protein G is recommended for all mouse isotypes and for human .gamma.3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C.sub.H3 domain, the Bakerbond ABX.TM.resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE.TM. chromatography on an anion or cation exchange resin (such as a polyaspartic acid column), chromatofocusing, SDS-PAGE, and ammonium sulfate precipitation are also available depending on the antibody to be recovered. Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g.,* from about 0-0.25 M salt).

### C. Pharmaceutical Formulations

Therapeutic formulations comprising the therapeutic agents, antagonists or antibodies of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes); and/or non-ionic surfactants such as TWEEN.TM., PLURONICS.TM. or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, *e.g.,* films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and .gamma. ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### D. Treatment Methods

The integrin beta7 antagonists, such as anti-beta7 antibodies of the invention (and adjunct therapeutic agents) are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Dosing can be by any suitable route, for example by injections, such as intravenous or subcutaneous injections, depending in part on whether the administration is brief or chronic.

The therapeutic agents of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic agent need not be, but is optionally formulated with one or more agents currently used to prevent or treat the disorder in question. The effective amount of such other agents depends on the amount of antibodies of the invention present in the formulation, the type of disorder or treatment, and other factors discussed above. These are generally used in the same dosages and with administration routes as used hereinbefore or about from 1 to 99% of the heretofore employed dosages.

For the prevention or treatment of disease, the appropriate dosage of a beta7-antagonist of the invention (when used alone or in combination with other agents such as chemotherapeutic agents) will depend on the type of disease to be treated, the type of antibody, the severity and course of the disease, whether the agent is administered for preventive or therapeutic purposes, previous therapy, the patient's clinical history and response to the antibody, and the discretion of the attending physician. The beta7-antagonist, such as a beta7 antibody is suitably administered to the patient at one time or over a series of treatments. Depending on the type and severity of the disease, about 1 mg/kg to 15 mg/kg of antibody is an initial candidate dosage for administration to the patient, whether, for example, by one or more separate administrations, or by continuous infusion. One typical daily dosage might range from about 1 mg/kg to 100 mg/kg or more, depending on the factors mentioned above. For repeated administrations over several days or longer, depending on the condition, the treatment is sustained until a desired suppression of disease symptoms occurs. One exemplary dosage of the antibody would be in the range from about 5 mg/kg to about 50 mg/kg. Thus, one or more doses of about 15 mg/kg, 20 mg/kg, 30 mg/kg or 50 mg/kg (or any combination thereof) may be administered to the patient. Such doses may be administered intermittently, *e.g.,* at least once every week or at least once every two, three, four, five, six, seven, eight, ten, twelve weeks (*e.g.,* such that the patient receives from about two to about twenty, *e.g.,* about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays. Depending on desired result of the treatment, the patient may be treated for at least about 1, 2, 6, 12, 24, 36, or 52 weeks.

The standard of care for subjects with active moderate-severe active UC involves therapy with standard doses of: an aminosalicylate, an oral corticosteroid, 6-mercaptopurine (6-MP) and/or azathioprine. Therapy with an integrin beta7 antagonist, such as an anti-beta7 integrin antibody as disclosed herein will result in an improvement in disease remission (rapid control of disease and/or prolonged remission), and/or clinical response, superior to that achieved with the standard of care for such subjects.

In one aspect, the treatment for inflammatory bowel disease (IBD) in a human subject with IBD comprises administering to the subject an effective amount of an therapeutic agent, such as an anti-beta7 integrin antibody, and further comprising administering to the subject an effective amount of a second medicament, that is an immunosuppressant, a pain-control agent, an antidiarrheal agent, an antibiotic, or a combination thereof.

In an exemplary aspect, said secondary medicine is selected from the group consisting of an aminosalicylate, an oral corticosteroid, 6-mercaptopurine (6-MP) and azathioprine. In another exemplary aspect, said secondary medicine is another integrin beta7 antagonist, such as another anti-beta7 integrin antibody or an antibody against a cytokine.

All these second medicaments may be used in combination with each other or by themselves with the first medicament, so that the expression "second medicament" as used herein does not mean it is the only medicament besides the first medicament, respectively. Thus, the second medicament need not be one medicament, but may constitute or comprise more than one such drug.

These second medicaments as set forth herein are generally used in the same dosages and with administration routes as used hereinbefore or about from 1 to 99% of the heretofore-employed dosages. If such second medicaments are used at all, optionally, they are used in lower amounts than if the first medicament were not present, especially in subsequent dosings beyond the initial dosing with the first medicament, so as to eliminate or reduce side effects caused thereby. For instance, therapy with a anti-beta7 integrin antibody herein permits tapering or discontinued administration of steroid.

Combined administration herein includes co-administration, using separate formulations or a single pharmaceutical formulation, and consecutive administration in either order, wherein preferably there is a time period while both (or all) active agents simultaneously exert their biological activities.

The combined administration of a second medicament includes co-administration (concurrent administration), using separate formulations or a single pharmaceutical formulation, and consecutive administration in either order, wherein preferably there is a time period while both (or all) active agents (medicaments) simultaneously exert their biological activities.

### E. Use of Biomarkers to Assess Treatment of Gastrointestinal Inflammatory Disorders with Integrin beta7 antagonists

The present specification discloses methods of assessing the effect, efficacy, safety, prognosis, and/or dosing of therapeutic agents (or drug), such as integrin beta7 antagonists, for the treatment of a patient having a gastrointestinal inflammatory disorder. In particular, the present specification discloses methods of using the level of gut-homing lymphocytes in the patient's peripheral blood, the level of occupancy of a drug on gut-homing lymphocytes, and/or the level of beta7 integrin receptors on gut-homing lymphocytes as indicators (or "biomarkers") of the effect, efficacy, safety, prognosis, and/or dosing of therapeutic agents such as beta7 integrin antagonists for the treatment of gastrointestinal inflammatory disorders. Such methods include, for example, assessing the responsiveness of a patient to treatment of a gastrointestinal disorder with an integrin beta7 antagonist, using one or more of these biomarkers. Additionally, the present specification discloses methods of using such biomarkers to design a drug treatment and/or dosing regimen, or for prognosis.

In some aspects, the level of a biomarker before treatment of the patient with a therapeutic agent is compared to the level of the same biomarker during and/or after treatment of the patient, and a change (*e.g.,* increase or decrease) in the level of this biomarker in the patient is indicative of the effect, efficacy, safety, and/or prognosis of the therapeutic agent, *e.g.,* a beta7 integrin antagonist, for the treatment of a gastrointestinal inflammatory disorder in a patient. Additionally, such methods of the present invention can be used to design a drug treatment or dosing regimen for treatment of a gastrointestinal inflammatory disorder in a patient.

### 1. Prediction and/or Monitoring of Responsiveness

In one aspect, the specification discloses a method of determining the efficacy of an integrin beta7 antagonist for treatment of a gastrointestinal inflammatory disorder in a patient, the method comprising comparing the amount of a biomarker in a sample obtained from the patient after or during treatment with the integrin beta7 antagonist, to an amount of the biomarker in a sample obtained from the patient before the treatment, wherein a change in the amount of the biomarker after or during the treatment, as compared to before the treatment, is indicative of the efficacy of the antagonist for treatment of the gastrointestinal disorder in the patient.

In another aspect, the specification discloses a method of predicting the responsiveness of a patient having a gastrointestinal inflammatory disorder to treatment with an integrin beta7 antagonist, the method comprising comparing the amount of a biomarker in a sample obtained from the patient after or during treatment with the integrin beta7 antagonist, to the amount of the biomarker in a sample obtained from the patient before the treatment, wherein a change in the amount of the biomarker after or during the treatment, as compared to before the treatment is indicative of the responsiveness of said patient to treatment with said antagonist. In one particular aspect, the present specification discloses a method for predicting whether a patient diagnosed with a gastrointestinal inflammatory disorder will be responsive to a treatment comprising administration of a beta7. The method comprises comparing the amount of gut-homing lymphocytes in a blood sample of the patient following treatment with the amount of gut-homing lymphocytes prior to treatment, wherein an elevated amount following treatment with the integrin beta7 antagonist indicates that the patient responds to the treatment.

Measurement of the amount of biomarkers, including gut-homing lymphocytes in the patient's peripheral blood, integrin beta7 antagonist occupancy on gut-homing lymphocytes, and beta7 integrin receptors on gut-homing lymphocytes, offers the advantage of predicting the responsiveness of a patient to a treatment with beta7 antagonist at early stage during the course of treatment, such as right after receiving the first dose of the beta7 antagonist. Thus, a patient and the patient's physician do not need to wait until the treatment is complete to find out whether the treatment offered any benefit to the patient.

In one aspect, the method of predicting the responsiveness to the treatment with a beta7 antagonist comprises a) obtaining a blood sample from a patient prior to the treatment; b) measuring the amount of biomarker in the blood sample; c) administering an integrin beta7 antagonist to the patient; d) obtaining a blood sample from the patient after receiving the integrin beta7 antagonist, e) measuring the amount of biomarkers in the blood sample; f) comparing the amount of biomarkers in b) and the amount of biomarkers in e). If an elevated amount between e) and b) is observed, the patient will be responsive to the treatment.

To establish a baseline pre-treatment level of biomarkers, measurement of amount of the biomarkers at multiple time points prior to the treatment is preferred, for example, at least 1, 2, 5, 10, 20, 30, 40 days prior to the treatment. In an exemplary embodiment, the amount of the gut-homing lymphocytes in the blood sample of the patient is measured at 5, 10, 20, 30, 60 days prior to the treatment. A average values of the measured amount at each points is calculated and will be used as the pre-treatment baseline level of gut-homing lymphocytes.

To predict patient responsiveness to treatment with integrin beta7 antagonists, the amount of biomarkers in patients' blood can be measured at any given point during the course of treatment, and preferably at the early stage during the course of treatment, for example, but not limited to, within about 8 hours, 12 hours, 1, 2, 4, 10, 20, 50, 100, 300 or 500 days after the patient receives the first dose, preferably from 1-50 days after the patient receives the first dose. In one embodiment, the amount of biomarkers is measured at a single point of interest during the course of treatment. The value obtained at the measured point is compared to the pre-treatment baseline level to generate the differential value for this point. In another embodiment, the amount of biomarkers is measured at multiple time points (for example, at least 2, 4, 6, 8, 10, 16, 20, 40 points) during the course of treatment. The value obtained at each point can be compared to the pre-treatment baseline level to generate the differential value for each measuring point. The responsive to the treatment will be evaluated based on the analysis of these differential values.

The gut-homing lymphocytes are identified by the expression levels of a combination of biomarkers, for example, but not limited to, CD4, CD45RA and Beta7, using the techniques available in the art, for example, flow cytometry analysis (FACS), etc. The subset population of lymphocytes with different expression patterns of these markers are identified with the standard techniques used in the art, such as flow cytometry (FACS).

Fluorescence-activated cell sorting (FACS) provides a method for sorting a heterogeneous mixture of biological cells into two or more containers, one cell at a time, based upon the specific light scattering and florescent characteristics of each cell. It is a useful scientific instrument as it provides fast, objective and quantitative recording of fluorescent signals from individual cells as well as physical separation of cells of particular interest. The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell being in a droplet. Just before the stream breaks into droplets the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement and the opposite charge is trapped on the droplet as it breaks from the stream. The charged droplets then fall through an electrostatic deflection system that diverts droplets into containers based upon their charge. In some systems the charge is applied directly to the stream and the droplet breaking off retains charge of the same sign as the stream. The stream is then returned to neutral after the droplet breaks off.

The data generated by flow-cytometers can be plotted in a single dimension to produce a histogram, or in two dimensional dot plots or even in three dimensions. The regions on these plots can be sequentially separated, based on fluorescence intensity, by creating a series of subset extractions, termed "gates." Specific gating protocols exist for diagnostic and clinical purposes. The plots are often made on logarithmic scales. Because different fluorescent dyes' emission spectra overlap signals at the detectors have to be compensated electronically as well as computationally. Often, data accumulated using the flow cytometer can be re-analyzed elsewhere freeing up the machine for other people to use (Loken MR. "Immunofluorescence Techniques in Flow Cytometry and Sorting": 341-53. Wiley. (1990).

Typically, in a human patient blood sample, three subsets of peripheral blood CD4⁺ lymphocytes can be distinguished by flow cytometry with the biomarkers of the present invention: CD45RA- β7_{⊐}^{high} cells, which home preferentially to intestinal lymph nodes and tissues, CD45RA-β7_{□}^{low} cells, which home preferentially to peripheral lymph nodes and tissues, and CD45RA⁺ β7_{□}^{intermediate} cells, which localize non-preferentially to intestinal and peripheral lymph nodes and tissues. Thus, gut-homing lymphocytes can be identified at least by CD4⁺ CD45RA⁻ β7_{□}^{high}, or other cell surface markers or combination thereof, such as CD8⁺ CD45RA⁻ β7_{⊐}^{high} in a flow cytometry. These three subsets of lymphocytes locate in distinct portions of a dot plot or histogram of a FACS analysis and therefore is distinguishable by a skilled artisan.

The amount of gut-homing lymphocytes can be quantified in various ways in the art. Absolute counts as percentage of baseline levels at predose can be calculated for the gut-homing CDR4⁺ lymphocyte subsets at each time point in the samples collected from a patient. Also can be calculated are the absolute counts for each respective subset of lymphocytes, which equal to the absolute lymphocyte counts (a value obtained from hematology measurements expressed as lymphocytes per microliter of peripheral blood) times the percentage of gated lymphocytes for each subset (obtained from flow cytometry analysis). Gut-homing lymphocytes can also be quantified by antibody counts, which are absolute counts as a percentage of baseline antibody counts obtained before dosing; calculated as antibody counts divided by average of predose absolute counts. As a control, the amount of other subset of lymphocytes, such as the amount of peripheral-homing lymphocytes can also be quantifies for the same sample at each time point.

The amount of integrin beta 7 antagonist occupancy on gut-homing lymphocytes and the amount of beta 7 integrin receptors on gut-homing lymphocytes can also be measured using the techniques known in the art and disclosed in Examples of the present application.

A treatment that will likely provide beneficial response to the patient should significantly increase the amount of the gut-homing lymphocytes in patient blood without substantially increasing the amount of peripheral-homing lymphocytes in the blood sample of the patient. Preferably, the treatment increase the amount of biomarkers by at least about 30%, 50%, or 1, 2, 3, 4, or 5 folds, 1, 2, 4, 6, 8, 20, 30, 50, 100 days after the treatment as compared to the pre-treatment base level. In a particular embodiment, an increase of at least 3 or 5 folds is indicative of an effective response to the treatment.

In another aspect, the present specification discloses a prognostic method for predicting the likelihood of beneficial response of a patient diagnosed with a gastrointestinal inflammatory disorder to a treatment comprising administering an integrin beta7 antagonist into said patient, comprising comparing the amount of biomarkers in a blood sample of said patient after receiving the integrin beta7 antagonist with that prior to the treatment, wherein an elevated amount of biomarkers in blood sample after receiving the integrin beta7 antagonist is indicative of a likelihood of beneficial response.

Like in predicting the responsiveness to a treatment, the amount of biomarkers in peripheral blood can be used to predicting the likelihood of long-term beneficial responses. The beneficial responses include, but not limited to, reducing or eliminating one or more of the symptoms typical for gastrointestinal inflammatory disorder, such as, abdominal pain, nausea, vomiting, diarrhea, frequent bowl movement, fever, fatigue and weight loss, or achieving partial or full remission of the diseases. The amount of the gut-homing lymphocytes in the patient's blood sample can be quantified with the same methods described herein. A treatment that will likely to provide long term beneficial responses to the patient should significantly increase the amount of biomarkers in patient's blood without substantially increasing the amount of peripheral-homing lymphocytes. Preferably, the treatment increases the amount of biomarkers by at least about 1, 3, 5, 8, 10 folds, 1, 2, 4, 6, 8, 20, 30, 50, 100 days after the first does of integrin beta7 antagonist compared is administered to the patient as compared to the pre-treatment baseline level.

In yet another aspect, the present specification discloses a method of monitoring responsiveness of a patient diagnosed with a gastrointestinal inflammatory disorder to a treatment with an integrin beta7 antagonist comprising monitoring that amount of biomarkers in peripheral blood of said patient during said treatment, wherein a maintained elevated amount as compared to that prior to the treatment is indicative that the responsiveness to said treatment is maintained. In particular, pre-treatment baseline level of the amount of biomarkers in a tested patient is calculated as described herein. To monitor patient responsiveness to a treatment with an integrin beta7 antagonist, the amount of biomarkers in the patient' blood sample can be measured at any given points during the course of the treatment and preferably at multiple points throughout the course of the treatment. For instance, the amount of biomarker is measured at least every 1, 2, 3, 4, 5, 6, 7, 8, 9, 10, 11, 12, 13, 14, 15, 20 days depending on the duration of the treatment or the desired efficacy of the treatment. The responsiveness to the treatment in reaction to a intervening event can also be monitored using this approach. For example, the amount of the biomarkers in the patient's blood sample can be measured right after administrating a second medication or starting a changed dosage, or any changes in patients' physical or mental states, etc. Ideally, a maintained elevated level of the gut-homing lymphocytes should be observed for a maintained responsiveness to the treatment. If the level of biomarkers decreases at some point during the treatment or in reaction to certain intervening events, it is indicative that the patient has a reduced responsiveness to the treatment. The treatment regimen should be modified accordingly to maintain an elevated amount of the biomarkers.

The present specification also discloses a method of modifying a treatment with a beta7 antagonist for a patient diagnosed with a gastrointestinal inflammatory disorder comprising: a) monitoring responsiveness of said patient to said treatment with the method described above; and b) changing said treatment so that an elevated amount of said lymphocytes is maintained.

The present invention includes a method of determining the dosing or dosing regimen of an integrin beta7 antagonist for treatment of a gastrointestinal inflammatory disorder in a patient, the method comprising adjusting the dose or a dosing regimen of the integrin beta7 antagonist based on a comparison of the amount of a biomarker in a sample obtained from the patient after or during treatment with a dose or dosing regimen of the integrin beta7 antagonist, to an amount of the biomarker in a sample obtained from the patient before the treatment, wherein a change in the amount of the biomarker after or during the treatment, as compared to before the treatment, is indicative of the efficacy of or responsiveness to the dose or dosing regimen of the integrin beta7 antagonist for treatment of the gastrointestinal disorder in the patient.

### 2. Design Treatment Regimens

Drug development is a complex and expensive process. The cost of bringing a new drug to market is estimated to be between $800 million and $1 billion. Less than 10% of drugs in phase I clinical trials make it to the approval phase. Two key reasons why drugs fail at late stages are a lack of understanding of the relationship between dose-concentration response and unanticipated safety events. Given this scenario, it is critical to have enabling tools that help predict how a drug will perform *in vivo* and assist in the success of a clinical therapeutic candidate (Lakshmi Kamath, Drug Discovery and Development; Modeling Success in PK/PD Testing Drug Discovery & Development (2006)).

Pharmacokinetics (PK) characterizes the absorption, distribution, metabolism, and elimination properties of a drug. Pharmacodynamics (PD) defines the physiological and biological response to the administered drug. PK/PD modeling establishes a mathematical and theoretical link between these two processes and helps better predict drug action. Integrated PK/PD modeling and computer-assisted trial design via simulation are being incorporated into many drug development programs and are having a growing impact (Lakshmi Kamath, Drug Discovery and Development; Modeling Success in PK/PD Testing Drug Discovery & Development (2006)).

PK/PD testing is typically performed at every stage of the drug development process. Because development is becoming increasingly complex, time consuming, and cost intensive, companies are looking to make better use of PK/PD data to eliminate flawed candidates at the beginning and identify those with the best chance of clinical success. (Lakshmi Kamath, *supra*).

PK/PD modeling approaches are proving useful in determining relationships between biomarker response, drug levels, and dosing regimens. The PK/PD profile of a drug candidate and the ability to predict a patient's response to it are critical to the success of clinical trials. Recent advances in molecular biology techniques and a better understanding of targets for various diseases have validated biomarkers as a good clinical indicator of a drug's therapeutic efficacy. Biomarker assays help identify a biological response to a drug candidate. Once a biomarker is clinically validated, trial simulations can be effectively modeled. Biomarkers have the potential to achieve surrogate status that may someday substitute for clinical outcomes in drug development. (Lakshmi Kamath, *supra*).

The amount of biomarkers in the peripheral blood can be used in identifying the biological response to a treatment with integrin beta7 antagonists and can therefore function as a good clinical indicator for the therapeutic efficacy of a candidate treatment.

The present invention includes a method of designing a treatment with a candidate agent for a human patient diagnosed with a gastrointestinal inflammatory disorder, comprising determining an effective dosage for the human patient based on a dosage that effectively increases the amount of a biomarker in peripheral blood of a non-human subject in response to a treatment with said candidate agent, wherein the biomarker is selected from a group consisting of gut-homing lymphocytes in the patient's peripheral blood, occupancy of said therapeutic agent on gut-homing lymphocytes, and beta7 integrin receptors on gut-homing lymphocytes.

In an exemplary embodiment, a method of designing a treatment with an integrin beta7 antagonist for a human patient diagnosed with a gastrointestinal inflammatory disorder, comprising: a) measuring the amount of biomarkers in blood sample of a non-human subject in response to a candidate treatment comprising administering a candidate dosage of an integrin beta7 antagonist into said non-human subject; b) determining whether the candidate dosage can effectively increase the amount of biomarkers in peripheral blood of said non-human subject; c) identifying a dosage that effectively increases the amount of the biomarkers in peripheral blood of said non-human subject; and d) determining an effective dosage for a human patient based on the identified dosage.

In one embodiment, the method of the present invention is used to identify candidate dosages that potentially provide desirable beneficial response in a Phase II and/or Phase III clinical trial study.

The blood sample of the tested non-human subject is collected prior to receiving a candidate treatment in Phase I clinical trial and a baseline level of the biomarkers in peripheral blood of the subject is established as described under Section E1 of the application. The tested non-human subject then receives a candidate treatment comprising administering a candidate dosage of an integrin beta7 antagonist into the tested subject. The blood sample is collected again from the subject at any desired time points during the course of the treatment as described in Section E1, or after the treatment ends. The amount of the biomarkers in response to the candidate treatment will first compared to the pre-treatment baseline to determine the differential value as compared to the pre-treatment baseline for the candidate treatment. If the candidate treatment gives rise to a significant increase, for example at least 50%, 1, 2, 3, 5, 8, or 10 fold increase, in the amount of biomarkers, the dosage will be selected as a candidate dosage for treatment design in Phase II or Phase III clinical trials. The gut-homing lymphocytes can be identified using standard techniques in the art and described under E1 of the present application, particularly by cell surface markers, such as CD45AR, CD4 (CD8) and beta7. The amount of biomarkers can be quantified via the approaches available in the art and described in Section E1.

The above-described process can be used for multiple times for testing a purity of candidate treatments, especially a purity of candidate dosages. The candidate dosage gives rise to the optimal increase of the amount of lymphocytes among the tested candidate dosages will be selected for treatment design in Phase II or III clinical trials. In one embodiment, the same non-human subject can be tested for each of a purity of candidate dosages. After the termination of a candidate treatment, the residual effect of the treatment will be examined using the methods of the present application, for instance, by examining whether the amount of the biomarkers returns to the pre-treatment baseline level. After the amount of the biomarkers returns to the pre-treatment baseline level, another candidate treatment can be delivered to the same subject to see its effect on the gut-homing lymphocytes. In another embodiment, a purity of candidate treatment may be tested in multiple subjects at the same time, with each candidate treatment given to each subject. Multiple subjects may need to be tested for the same candidate regimen to eliminate the individual difference among the subjects in response to same treatment.

The selected candidate dosage from Phase I clinical trial will be used to design the optimal dosage for Phase II and III clinical trials using standard approaches in the art, including, but not limited to, PK/PD modeling. Traditional PK/PD modeling in drug development defines parameters such as drug dose concentration, drug exposure effects, drug half-life, drug concentrations against time, and drug effects against time. When used more broadly, quantitative techniques such as drug modeling, disease modeling, trial modeling, and market modeling can support the entire development process, which results in better decisions through explicit consideration of risk and better utilization of knowledge. A variety of PK/PD modeling tools are available to drug development researchers, for example, WinNonlin and the Knowledgebase Server (PKS) developed by Pharsight, Inc. Mountain View, California.

### F. Biomarker for Responsiveness to the Treatment of Other Candidate Agents

The amount of the biomarkers can also be used to predict the responsiveness to a treatment with a candidate therapeutic agent other than integrin beta7 antagonist. In particular, the methods predicting the outcome of a treatment with an integrin beta7 antagonist described herein can be used in the same manner to: 1) predicting whether a patient will be responsive to a treatment comprising administering a candidate therapeutic agent to the patient; 2) predicting the likelihood of beneficial of the treatment; 3) monitoring responsiveness of the patient to the treatment; and 4) modifying the treatment with the candidate therapeutic agent. The amount of the biomarkers can also be used to design a treatment with a candidate therapeutic agent other than integrin beta7 antagonist using the methods describer above.

Preferably, the candidate agent offers beneficial effect to the patients with inflammatory bowels disease, such as such as an immunosuppressive agent described herein. More preferably, the agent inhibits infiltration of gut-homing lymphocytes into the intestine of the patient, either directly or indirectly.

### G. Predicting Prognosis

In addition to predicting the responsiveness to the treatment, the amount of biomarkers is also useful in predicting the prognosis of an inflammatory bowel disease.

The present description therefore includes a method predicting prognosis of an inflammatory bowel disease for a patient comprising: a) measuring a ratio between that amount of gut-homing lymphocytes and that amount of peripheral-homing lymphocytes in a blood sample of said patient; and b) measuring a ratio between amount of gut-homing lymphocytes and amount of peripheral homing lymphocytes in a blood sample of a healthy individual, wherein a changed ratio of said patient as compared to that of the healthy individual is inductive of prognosis of the disease.

In order to predicting the prognosis, the blood samples of a number of healthy individuals are obtained. The amount of gut-homing lymphocytes, specifically CD45RA⁻ β7_{□}^{high} CD4+ cells, are measured with the methods of the present invention. The amount of peripheral homing lymphocytes (CD45RA⁻ □7_{⊐}^{low} CD4⁺) are also measured in the same blood samples. The ratio of amount of gut-homing lymphocytes versus the amount of peripheral homing lymphocytes is then calculated for each blood sample. An average baseline ratio for healthy individuals is established based on the ratio for each healthy individual. This baseline ratio will be used as a control for deterring whether a decreased ratio in an examined patient is observed. The blood sample is then obtained from the tested patient and the ratio of the amount of gut-homing lymphocytes versus the amount of peripheral homing lymphocytes is calculated. If a significant change (increase or decrease) in the ratio is observed for the patient as compared to the baseline control, such as at least about 30%, 50%, 100%, 200% change , the patient will likely have poor prognosis, for example, a low probability of remission, high probability of recurrence, flaring, or drug resistance.

### H. Method of Identifying a Lymphocyte Population

The humanized anti-beta7 integrin antibody, rhuMAb Beta7, binds to the beta7 integrin subunit regardless of its binding partner. Thus, rhuMAbBeta7 binds to beta7 when it is alone, or associated with alpha4 or alphaE. Thus, this antibody and its variants are good markers to identify a population of lymphocytes comprising lymphocytes expressing alphaEbeta7 integrin and/or alpha4beta7 integrin. In a preferred embodiment, the identified population of lymphocytes comprises lymphocytes expressing alphaEbeta7 integrin, lymphocytes expressing alpha4beta7 integrin and lymphocytes expressing both alpha4beta7 integrin and alphaEbeta7. This is distinct from the existing anti-beta7 antibodies in the art, such as, *act1* (Meenan *et al., supra*), which can only bind to the beta7 subunit when it is connected to alpha4.

The present specification discloses a method of identifying a population of lymphocytes comprising lymphocytes expressing alphaEbeta7 integrin and/or alpha4 beta7 , comprising binding said lymphocytes with an isolated antibody that binds to the same epitope of an antibody comprising a light chain variable region sequence of SEQ ID NO:10, and a heavy chain variable region sequence of SEQ ID NO:11.

In one aspect, the method is used to identify lymphocytes expressing beta7 in peripheral blood of a patient. In particular, patient's blood sample is collected and the lymphocyte population comprising cells expressing alphaEbeta7 integrin, cells expressing alpha4beta7 integrin and cells expressing both can be identified and isolated by using flow cytometry with beta7, CD4 and CD45AR as cell surface markers.

In another aspect, the method is used to visualize lymphocyte population comprising cells expressing alphaEbeta7 integrin and cells expressing alpha4beta7 integrin in lymph node and tissues of the intestine of a patient via standard procedure used in the art, such as immunohisto-staining with rhuMAb Beta7.

The method described herein is particularly useful for detecting the lymphocyte population closely related to the development and progress of inflammatory bowel disease. The identified population, especially the population obtained from a patient's peripheral blood, can be used as a biomarker in predicting patient's responsiveness to a treatment, as well as designing treatments regimens as described in Sections E and F.

The foregoing written specification is considered to be sufficient to enable one skilled in the art to practice the invention. The present invention is not to be limited in scope by the constructs deposited, since the deposited embodiments are intended to illustrate only certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims. Indeed, various modifications of the invention in addition to those shown and described herein will become apparent to those skilled in the art from the foregoing description and fall within the scope of the appended claims.

It is understood that the application of the teachings of the present invention to a specific problem or situation will be within the capabilities of one having ordinary skill in the art in light of the teachings contained herein.

Further details of the invention are illustrated by the following non-limiting Examples. The disclosures of all citations in the specification are expressly incorporated herein by reference.

### Examples

### Example 1

### 12-Week Safety and Toxicokinetic Study with rhuMAb Beta7 Administered Weekly via Intravenous and Subcutaneous Injection in Cynomolgus Monkeys with a 15-Week Recovery Phase and an Extension Phase to Evaluate the Reversal of Pharmacodynamic Effects

### 1. Abstract

rhuMAb Beta7 is a humanized anti-human integrin β7 monoclonal antibody that binds the integrins αEβ7 □ and □ α4β7. Cells that express these integrins have been associated with inflammatory diseases of the gut such as ulcerative colitis and Crohn's disease (Feagan et al. 2005; Sandborn *et al.* 2005). rhuMAb Beta7 is being investigated as a potential therapeutic for immune system-mediated diseases such as inflammatory bowel disease. The purpose of this study was to evaluate the safety, toxicokinetics, and pharmacodynamics of rhuMAb Beta7 when administered weekly via intravenous (IV) injection or subcutaneous (SC) injection to cynomolgus monkeys *(Macaca fascicularis)* for at least 12 weeks and to assess the reversibility, persistence, and delayed occurrence of any drug-related effects after a 15-week recovery. In addition, subsets of animals from the control, mid-, and high-dose groups were observed for an additional period before necropsy (during an extension phase) to assess the reversal of pharmacodynamic effects.

Naive cynomolgus monkeys were assigned to six groups (7 animals/sex/group in Groups 1, 3, 4, 5, and 6 and 5 animals/sex in Group 2). Group 1 animals were given a total of 12 weekly doses of vehicle via IV and SC injection. Animals in Groups 2-6 were given a total of 12 weekly doses of rhuMAb Beta7 at 5 mg/kg IV (Group 2), 15 mg/kg IV (Group 3), 50 mg/kg IV (Group 4), 15 mg/kg SC (Group 5), or 50 mg/kg SC (Group 6), respectively. Following the 12-week dosing phase, 3 male and 3 female animals from each dose group were necropsied on Study Day 80, which was 2 days after the final dose. The remaining animals were observed for 15 additional weeks during a recovery phase. At the end of the recovery phase (on Study Day 184), 2 animals/sex/group were necropsied. After the 15-week recovery necropsy, the remaining animals (2 males and 1 female from each of Groups 1 and 3 and 2 males and 2 females from each of Groups 4, 5, and 6) were observed to assess the reversal of PD effects. PD endpoints were monitored during this extension phase and the results were used to determine the end of the in-life phase of the study.

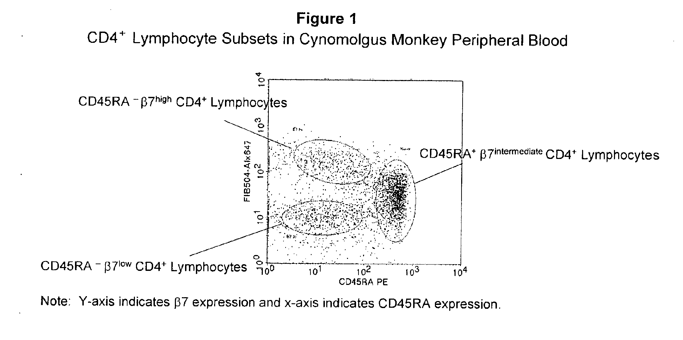

Three subsets of peripheral blood CD4⁺ lymphocytes, subdivided according to their homing properties, were monitored by flow cytometry as exploratory PD markers: CD45RA-β7_{□}^{high}, CD45RA⁻β7_{□}^{low}, and CD45RA⁺ β7_{⊐}^{intermediate} CD4⁺ cells (see Figure 1). CD45RA- β7_{□}^{high} CD₄⁺ cells are phenotypically similar to human and mouse memory/effector lymphocytes that home preferentially to intestinal lymph nodes and tissues, whereas CD45RA⁻ β7_{□}^{low} CD4⁺ cells are phenotypically similar to human and mouse memory/effector lymphocytes that home preferentially to peripheral lymph nodes and tissues (Rott *et al.* 1996; Rott *et al.* 1997; Williams *et al.* 1998; Rose *et al.* 1998; Williams and Butcher 1997; Butcher *et al.* 1999). CD45RA⁺ β7_{□}^{intermediate} CD4⁺ cells, phenotypically similar to human and mouse naive lymphocytes, traffic without apparent preference to both intestinal and peripheral lymph nodes (Rott *et al.* 1996; Rott *et al.* 1997; Williams *et al.* 1998; Rosé *et al.* 1998; Williams and Butcher 1997; Butcher *et al.* 1999).

Group mean values of CD45RA⁻ β7^{high} CD4⁺ lymphocytes increased approximately 4-to 5-fold over baseline levels during the dosing phase, following IV administration of 5 mg/kg rhuMAb Beta7. Administration of 15 mg/kg or 50 mg/kg rhuMAb Beta7 (IV or SC) resulted in slightly greater increases (approximately 5- to 6-fold) in group mean numbers of CD45RA-7^{high} CD4⁺ lymphocytes during the dosing phase, compared with baseline levels. In general, the majority of the animals given 15 or 50 mg/kg (IV or SC) had sustained PD effects during the dosing phase, unlike the majority of animals in the 5 mg/kg dose group. Animals that were given vehicle showed no substantial changes in group mean numbers of peripheral blood CD45RA⁻ β7_{□}^{high} CD4⁺ lymphocytes. Group mean numbers of CD45RA⁺β7^{intermediate} CD4⁺ lymphocytes and CD45RA- β7^{low} CD4⁺ lymphocytes were not substantially different in cynomolgus monkeys dosed with vehicle or rhuMAb Beta7. These findings are consistent with the proposed mechanism of action of rhuMAb Beta7- - the inhibition of homing of β7-positive lymphocytes to the gut, through prevention of α4β7 binding to its ligands. This proposed mechanism is expected to lead to accumulation of CD45RA⁻β7^{high} CD4⁺ lymphocytes in the peripheral circulation.

By the end of the 15-week recovery phase (on Study Day 184), the numbers of CD45RA⁻ β7_{□}^{high} CD4⁺ lymphocytes returned to baseline levels in all of the animals given 5 mg/kg IV doses of rhuMAb Beta7 (10 of 10). In the groups given IV doses of 15 or 50 mg/kg rhuMAb Beta7, the numbers of CD45RA⁻ β7_{⊐}^{high} CD4⁺ lymphocytes returned to baseline levels in 7 of 13 and 2 of 14 animals, respectively. In the groups given SC doses of 15 or 50 mg/kg rhuMAb Beta7, the numbers of CD45RA⁻ β7_{□}^{high} CD4⁺ lymphocytes returned to baseline levels in 6 of 14 and 1 of 14 animals, respectively. Thus, the rate of return of absolute numbers of CD45RA⁻ β7_{□}^{high} CD4⁺ lymphocytes to baseline levels appeared to be dependent upon the dose. By the end of the PD extension phase (on Study Day 288), all cynomolgus monkeys remaining in the study (n=3 in the group given 15 mg/kg IV and n=4 in each of the groups given 50 mg/kg IV, 15 mg/kg SC, or 50 mg/kg SC) showed reversal of PD effects (return to baseline levels of CD45RA⁻ β7_{□}^{high} CD4⁺ lymphocytes).

Furthermore, PD effects appeared to be related to pharmacokinetics, because the observed reversibility and return to baseline levels of CD45RA⁻β7^{high} CD4⁺ lymphocytes correlated with clearance of rhuMAb Beta7 and the reversibility appeared to be dose dependent.

Variability between animals in the same dose groups in the rate of return of PD effects to predose levels correlated with changes in clearance of rhuMAb Beta7.

### 2. Safety, Toxicokinetics and Pharmacodynamics

The safety, toxicokinetics, and pharmacodynamics of rhuMAb Beta7 was evaluated when administered weekly via intravenous (IV) injection or subcutaneous (SC) injection to cynomolgus monkeys *(Macaca fascicularis)* for at least 12 weeks and to assess the reversibility, persistence, and delayed occurrence of any drug-related effects after a 15-week recovery. In addition, subsets of animals from the control, mid-, and high-dose groups were observed for an additional period before necropsy (during an extension phase) to assess the reversal of pharmacodynamic effects.

### 2.1 Test Material

rhuMAb Beta7, Lot M3-TOX165, was provided by Genentech as a clear liquid at a concentration of 150 mg/mL (1.3 mL per vial). Before its use in the study, the test agent was stored in a refrigerator set to maintain a temperature range of 2°C-8°C.

The vehicle, rhuMAb Beta7 Vehicle, Lot M3-TOX166, was provided by Genentech as a clear liquid in 25-mL vials. Before its use in the study, the vehicle was stored in a refrigerator set to maintain a temperature range of 2°C-8°C.

### 2.2 Species

Male and female naive cynomolgus monkeys (*Macaca fascicularis*), of Mauritius origin, were obtained from Covance Research Products, Inc. (Harrisburg, PA). The animals were acclimated for approximately 4 weeks before dosing began.

Forty-seven male and 47 female animals were initially acclimatized for possible use in this study. Data collected during acclimation (predose phase) was used to eliminate animals from consideration for study selection to produce minimal variation. Forty male and 40 female cynomolgus monkeys were assigned to the study using a computerized procedure designed to achieve body weight balance with respect to groups.

### 2.3 Study Design

This study comprised three phases: a 12-week dosing phase, a 15-week recovery phase, and an extension phase. The 12-week dosing phase ended at necropsy on Study Day 80, 2 days after the final dose of rhuMAb Beta7 was administered. The 15-week recovery phase followed the 12-week dosing phase and ended at necropsy on Study Day 184. Following the 15-week recovery phase, there was an additional period (an extension phase through Study Day 288) to assess the reversal of PD effects. The complete study design is summarized in Table 1.

**Table 1**

| Study Summary | | | | | |
|---|---|---|---|---|---|
| Group | Route of Administration | No./Sex^{a} | Dose Level^{b} (mg/kg/dose) | Dose Conc.^{b} (mg/mL) | Dose Volume^{b} (mL/kg/dose) |
| 1 (Control)^{c} | IV/SC | 7/M, 7/F | 0 | 0 | 0.33 |
| 2 (Low) | IV | 5/M, 5/F | 5 | 15 | 0.33 |
| 3 (Mid) | IV | 7/M, 7/F | 15 | 45 | 0.33 |
| 4 (High) | IV | 7/M, 7/F | 50 | 150 | 0.33 |
| 5 (Mid)^{d} | SC | 7/M, 7/F | 15 | 45 | 0.33 |
| 6 (High)^{d} | SC | 7/M, 7/F | 50 | 150 | 0.33 |

| | | | | | |
|---|---|---|---|---|---|
| IV=intravenous; SC=subcutaneous. ^{a}Following a 12-week dosing phase (2 days after the final dose), 3 animals/sex/group were euthanized. The remaining animals underwent a recovery phase. Following a 15-week recovery phase, 2 animals/sex/group were euthanized. Two animals/sex/group from Groups 4, 5, and 6, and 2 male and 1 female animal/group from Groups 1 and 3 were observed for an additional period before necropsy to assess the reversal of PD effects. PD endpoints were monitored during this extension phase and results were used to determine the timing of termination. ^{b}Animals were dosed on Study Days 1, 8, 15, 22, 29, 36, 43, 50, 57, 64, 71, and 78. ^{c}Animals in Group 1 received vehicle (control) by IV and SC injections. ^{d}Groups 5 and 6 received only SC injections of rhuMAb Beta7. | | | | | |

### 2.5 Dose Administration

Animals received 12 weekly IV injections via the saphenous vein or SC injection on Study Days 1, 8, 15, 22, 29, 36, 43, 50, 57, 64, 71, and 78. The injection sites of the SC doses were documented. Doses were determined based on the most recent recorded body weight. Animals were dosed at volumes of 0.33 mL per kg of body weight. IV injections were followed by a saline flush of approximately 1 mL.

These deviations were documented and did not affect the interpretation of PD results.

### 2.6 PD Blood Sample Collection

Blood samples (approximately 1 mL) for PD analysis were collected from each animal by venipuncture from the femoral vein once during the predose phase (approximately 6 days before dose initiation), before dosing on Study Day 1, on Study Day 2 (24 hours after the first dose), before dosing on Study Days 15, 29, 43, 57, 71, and 78, and on Study Day 80 before necropsy. During the 15-week recovery phase (R), samples were collected on Study Days 92 (R13), 106 (R27), 120 (R41), 134 (R55), 148 (R69), 162 (R83), and 184 (R105). During the extension phase, blood samples were collected from all surviving animals every other week, starting on Study Day 190 (R111) until and including the day of the final necropsy (Study Day 288), to assess the reversal of PD effects.

### 2.7 PD Blood Sample Processing

The samples for PD analysis were packed on refrigerated gel packs to maintain a temperature range of approximately 2°C-15°C for at least 72 hours and shipped to Genentech on the day of collection.

### 2.8 PD Flow Cytometry Assays

Three subsets of peripheral blood CD4⁺ lymphocytes were evaluated by flow cytometry as exploratory PD biomarkers: CD45RA⁻ β7_{⊐}^{high} cells (phenotypically similar to human memory/effector CD4⁺ lymphocytes that home preferentially to intestinal lymph nodes and tissues), CD45RA⁻ β7_{⊐}^{low} cells (phenotypically similar to human memory/effector CD4⁺ lymphocytes that home preferentially to peripheral lymph nodes and tissues), and CD45RA⁺ β7_{□}^{intermediate} cells (phenotypically similar to human naive CD4⁺ lymphocytes that localize non-preferentially to intestinal and peripheral lymph nodes and tissues) *(Rott et al.* 1996; Rott *et al.* 1997; Williams *et al.* 1998; Rose *et al.* 1998; Williams and Butcher 1997; Butcher *et al.* 1999). A representative flow cytometry plot of the CD4⁺ lymphocyte subsets in cynomolgus monkeys is presented in Figure 1. Subsets of peripheral blood CD8⁺lymphocytes (CD45RA⁻β7_{⊐}^{high}, CD45RA-β7_{□}^{low}, and CD45RA⁺ β7_{□} intermediate CD8⁺ lymphocytes) were examined in a similar manner, but were not used in PD evaluations.

### 2.8.1 Expression Assay: Evaluation of β7-Expressing Lymphocyte Subsets in Peripheral Blood

CD45RA⁻ β7_{□}^{high}□ □CD45RA⁻ β7_{□}^{low}, and CD45RA⁺ β7_{□}^{intennediate}□CD4⁺ or CD8⁺ lymphocytes were evaluated by flow cytometry using a non-blocking anti-β7 antibody (9D8) that binds β7 in the presence of rhuMAb Beta7. The panel configuration for the analysis of whole blood samples is presented in Table 2. All antibodies were purchased from BD Biosciences (BD; San Jose, CA) with the exception of streptavidin-allophycocyanin (SA-APC), which was purchased from Invitrogen Corp. (Carlsbad, CA) and 9D8-biotin, which was provided by Genentech.

**Table 2**

| Whole Blood Panel Configuration to Measure β7-Expressing Lymphocytes | | |
|---|---|---|
| Tube Antigen Markers and Fluorochromes Cell Types Identified | | |
| 1 | IgG-FITC, IgG-PE, CD4-PerCP-Cy5.5, SA-APC | Isotype used for gating population on SSC and FSC and for setting quadrant markers |
| 2 | CD45RA-FITC, CD49d-PE, CD4-PerCP-Cy5.5, 9D8-Biotin/SA-APC^{a} | α4- and β7-expressing cell subsets of CD4⁺ lymphocytes |
| 3 | CD45RA-FITC, CD49d-PE, CD8-PerCP-Cy5.5, 9D8-Biotin/SA-APC^{a} | α4- and β7-expressing cell subsets of CD8⁺ lymphocytes |

| | | |
|---|---|---|
| APC=allophycocyanin; FITC=fluorescein isothiocyanate; FSC=forward scatter; IgG=immunoglobulin G; PE=phycoerytherin; PerCP-Cy5.5=peridinin chlorophyll protein-cyanine-5.5; SA=streptavidin; SSC=side scatter. ^{a}9D8-biotin was incubated first, before SA-APC was added. | | |

For all whole blood samples, the flow cytometry assay was carried out according to Procedure 55656-43. For each sample, Tubes 2 and 3 were first incubated with a saturating concentration of 9D8-biotin. Instrument set-up control tubes (isotype control and single stains) and assay tubes (Tubes 1, 2, and 3) were prepared manually. After the first incubation, 2 mL of lyse solution was added to lyse red cells, followed by one cycle of cell wash. For the second incubation, samples (Tubes 2 and 3) were incubated with saturating concentrations of fluorescence-conjugated monoclonal antibodies either to CD4 (Clone L200) (Tube 2) or CD8 (Clone SK1) (Tube 3), along with CD45RA (Clone 5H9) and CD49d (Clone 9F10), or isotype controls antibodies (Tube 1). Each of the antibodies stated above, with the exception of the isotype control antibodies, cross-react with cynomolgus monkey leukocytes. SA-APC was also added to Tubes 1, 2, and 3 to detect biotinylated antibodies. Incubations were performed in the dark on wet ice for 30 + 5 minutes. A series of washing steps was performed and then cells were resuspended in fixative buffer. Samples were held at 2°C□4°C for a minimum of 30 minutes and acquired on the same day.

Flow cytometric acquisition was performed on a FACSCalibur^{®} (BD, serial number E2020). Twenty-five thousand lymphocyte events were acquired using a forward-scatter (FSC)/side-scatter (SSC) plot. Data analysis was based on FSC/SSC-gated lymphocytes and either CD4⁺/SSC^{low}-gated lymphocytes or CD8⁺/SSC^{low}-gated lymphocytes using CellQuest Pro software (BD CellQuest Pro, version 5.2.1). Flow cytograms were generated to establish the fraction of cells positive for each cell surface marker.

The □β7□ sub-populations were analyzed as percentage of gated CD4⁺ cells or gated CD8⁺ cells, and separately, as geometric mean fluorescence intensity (GMFI). To analyze the relative levels of β7 on the cell surface of CD4⁺ cells and CD8⁺ cells, molecules of equivalent soluble fluorescence (MOEF) were calculated by multiplying the GMFI of each population with a standard curve generated using SpheroTech Beads (Spherotech, Inc.; Lake Forest, IL). The GMFI and percentage of gated cells were monitored throughout the study to examine the effect of the test article on each of the three CD4⁺ and CD8⁺ lymphocyte subsets. The absolute cell count was calculated for each sample at each timepoint, based on the lymphocyte count per microliter of peripheral blood (provided by Covance).

### 2.8.2 Occupancy Assay: Available CD45RA^{⊐}β7^{high} Lymphocytes in Peripheral Blood

Occupancy of β7 on CD45RA-β7_{□}^{high} CD4⁺ lymphocytes and CD45RA-β7_{□}^{high} CD8⁺ lymphocytes was measured using a blocking anti-β7 antibody (fluorescently-labeled test article, rhuMAb Beta7) that does not bind in the presence of saturating concentrations of rhuMAb Beta7. Fluorescence-conjugated Herceptin^{®}, a humanized IgG1 antibody, was used as an isotype control for rhuMAb Beta7.

The panel configuration for the analysis of whole blood samples is presented in Table 3. All antibodies were purchased from BD, with the exception of Herceptin-Alexa 647 and rhuMAb Beta7-Alexa 647, which were provided by Genentech.

**Table 3**

| Whole Blood Panel Configuration to Measure Occupancy of β7 on Lymphocytes | | |
|---|---|---|
| Tube Antigen Markers and Fluorochrome Characteristics of Sample | | |
| 1 | CD4-FITC, CD45RA-PE, CD8PerCP, Her-Alx647 | Her-Alx647 used as isotype control for rhuMAb Beta7 |
| 2 | CD4-FITC, CD45RA-PE, CD8PerCP, rhuMAb Beta7-Alx647 | Unsaturated, to detect β7 occupancy |
| 3 | CD4-FITC, CD45RA-PE, CD8PerCP, rhuMAb Beta7-Alx647 | Saturated with 10 µg/mL rhuMAb Beta7 |

| | | |
|---|---|---|
| Alx647=Alexa 647; FITC=fluorescein isothiocyanate; Her=Herceptin; PE=phycoerytherin; PerCP=peridinin chlorophyll protein. | | |

Blood samples were stained according to Procedure 55656-50, with saturating concentrations of the following antibody conjugates: CD4 conjugated to fluorescein isothiocyanate (FITC; clone M-T477), CD45RA conjugated to phycoerythrin (PE; clone 5H9), CD8 conjugated to PerCP (clone SK1), and Herceptin conjugated to Alexa-647 or rhuMAb Beta7 conjugated to Alexa 647. In Tube 3, rhuMAb Beta7 was added to peripheral blood samples at saturating concentrations (10 µg/mL).

All incubations were performed on ice in the dark for 30-35 minutes. After staining, red blood cells were lysed, samples were washed, and cells were resuspended in fixative buffer using a Lyse Wash Assistant (BD). Twenty-five thousand gated lymphocyte events were acquired using a FSC/SSC gate on the BD FACSCalibur.

Lymphocytes were identified from a SSC/FSC scattergram. CD4^{□} and CD8^{□} cellos were then identified using a SSC/CD4 FITC or a SSC/CD8 PerCP plot, respectively. Tube 1 was used as negative control for gating. The CD45RA^{⊐} β 7^{high}, CD45RA^{⊐} β^{low}, and CD45RA^{⊐} β7^{intermediate} CD4^{□} cells or CD8^{□} cells were identified for the tubes with and without the saturating concentration of rhuMAb Beta7. Figure 1 shows CD4 □ lymphocyte subsets in cynomolgus monkey peripheral blood.

### 2.9 PD Data Analyses

Absolute counts as percentage of baseline levels at predose (Abs counts, % BL) were calculated for each of the CD4⁺ lymphocyte subsets (CD45RA- β7^{high}, CD45RA- β7^{low}, and CD45RA⁺ β7^{intermediate}) and CD8⁺lymphocyte subsets (CD45RA- β7^{high}, CD45RA⁻ β7^{low}, and CD45RA⁺ β7^{intermediate}) at each timepoint in the samples collected from animals given vehicle or rhuMAb Beta7. The mean values of individual absolute counts, as a percentage of baseline, were determined for each group. For females, two predose values were used to calculate baseline values for all flow cytometry evaluations. For males, because of technical difficulties (instrument failure), only one predose value (from Study Day 1) was used to calculate baseline values for expression assays with the 9D8 non-blocking antibody. Gated values for total CD4⁺ lymphocytes and total CD8⁺ lymphocytes obtained (as percentages) from flow cytometry analysis with the blood panel configurations shown in Table 3 were used to calculate absolute counts for total CD4⁺ lymphocytes and total CD8⁺ lymphocytes, respectively, in both occupancy and expression assays. Figure 1 shows CD+ lymphocyte subsets in cynomolgus monkey peripheral blood. The Definitions of values determined by flow cytometry analysis are as follows:
Abs counts: absolute counts for each respective subset of lymphocytes; equal to the absolute lymphocyte counts (a value obtained from hematology measurements expressed as lymphocytes per microliter of peripheral blood) times the percentage of gated lymphocytes for each subset (obtained from flow cytometry analysis).
Abs counts (% BL): absolute counts as a percentage of baseline Abs counts obtained before dosing; calculated as Abs counts divided by average of predose absolute counts.
MOEF: molecules of equivalent fluorescence; calculated using the slope of the FL4 channel times the geometric mean fluorescence intensity
MOEF (% predose): MOEF as a percentage of MOEF before dosing; calculated as the MOEF at the respective timepoint after dosing divided by the average MOEF before dosing.

### 3. Results and Discussion

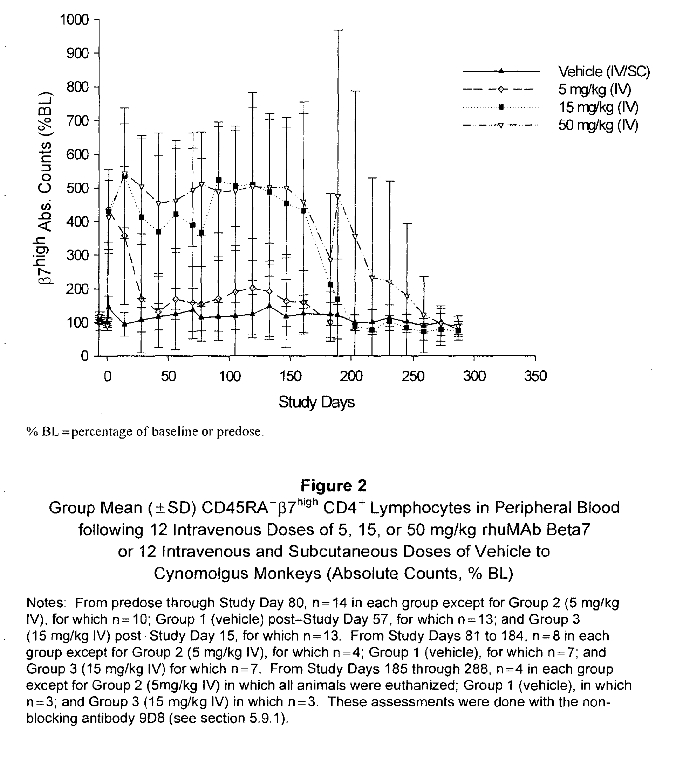

Group mean (± SD) and individual cynomolgus monkey absolute counts (% BL) for subsets of peripheral blood CD4⁺ lymphocytes assessed with non-blocking 9D8 antibodies are presented in Figures 2□-7.

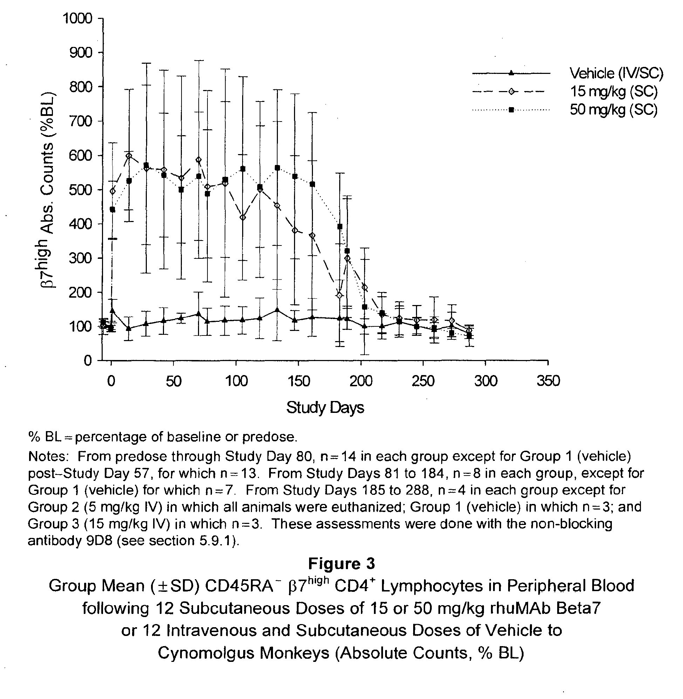

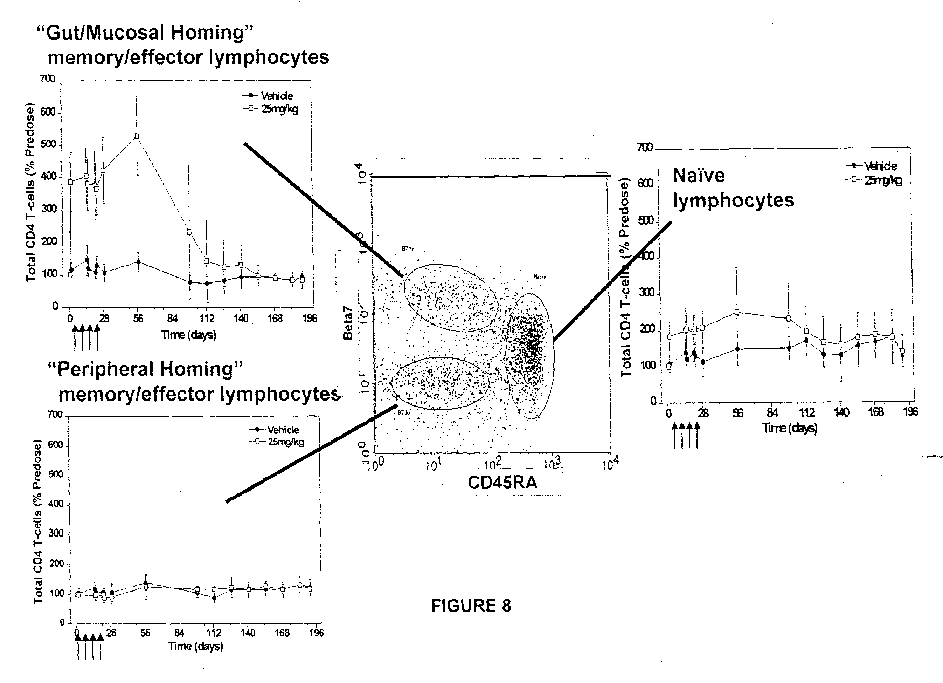

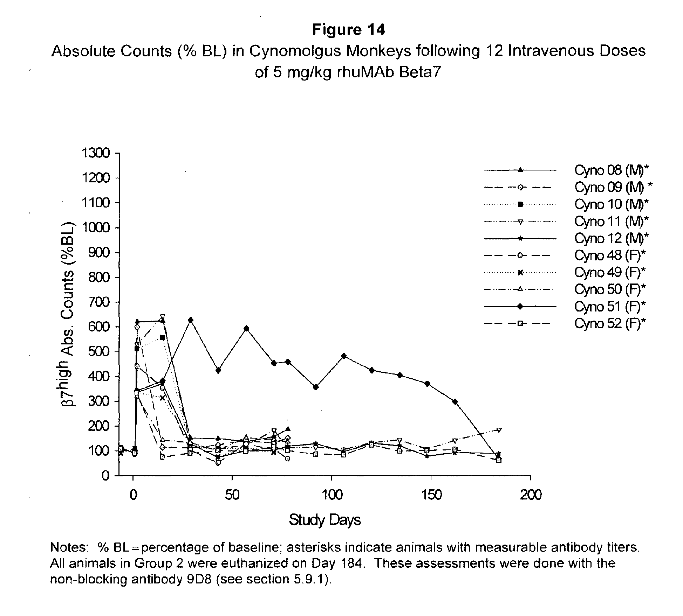

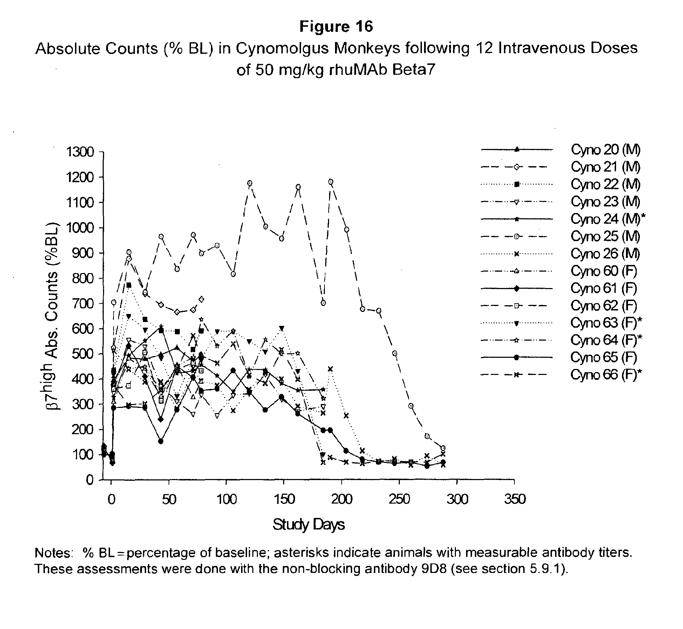

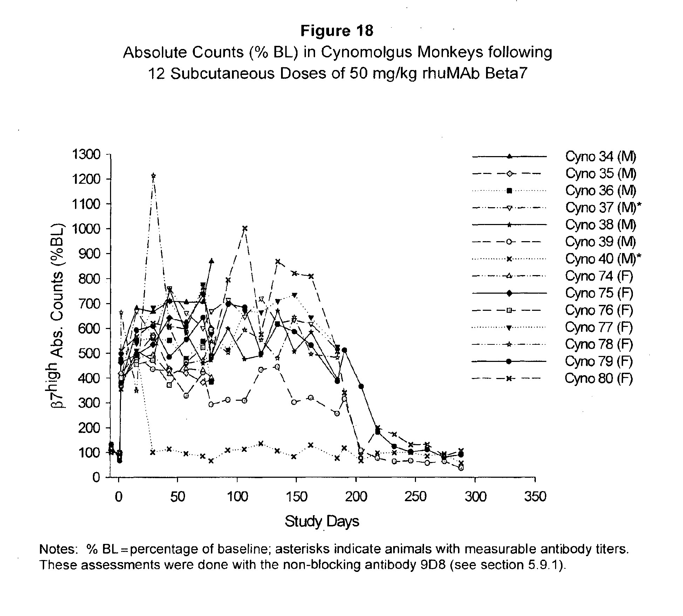

Group mean numbers of CD45RA-β7^{high} CD4⁺ lymphocytes increased approximately 4- to 5-fold over baseline levels following IV administration of 5 mg/kg rhuMAb Beta7 (see Figure 2 ). Administration of 15 mg/kg or 50 mg/kg rhuMAb Beta7 (IV or SC) resulted in slightly greater increases (approximately 5- to 6-fold) in group mean numbers of CD45RA⁻β7^{high} CD4⁺ lymphocytes, compared with baseline levels (see Figures 2 and 3 ). In general, the majority of the animals given15 or 50 mg/kg (IV or SC) had sustained PD effects during the dosing phase, unlike the majority of animals in the 5 mg/kg dose group ( , Figures 14-18). Animals given vehicle showed no substantial changes in group mean numbers of CD45RA⁻ β7^{high} CD4⁺ lymphocytes (see Figures 2 and 3 ). Group mean numbers of CD45RA⁻β7^{high} CD₄⁺ lymphocytes increased approximately 4- to 6-fold over baseline levels following IV administration of 25 mg/kg rhuMAb Beta7 (see Figure 8).

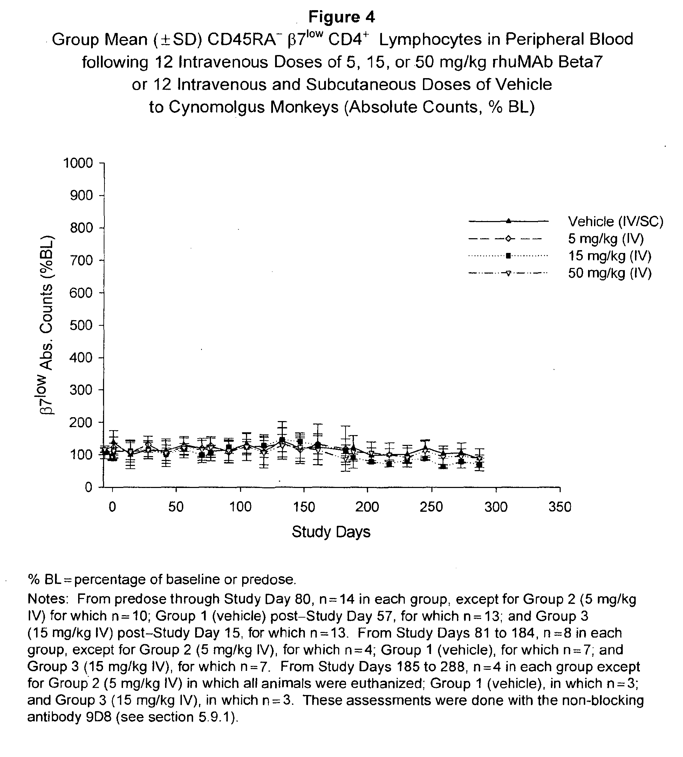

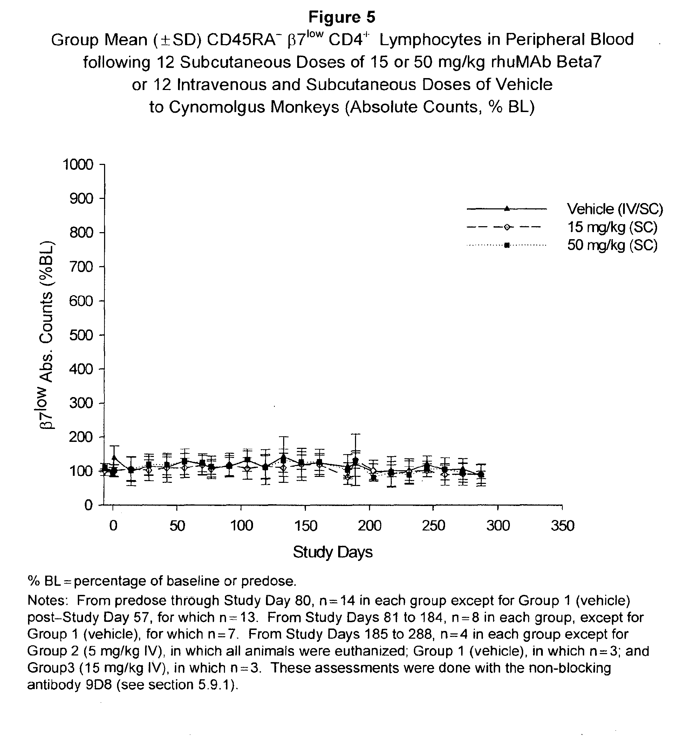

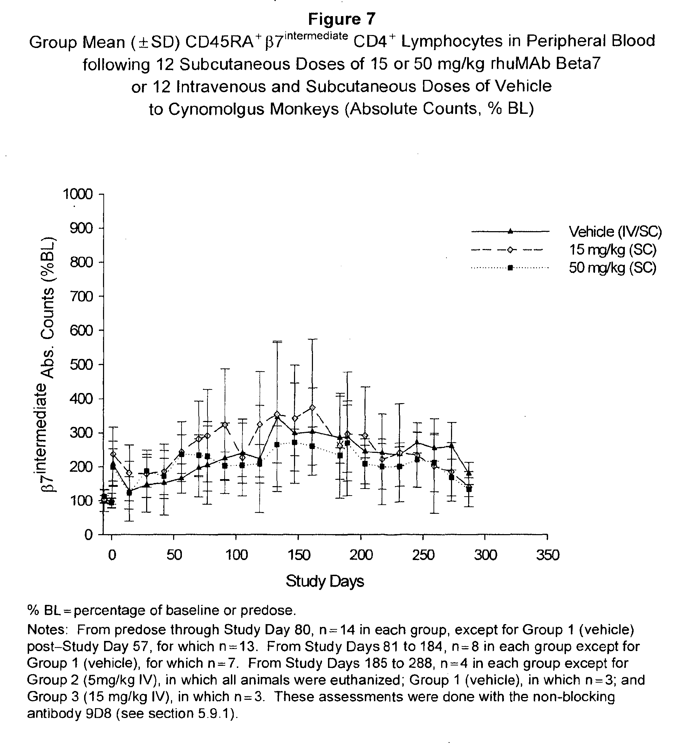

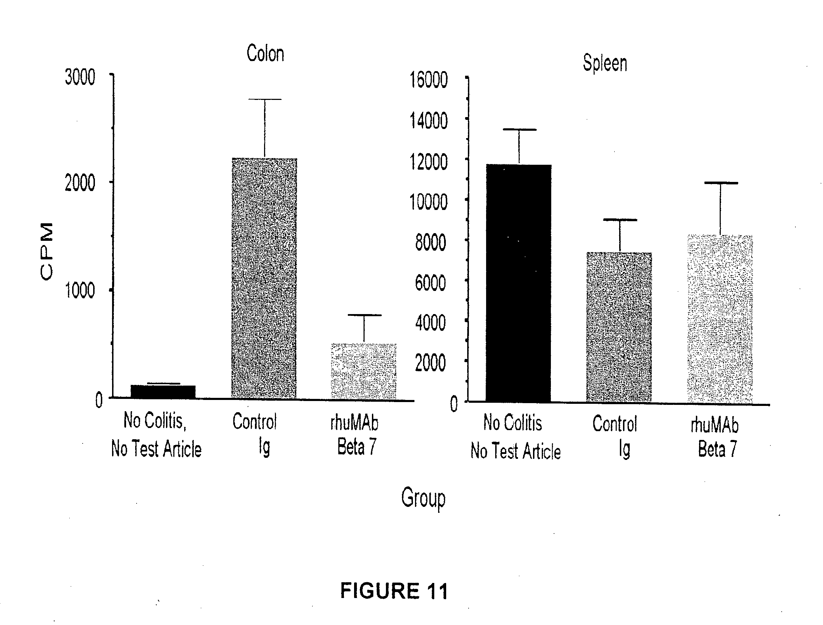

These findings are consistent with the proposed mechanism of action of rhuMAb Beta7: inhibition of trafficking of CD45RA⁻ β7_{□}^{high} lymphocytes to the gut, leading to accumulation of these cells in the peripheral circulation (see Figure 11). Also in support of the proposed mechanism of action, there were no substantial differences in group mean levels of CD45RA⁺β7^{intermediate}CD4⁺ lymphocytes or CD45RA-β7^{low} CD4⁺ lymphocytes in animals given vehicle compared with those given rhuMAb Beta7 (see Figures 4-8).

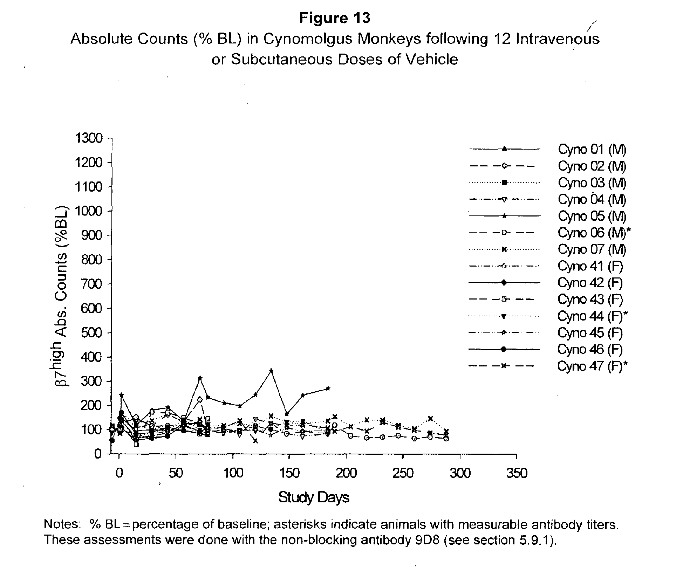

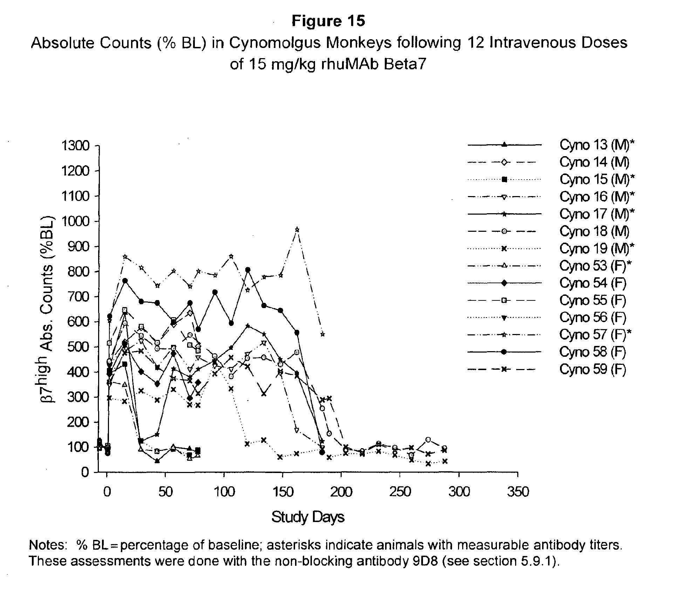

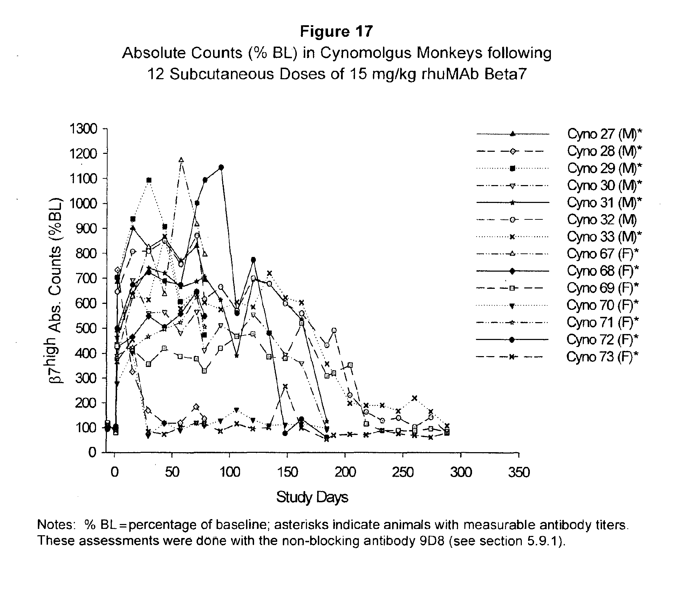

By the end of the 15-week recovery phase (Study Day 184), the numbers of peripheral blood CD45RA⁻β7^{high} CD4⁺ lymphocytes returned to baseline levels in 10 of 10 animals in the group given IV doses of 5 mg/kg rhuMAb Beta7 (see Figure 14). In groups given IV doses of 15 mg/kg and 50 mg/kg, the numbers of peripheral blood CD45RA-β7^{high} CD4⁺ lymphocytes returned to baseline in 7 of 13 and 2 of 14 animals, respectively (see Figures 15-16 ). In animals given SC doses of 15 mg/kg and 50 mg/kg, the numbers of CD45RA⁻ β7^{high}CD4⁺ lymphocytes returned to predose levels in 6 of 14 and 1 of 14 animals, respectively (see , Figures 17-18 ). Thus, the return to baseline of the observed PD effects appeared to be dose dependent. There were no substantial changes in numbers of CD45RA⁻ β7^{high} CD4⁺ lymphocytes in the peripheral blood of animals given vehicle, with the exception of 1 animal, which showed slight variability (see Figure 13 ).

By the end of the PD extension phase (Study Day 288), all cynomolgus monkeys dosed with rhuMAb Beta7 that remained in the study (n=3 in the 15 mg/kg IV dose group; n=4/group in the 50 mg/kg IV, 15 mg/kg SC, and 50 mg/kg SC dose groups) showed reversal of PD effects (a return to baseline levels of CD45RA- β7^{high} CD4⁺ lymphocytes; see Figures 15-18). Subsets of peripheral-blood CD8⁺ lymphocytes (CD45RA- β7_{□}^{high}, CD45RA⁻β7_{□}^{low}, and CD45RA⁺ β7_{□}^{intermediate} CD8⁺ lymphocytes) were also examined and similar results were observed,

Relative levels of β7 expression on peripheral blood CD4⁺ lymphocytes were examined as MOEF. There was no evidence of downmodulation of β7 on peripheral blood CD4⁺ lymphocytes in cynomolgus monkeys following IV doses of 5, 15, or 50 mg/kg rhuMAb Beta7 or SC doses of 15 or 50 mg/kg rhuMAb Beta7 (seeFigures 19-22).

In conclusion, the rate of return to baseline levels of CD45RA⁻β7^{high} CD4⁺ lymphocytes in peripheral blood appeared to be dose dependent in cynomolgus monkeys given rhuMAb Beta7. Also, the majority of the animals given 15 or 50 mg/kg (IV or SC) had sustained PD effects during the dosing phase, unlike the majority of animals in the 5 mg/kg dose group. Furthermore, the PD effects appeared to be related to pharmacokinetics, because the observed reversibility and return to baseline levels of CD45RA⁻ β7^{hig} CD4⁺ lymphocytes correlated with clearance of rhuMAb Beta7 and appeared to be dose dependent. Variability in the rate of return of PD effects to predose levels between animals in the same dose groups correlated with changes in clearance of rhuMAb Beta7. These changes in rhuMAb Beta7 clearance were generally associated with the presence of anti-therapeutic antibodies (ATAs) in some animals (see Figures 14-18; ).

### 7. Conclusions

Group mean numbers of CD45RA⁻β7^{high} CD4⁺ lymphocytes (phenotypically similar to gut-homing cells) increased approximately 4- to 6-fold, compared with predose levels, following IV or SC doses of 5, 15, or 50 mg/kg rhuMAb Beta7 in cynomolgus monkeys. In contrast, animals that were dosed with vehicle showed no substantial changes in group mean numbers of peripheral blood CD45PA⁻β7^{high} CD4⁺ lymphocytes through Study Day 184.

These findings are consistent with the mechanism of action of rhuMAb Beta7□inhibition of homing of β7-positive lymphocytes to the gut, through prevention of α4β7 binding to its ligands. This proposed mechanism is expected to lead to accumulation of CD45RA⁻ β7^{high} CD4⁺ lymphocytes in the peripheral circulation, which was observed in the study. Also in support of the proposed mechanism of action, there were no substantial differences in group mean levels of CD45RA⁺ β7^{intermediate}CD4⁺ lymphocytes (phenotypically similar to naïve CD4⁺ cells) or CD45RA⁻ β7^{low} CD4⁺ lymphocytes (phenotypically similar to peripheral-homing CD4⁺ cells) in cynomolgus monkeys dosed with vehicle, compared with animals given rhuMAb Beta7.

By the end of the 15-week recovery phase (on Study Day 184), the numbers of peripheral blood CD45RA⁻β7^{high} CD4⁺ lymphocytes returned to predose levels in all animals (10 of 10) in the group given IV doses of 5 mg/kg rhuMAb Beta7. In animals given 15 mg/kg or 50 mg/kg rhuMAb Beta7, numbers of peripheral blood CD45RA-β7^{high} CD4⁺ lymphocytes returned to predose levels in 7 of 13 and 2 of 14 animals in the IV dose groups (respectively) and in 6 of 14 and 1 of 14 animals in the SC dose groups (respectively). By the end of the PD extension phase (on Study Day 288) all cynomolgus monkeys dosed with rhuMAb Beta7 that remained in the study showed reversal of PD effects.

These findings indicate an apparent dose-dependency in the return to baseline levels of CD45RA⁻β7^{high}CD4⁺ lymphocytes in peripheral blood. Furthermore, the PD effects appeared to be related to pharmacokinetics, because the observed reversibility and return to baseline of the numbers of CD45RA⁻β7^{high} CD4⁺ lymphocytes correlated with clearance of rhuMAb Beta7. Variability in the rate of return of PD effects to predose levels between animals in the same dose groups correlated with changes in clearance of rhuMAb Beta7, which was associated with the presence of AT As in some animals.

### REFERENCES

1. Andrew DP, Berlin C, Honda S, et al. Distinct but overlapping epitopes are involved in α4β7-mediated adhesion to vascular cell adhesion molecule-1, mucosal adhesion molecule-1, fibronectin, and lymphocyte aggregation. J Immunol 1994;153:3847-61.
2. Butcher EC, Williams M, Youngman K, et al. Lymphocyte trafficking and regional immunity. Adv Immunol 1999;72:209-53.
3. Cepek KL, Parker CM, Madara JL, et al. Integrin αEβ7 mediates adhesion of T lymphocytes to epithelial cells. J Immunol 1993;150:3459-70.
4. Feagan BG, Greenberg GR, Wild G, et al. Treatment of ulcerative colitis with a humanized antibody to the α4 β 7 integrin. N Engl J Med 2005;352:2499-507.
5. Holzmann B, McIntyre BW, Weissman IL. Identification of a murine Peyer's patch-specific lymphocyte homing receptor as an integrin molecule with an α-chain homologous to human VLA-4-α. Cell 1989;56:37-46.
6. Hu MC, Crowe DT, Weissman IL, et al. Cloning and expression of mouse integrin βp(β7): a functional role in Peyer's patch-specific lymphocyte homing. Proc Natl Acad Sci U S A 1992;89:8254-8.
7. Rose JR, Williams MB, Rott LS, et al. Expression of the mucosal homing receptor α4β7 correlates with the ability of CD8+ memory T cells to clear rotavirus infection. J Virol 1998;72:726-30.
8. Rott LS, Briskin MJ, Andrew DP, et al. A fundamental subdivision of circulating lymphocytes defined by adhesion to mucosal addressin cell adhesion molecule-1. Comparison with vascular cell adhesion molecule-1 and correlation with β7 integrins and memory differentiation. J Immunol 1996;156(10):3727-36.
9. Rott LS, Rose JR, Bass D, et al. Expression of mucosal homing receptor α4β7 by circulating CD4+ cells with memory for intestinal rotavirus. J Clin Invest 1997;100:204-8.
10. Sandborn WJ, Colombel JF, Enns R, et al. Natalizumab induction and maintenance therapy for Crohn's disease. N Engl J Med 2005;353:1912-25.
11. Williams MB, Butcher EC. Homing of naive and memory T lymphocyte subsets to Peyer's patches, lymph nodes, and spleen. J Immunol 1997;159:1746-52.
12. Williams MB, Rose JR, Rott LS, et al. The memory B cell subset responsible for the secretory IgA response and protective humoral immunity to rotavirus expresses the intestinal homing receptor, α4β7. J Immunol 1998;161:4227-35.

### Example 2

### A Four-Week Pilot Safety and Pharmacokinetic Evaluation of rhuMAb β7 (PRO145223) Administered Weekly via Intravenous Injection to Cynomolgus Monkeys with Twenty-Three Week Recovery

### ABSTRACT

rhuMAb β7 (also referred to as PRO145223) is a humanized immunoglobulin G1 (IgG1) anti □human β7 antibody that binds to αEβ7 and α4β7 integrins on lymphocytes in humans and cynomolgus monkeys *(Macaca fascicularis).* Cells that express αEβ7and α4β7 integrins are thought to have a significant role in inflammatory diseases of the gut, such as ulcerative colitis (UC) and Crohn's disease (CD). rhuMAb β7 is being investigated as a potential therapeutic for patients with inflammatory bowel diseases such as CD and UC. The objectives of this study were to evaluate the safety, pharmacokinetic (PK), and pharmacodynamic (PD) effects of rhuMAb β7 following intravenous (IV) administration once weekly for 4 weeks to cynomolgus monkeys and to characterize the reversibility of any adverse drug-related effects during a 23-week recovery period. Groups of cynomolgus monkeys were given four weekly IV doses of vehicle or rhuMAb β7 at 5 or 25 mg/kg. Three male and 3 female animals from each dose group were necropsied 7 days after the final dose; the remaining animals (2/sex/group) from the vehicle and 25 mg/kg dose groups were observed for 23 weeks after the final dose before necropsy.

Overall, the serum concentration-time profile of rhuMAb β7 following four weekly IV doses was biphasic, with a fast initial distribution phase followed by a slower elimination phase. Following four weekly IV bolus doses of 5 or 25 mg/kg rhuMAb β7 to cynomolgus monkeys, the trough serum concentrations increased after the second dose except when anti-therapeutic antibodies (ATAs) were detected. The dose-normalized area under the serum concentration-time profile from Day 0 to Day 7 (AUC_{0□7}/dose) following the first dose was similar for the 5 mg/kg and 25 mg/kg dose groups, suggesting that rhuMAb β7exhibits dose-proportional pharmacokinetics in cynomolgus monkeys at the dose range studied. For the 25 mg/kg dose group, the AUC_{0□7} and maximum serum concentration (Cₘₐₓ) increased approximately 2-fold after the last dose, compared with the first dose. Both of these findings suggested that there was accumulation of rhuMAb β7 following four weekly IV doses of 25 mg/kg to cynomolgus monkeys; however, such accumulation is expected for an antibody with a long half-life at these doses. Compartmental analysis of the data from the 25 mg/kg recovery animals estimated a slow clearance of 2.93 mL/day/kg and a relatively long terminal half-life of 14.5 days. However, 3 of the 4 monkeys had ATAs that probably contributed to the faster clearance of rhuMAb β7.

Saturation of peripheral blood T cells and increases in the levels of circulating β7⁺ T cells were explored as PD markers. Integrin β7-receptor occupancy assays suggested that β7 receptors on peripheral-blood T-cell subsets were fully saturated following the first of four weekly doses of 5 or 25 mg/kg rhuMAb β7 to cynomolgus monkeys. The occupancy of β7 receptors on peripheral-blood T cells was maintained when serum concentrations were above approximately 1□10 µg/mL and the detection of available β7 correlated with a decrease in serum concentrations of rhuMAb β7. CD45RA^{□} β7^{high} T cells (phenotypically similar to gut-homing T cells in humans and mice) increased approximately 5-fold over baseline levels following four weekly doses of 25 mg/kg to cynomolgus monkeys. Likewise, CD45RA⁺β7^{intermediate} T cells (phenotypically similar to human naive T cells) also increased following four weekly doses of 25 mg/kg (approximately 2.5-fold over baseline levels). In contrast, the CD45RA^{□}β7^{low} T cells remained stable throughout the study. These findings are consistent with the mechanism of action of rhuMAb β7namely, the inhibition of trafficking of β7-positive lymphocytes to the gut through inhibition of α4β7 binding to its ligands, which then leads to the accumulation of these cells in the peripheral circulation. These phenomena will be further explored in future studies in cynomolgus monkeys.

### INTRODUCTION

rhuMAb β7 (also referred to as PRO145223) is a humanized immunoglobulin G1 (IgG1) anti-human β7 antibody that binds to αEβ7 and α4β7 integrins on lymphocytes in humans and non-human primates. rhuMAb β7 blocks interactions of α4β7 with MadCAM-1, VCAM-1, and fibronectin with affinities (50% maximal inhibitory concentration [IC₅₀]) of 0.07-0.1 nM, and also blocks E-cadherin binding to αEβ7 with an IC₅₀ of 5 nM. Cells that express αEβ7 and α4β7 integrins are thought to have a significant role in inflammatory diseases of the gut, such as Crohn's disease (CD) and ulcerative colitis (UC). rhuMAb β7 is being investigated as a potential therapeutic for patients with inflammatory bowel diseases such as CD and UC.

### OBJECTIVES

The objectives of this study were to evaluate the safety, pharmacokinetic (PK), and pharmacodynamic (PD) effects of rhuMAb β7 following intravenous (IV) administration once weekly for 4 weeks to cynomolgus monkeys *(Macaca fascicularis)* and to characterize the reversibility of any adverse test article-related effects during a 23-week recovery period. The objective of this study is to report the results of the PK and flow cytometry analyses.

### MATERIALS AND METHODS

### TEST MATERIAL

rhuMAb β7 (PRO145223), Lot M3-TOX132, was provided by Genentech as a clear to slightly opalescent, colorless to slightly yellow liquid at a concentration of 20 mg/mL (4.5 mL/vial). rhuMAb β7 Vehicle, Lot M3-TOX133, was also provided as a clear to slightly opalescent, colorless to slightly yellow liquid (10.5 mL/vial). Before being used in the study, test articles were stored in a refrigerator set to maintain a temperature range of 2°C-8°C.

### SPECIES

Twenty-six test article-naive cynomolgus monkeys were obtained from the SNBL USA stock colony. The animals weighed 2-6 kg and were 2-5 years old at the initiation of treatment. The animals were acclimated for at least 1 week before the initiation of treatment. Only animals that appeared to be healthy and that were free of obvious abnormalities were used for the study.

### STUDY DESIGN

The animals were randomized into one of three groups and received four weekly IV doses of the test material. Animals in Group 1 (5 males and 5 females) received rhuMAb β7 Vehicle. Animals in Group 2 (3 males and 3 females) and Group 3 (5 males and 5 females) received rhuMAb β7 at 5 and 25 mg/kg, respectively. Three males and 3 females from each group had terminal necropsies 7 days after the last dose. Two males and 2 females from the groups administered vehicle or 25 mg/kg rhuMAb β7 were followed for a recovery period of approximately 23 weeks before a recovery necropsy. The study design is summarized in Table 2_1.

**Table 2_1**

| Study Design | | | | | | |
|---|---|---|---|---|---|---|
| Group | No./Sex | Route | Test Material | Dose Level (mg/kg) | Dose Conc. (mg/mL) | Dose Volume^{a} (mL/kg) |
| 1 | 5/M, 5/F | IV | rhuMAb β7 Vehicle | 0 | 0 | 1.25 |
| 2 | 3/M, 3/F | IV | rhuMAb β7 | 5 | 20 | 0.25 |
| 3 | 5/M, 5/F | IV | rhuMAb β7 | 25 | 20 | 1.25 |

| | | | | | | |
|---|---|---|---|---|---|---|
| Conc.=concentration; IV=intravenous. Note: Three males and 3 females from each group were designated as terminal necropsy animals. The remaining animals from Groups 1 and 3 were designated as recovery necropsy animals. ^{a} Dose volume was calculated based on the most recent body weight. | | | | | | |

### DOSE PREPARATION

The test article for each group was provided at the appropriate concentration by Genentech.

### DOSE ADMINISTRATION

Individual dose volumes were calculated based on body weights that were recorded on the day of drug administration. Animals were given test material intravenously via a saphenous vein. Immediately following dose administration, but before the needle was removed from the animal, the dosing apparatus was flushed with approximately 1 mL of 0.9% NaCl.

### BLOOD SAMPLE COLLECTION

All blood samples were collected using a needle and syringe via a saphenous, femoral, or cephalic vein.

### PK Analysis

Blood (approximately 1.2 mL) was collected from each animal and serum was harvested at the following timepoints for PK analysis:
- Predose on Study Day 1 and 15 minutes and 6, 24, and 72 hours post-dose
- Predose on Study Days 8 and 15
- Predose on Study Day 22 and 15 minutes and 6, 24, and 72 hours after the last dose
- Study Days 36, 43, 50, 57, 64, 71, 78, 85, 92, 99, 106, 113, 120, 127, 134, 141, 148, 155, 162, 169, 176, and 183
- On day of terminal and recovery necropsies (Study Days 29 and 191)

However, for PK analysis, timepoints were converted to start at Day 0 and are referred to in this report as Days 0, 0.0104, 0.25, 1, 3, 7, 14, 21, 21.0104, 21.25, 22, 24, 28, 35, 42, 49, 56, 63, 70, 77, 84, 91, 98, 105, 112, 119, 126, 133, 140, 147, 154, 161, 168, 175, 182, and 190.

### Anti-Therapeutic Antibody Analysis

The same serum samples used for PK analysis were used for anti-therapeutic antibody (ATA) analysis. ATA response was analyzed at the following timepoints:
- Predose on Study Days 1, 8, 15, and 22
- Study Days 50, 71, 92, 113, 134, 155, and 176
- On day of terminal and recovery necropsies (Study Days 29 and 191)

However, for ATA analysis, timepoints were converted to start at Day 0 and are referred to in this report as Days 0, 7, 14, 21, 28, 49, 70, 91, 112, 133, 154, 175, and 190.

### Flow Cytometry Analysis

Blood (approximately 1.5 mL) was collected from each animal at the following timepoints for the total lymphocyte (B cells, T cells, and Natural Killer [NK] cells) flow cytometry assay:
- Day 8 (predose)
- Predose on Day 1 and 24 hours after the first dose
- Predose on Day 15 and 24 hours after the second dose
- Predose on Day 22 and 24 hours after the third dose
- Days 43, 57, 71, 85, 99, 113, 127, 141, 155, 169, and 183
- On day of terminal and recovery necropsies (Study Days 29 and 191)

Blood (approximately 1.5 mL) was collected from each animal at the following timepoints for the occupancy flow cytometry assay:
- Predose on Day 1 and 24 hours after the first dose
- Predose on Day 15 and 24 hours after the second dose
- Predose on Day 22 and 24 hours after the third dose
- Days 57, 85, 99, 113, 127, 141, 155, 169, and 183
- On day of terminal and recovery necropsies (Study Days 29 and 191)

For the total occupied and unoccupied β7-expressing CD4⁺ and CD8⁺ T-cell analysis, blood (approximately 1.5 mL) was collected from each animal at the following timepoints:
- Predose on Day 1 and 24 hours after the first dose
- Predose on Day 15 and 24 hours after the second dose
- Predose on Day 22 and 24 hours after the third dose
- Days 57, 99, 113, 127, 141, 155, 169, and 183
- On day of terminal and recovery necropsies (Study Days 29 and 191)

### BLOOD SAMPLE PROCESSING

### PK and ATA Analysis

Immediately after collection, samples were transferred into serum separator tubes and allowed to clot at room temperature for 30 to 80 minutes. The samples were centrifuged at 2000 µg for 15 minutes at room temperature. The serum was harvested and transferred to labeled 1.5-mL Eppendorf tubes.

### Flow Cytometric Analysis

Immediately after collection, whole blood samples for flow cytometric analysis were transferred into sodium-heparinized tubes at SNBL according to the study protocol and shipped to Genentech for analysis. Samples were placed immediately on wet ice or stored at a temperature range of 2°C to □8°C until analyzed by flow cytometry.

### ASSAYS

### Serum Concentrations

Serum samples were analyzed by quantitative ELISA to measure total rhuMAb β7 levels. This assay was run according to the Notebook 47886-27 draft assay development summary and the Notebook 47886-22 draft standard operating procedure (SOP) for all samples. First, 96-well microtiter plates were coated with sheep anti □ human IgG heavy and light chain (H&L), pre-absorbed with monkey serum. This antibody was obtained from The Binding Site (Birmingham, UK; Catalog AU003.CUS01; Lot 086001) and diluted to 1 µg/mL in 0.05 M sodium carbonate, pH 9.6. Recombinant human rhuMAb β7 (Lot 46994-8, produced at Genentech) was used as the standard. The standard curve ranged from 0.781 to 50.0 ng/mL. Antibodies used to determine the standard curve, matrix controls, and samples were diluted in assay diluent (phosphate-buffered saline [PBS], 0.5%, bovine serum albumin [BSA], 0.05% polysorbate 20, 0.05% Proclin 300, 0.25% CHAPS buffer, 5 mM ethylenediaminetetraacetic acid [EDTA], 0.35M NaCl). Samples were diluted by a minimum of 1/20. Horseradish peroxidase (HRP) □tagged sheep anti □ human IgG (H&L, pre-absorbed with monkey serum) was obtained from The Binding Site (Catalog CUS1684.H; Lot 11245) and was used as the conjugate. The signal was generated using tetramethyl benzidine peroxidase as substrate for HRP. Data for controls and samples were corrected for dilution; values for each dilution were averaged. The minimum quantifiable concentration for neat cynomolgus monkey serum was determined to be 20 ng/mL, based on the assay limit of 1 ng/mL and the minimum dilution of 1/20.

### Determining ATA Response

Serum samples from cynomolgus monkeys were analyzed by a bridging electrochemiluminescence assay to determine the level of antibody response against rhuMAb β7. This assay was run according to the Notebook 47886-48 draft assay development summary for antibodies against rhuMAb β7. Polyclonal antibodies directed against human IgG (H&L, pre-absorbed with monkey serum) were obtained from The Binding Site and were used as surrogate positive control. Controls and samples were diluted into sample diluent (HEPES buffer, 2% fish gelatin, 0.05% polysorbate 20, 0.05 ProClin300, 0.15M NaCl, 10% fetal bovine serum) and added to a 96-well, round-bottom polypropylene plate. The samples were diluted by a minimum of 1/10. Biotinylated rhuMAb β7 and BV-tagged rhuMAb β7 were used as conjugates. The next day, streptavidin-coated Dynabeads M280 (Invitrogen Corp.; Carlsbad, CA) were diluted in assay diluent (HEPES buffer, 2% fish gelatin, 0.5% polysorbate 20, 0.05% ProClin300, 0.15 M NaCl) and added to the plates. Assay response was measured in electrochemiluminescence units. The cutpoint was set based on the value of the negative control, and was used to calculate the titer values for the samples and positive controls. The minimum reporting titer for the rhuMAb β7 antibody assay is 1.0.

### Flow Cytometry Analysis

### Total β7-expressing CD4⁺ and CD8⁺ T-Cell Subsets in Peripheral Blood

The total β7-expressing CD4⁺ and CD8⁺ T cells were measured using 9D8, an anti-β7 antibody targeting a different epitope than rhuMAb β7. The panel configuration for the analysis of whole blood samples is presented in Table 2-2.

**Table 2-2**

| Whole Blood Panel Configuration | | |
|---|---|---|
| Tube No. | Antigen Markers: Fluorochrome | Cell Types Identified |
| 1 | IgG-FITC, IgG-PE, CD4-PerCP, SA-APC | Isotype used for gating population on SSC and FSC. Setting quadrant markers. |
| 2 | CD45RA-FITC, CD49d-PE, CD4-PerCP or CD4-PerCP-Cy5.5, 9D8-Biotin/SA-APC^{a}, | α4 and β7 expressing cell subsets of CD4 T cells |
| 3 | CD45RA-FITC, CD49d-PE, CD8-PerCP or CD8-PerCP-Cy5.5, 9D8-Biotin/SA-APC^{a}, | α4 and β7 expressing cell subsets of CD8 T cells |

| | | |
|---|---|---|
| FSC=forward scatter; SA=streptavidin; SSC=side scatter. ^{a} 9D8-biotin was incubated first before SA-APC was added. | | |

For all whole blood samples, the flow cytometry assay was carried out according to Procedures 48038-29 and 48038-84. Tubes 2 and 3 for each sample were first incubated with saturating concentrations of 9D8-biotin, an anti-β7 antibody that binds β 7 in the presence of rhuMAb β7. Instrument set-up control tubes (isotype control and single stains) and assay tubes (Nos. 1, 2, and 3) were prepared manually. After the first incubation, 2 mL of lyse solution was added to lyse red cells followed by one cycle of cell wash. For the second incubation, samples (Tubes 2 and 3) were incubated with saturating concentrations of fluorescently conjugated monoclonal antibodies to CD4 (Clone M-T477), CD8 (Clone SK1), CD45RA (Clone 5H9), and CD49d (Clone 9F10), which are known to cross-react with cynomolgus monkey leukocytes, or isotype controls antibodies (Tube 1). Streptavidin-APC (SA-APC) was also added to Tubes 1, 2, and 3 to detect biotinylated antibodies. Both incubations were performed in the dark on wet ice for 60 □ 5 minutes. A series of washing steps was performed with a final step of resuspending the cells in fixative buffer. Samples were held at 2°C to 4°C for a minimum of 30 minutes and acquired on the same day.

Flow cytometric acquisition was performed on a FACSCalibur™ (BD Biosciences; San Jose, CA) with serial number E3982. Twenty-five thousand lymphocyte events were acquired using a forward-scatter (FSC)/side-scatter (SSC) plot. Data analysis was based on both FSC/SSC gated lymphocytes and, depending on the cocktail combination, CD4⁺/SSC^{low}- or CD8⁺/SSC^{low}-gated T cells using CellQuest Pro software (BD CellQuest Pro, version 5.2). Flow cytograms were generated to establish the fraction of cells positive for each cell surface marker.

β7 sub-populations were analyzed as percent of gated T cells, and separately, as geometric mean fluorescence intensity (GMFI). For each sample at each timepoint, molecules of equivalent soluble fluorescence (MOEF) were calculated by multiplying the GMFI of each population with a standard curve, generated by the SpheroTech Beads standard. GMFI and percentage of gated cells for CD4⁺ or CD8⁺ T-lymphocytes population-expressing CD49d⁺9D8^{inf}, CD45RA⁻9D8^{high}, CD45RA⁻9D8^{low}, CD45RA⁺, and CD45RA- were monitored throughout the study to examine the effect of drug on each subset. For each sample at each timepoint for each lymphocytes subset, the absolute cell count was calculated based on the lymphocyte count per µL of peripheral blood provided by SNBL.

β7 □ sub-populations were analyzed as percent of gated T cells, and separately, as geometric mean fluorescence intensity (GMFI). For each sample at each timepoint, molecules of equivalent soluble fluorescence (MOEF) were calculated by multiplying the GMFI of each population with a standard curve, generated by the SpheroTech Beads standard. GMFI and percentage of gated cells for CD4⁺ or CD8⁺ T-lymphocytes population expressing CD49d⁺9D8^{inf}, CD45RA^{□}9D8^{high}, CD45RA^{□}9D8^{low}, CD45RA⁺, and CD45RA^{⊐} were monitored throughout the study to examine the effect of drug on each subset. For each sample at each timepoint for each lymphocytes subset, the absolute cell count was calculated based on the lymphocyte count per µL of peripheral blood provided by SNBL.

### Occupancy of β7 on CD4⁺ and CD8⁺ T-Cell Subsets in Peripheral Blood

The panel configuration for the analysis of whole blood samples is presented in Table 2-3.

**Table 2-3**

| Whole Blood Panel Configuration | | |
|---|---|---|
| Tube No. | Antigen Markers: Fluorochrome | Cell Types Identified |
| 1 | CD4-FITC, CD45RA-PE, CD8PerCP, Her-Alx647 | Negative control |
| 2 | CD4-FITC, CD45RA-PE, CD8PerCP, rhuMAb β7-Alx647 | Unsaturated tube to detect β7 occupancy |
| 3 | CD4-FITC, CD45RA-PE, CD8PerCP, rhuMAb β7-Alx647 | Saturated tube with 10 µg/mLPR0145223 |

| | | |
|---|---|---|
| Her=Herceptin^{®}. | | |

Blood samples were stained according to the protocol in Laboratory Notebook 47237, p. 48, with saturating concentrations of the following antibody conjugates: CD4 conjugated to FITC (clone M-T477), CD45RA conjugated to phycoerythrin (PE) (clone 5H9), CD8 conjugated to PerCP (clone SK1), and Herceptin^{®} conjugated to Alexa-647 or rhuMAb β 7 conjugated to Alexa 647. For Tube 3, the test article rhuMAb β 7 (PRO145223) was diluted to the saturating concentration (10 µg/mL) and added to the blood sample.

All incubations were performed on ice in the dark for 30 ± 5 minutes. After staining, red blood cells were lysed, samples were washed and cells were resuspended in fixative buffer using a Lyse Wash Assistant (BD Biosciences). Twenty-five thousand gated lymphocyte events were acquired using a forward scatter/side scatter gate on the BD FACSCalibur (BD Biosciences).

Lymphocytes were identified from a SSC/FSC scattergram. CD4⁺ and CD8⁺ cells were then identified using a SSC/CD4FITC plot and a SSC/CD8 PerCP plot, respectively. Tube 1 was used as negative control for gating. The lymphocytes showing β7/CD45RA^{□}, β7^{low}/CD45RA^{□}, and β7^{intermediate}/CD45RA⁺ cells were identified for the tubes with and without the saturating concentration of rhuMAb β7. The GMFI and the percentage of gated cells were noted and the GMFI was then expressed as MOEF using the slope of the APC channel where MOEF GMFI^{®} slope of the FL4 channel.

All incubations were performed on ice in the dark for 30 □ 5 minutes. After staining, red blood cells were lysed, samples were washed and cells were resuspended in fixative buffer using a Lyse Wash Assistant (BD Biosciences). Twenty-five thousand gated lymphocyte events were acquired using a forward scatter/side scatter gate on the BD FACSCalibur (BD Biosciences).

Lymphocytes were identified from a SSC/FSC scattergram. CD4⁺ and CD8⁺ cells were then identified using a SSC/CD4FITC plot and a SSC/CD8 PerCP plot, respectively. Tube 1 was used as negative control for gating. The lymphocytes showing β7^{high}/CD45RA⁻, β7^{low}/CD45RA^{□}, and β7^{intermediate}/CD45RA⁺ cells were identified for the tubes with and without the saturating concentration of rhuMAb β7. The GMFI and the percentage of gated cells were noted and the GMFI was then expressed as MOEF using the slope of the APC channel where MOEF^{®} GMFI slope of the FL4 channel.

### Flow Cytometry Analysis of Peripheral Blood Lymphocyte Subsets (Total B Cells, T Cells, and NK Cells)

The panel configuration for the analysis of whole blood samples is presented in Table 2-4.

**Table 2-4**

| Whole Blood Panel Configuration | | |
|---|---|---|
| Tube No. | Antigen Markers: Fluorochrome | Cell Types Identified |
| 1 | IgG-FITC, IgG-PE, IgG-PerCP, IgG-APC, | Isotype used for gating population on SSC and FSC. Setting quadrant markers. |
| 2 | CD4-FITC, CD45-PE, CD3-PerCP, CD20-APC | B cells, T cells, and T-cell subsets |

| | | |
|---|---|---|
| FSC=forward scatter; SSC=side scatter. | | |

For all whole blood samples, the flow cytometry assay was carried out according to Procedure 48038-36. Samples were incubated with saturating concentrations of fluorescently conjugated monoclonal antibodies CD4 (Clone M-T477), CD45 (Clone TU116), CD3 (Clone SP34-2), and CD20 (Clone L27), which are known to cross-react with cynomolgus monkey leukocytes, along with appropriate isotype controls. Instrument set-up control tubes and assay tubes were prepared manually. All incubations were performed in the dark at room temperature for 30±5 minutes.

### PK DATA ANALYSIS

Nominal sample collection times were used in the data analysis, with minimal deviation from the schedule. Mean (±SD) concentrations of rhuMAb β7 were calculated for each serum sample using MS Excel^{®} (Microsoft Corp.; Redmond, WA) software, and data points were plotted using KaleidaGraph™ (Synergy Software; Reading, PA). Except for the predose timepoint, serum concentrations determined to be less than reportable (LTR) were treated as missing and not used in presentation or analysis of the PK data. For PK data calculations, Study Day 1 was converted to PK Day 0 to indicate the start of dosing (see blood sample collection).

### Non-Compartmental PK Analysis

The serum concentrations-time data from each animal in the 5 mg/kg dose group until 7 days after the first dose were analyzed using the IV bolus input model (Model 201, WinNonlin-Pro, version 5.0.1; Pharsight Corporation; Mountain View, CA). The data following the last dose in the 5 mg/kg dose group were not analyzed because the serum concentrations were affected by ATAs after the second or third dose. The serum concentrations-time data from each animal in the 25 mg/kg dose group for 7 days after the first and last dose were also analyzed using the IV bolus input model (Model 201). The following methods were used to estimate specific PK parameters:
*C*ₘₐₓ: Maximum serum concentration following an IV bolus dose.
AUC₀₋₇: Area under the serum concentration-time curve from Time=0 to PK Day 7 was calculated using the linear trapezoidal rule.
AUC₂₁₋₂₈: Area under the serum concentration-time curve from Time=PK Day 21 to PK Day 28 was calculated using the linear trapezoidal rule.
AUCall: Area under the serum concentration-time curve from Time=0 to last PK Day of data from the recovery animals was calculated using the linear trapezoidal rule.
*V*₁: Volume of distribution to the central compartment (Dose/*C*ₘₐₓ).
Two-Compartment PK Analysis.

A two-compartment model with IV-bolus input, first-order elimination, and micro-rate constants (Model 7, WinNonlin Pro, version 5.0.1) was used to analyze the mean concentration-time data from the 4 recovery animals in the 25 mg/kg dose group. The following modeling options and methods were used to estimate specific PK parameters:
Initial estimates for A, B, α, and β were determined graphically by WinNonlin.
Weighted analysis used WEIGHT=-2.

The Nelder-Mead minimization algorithm was used.

Model selection was based on goodness of fit by visual inspection and by the between-model comparison of Akaike's Information Criterion (AIC).
CL: Clearance (Dose/AUC)
*t*_{1/2,β}: Half-life of the beta phase; terminal half-life (ln[2]/β)
*V*ₛₛ: Volume of distribution at steady state (MRT_{inf}• CL)

The zero-time intercepts associated with the alpha and beta phases (A and B, respectively), macro-rate constants (α and β), and intercompartmental micro-rate constants (*k*₁₀, *k*₁₂, and *k*₂₁) are provided in Table 7.

### FLOW CYTOMETRY ANALYSIS

Absolute counts as percentage of baseline were calculated for each of the CD4 and CD8 T-cell subsets (CD45RA⁻ β7^{high}, CD45RA⁻β7^{low}, and CD45RA⁺ β7^{intermediate}) and the CD4⁺CD3⁺ T cells, CD4⁻CD3⁺ (CD8) T cells, CD3⁻CD20⁻ (NK) cells, and CD20⁺ B cells at each timepoint in the study for vehicle- and rhuMAb β7-treated animals. Individual absolute counts as a percentage of baseline were averaged for each treatment group (vehicle, 5 mg/kg rhuMAb β7, and 25 mg/kg rhuMAb β7) to calculate group means. Definitions of values determined by flow cytometry analysis are as follows:
Abs counts: absolute counts for each respective subset of lymphocytes; equal to the absolute lymphocyte counts (a value obtained from hematology measurements expressed as lymphocytes per µL of peripheral blood) times the percentage of gated lymphocytes for each subset (obtained from flow cytometry analysis)
Abs counts (%BL): absolute counts as a percentage of baseline abs counts obtained before dosing; calculated as Abs counts divided by average of predose absolute counts
MOEF: molecules of equivalent fluorescence; calculated using the slope of the FL4 channel times the geometric mean fluorescence intensity
MOEF (% predose): MOEF as a percentage of MOEF before dosing; calculated as the MOEF at the respective timepoint after dosing divided by the average MOEF before dosing.

### RESULTS AND DISCUSSION

### PK DATA ANALYSIS

Following four weekly IV bolus doses of 5 mg/kg rhuMAb β7 to cynomolgus monkeys, the average serum concentration 15 minutes following the first dose was 173 µg/mL (range: 125-207 µg/mL) compared with 15 minutes following the last dose, which was 196 µg/mL (range: 70.9-326 µg/mL). The average trough serum concentrations before the second, third, and fourth doses were 56.5 µg/mL (range: 28.7-77.1 µg/mL), 35.1 µg/mL (range: 0.0401-97.1 µg/mL), and 60.2 µg/mL (range: 4.29-137 µg/mL), respectively. The variability in the trough serum concentrations after the second dose may be explained by the presence of ATAs, because all 6 monkeys in this 5 mg/kg dose group developed an ATA response that was detected after the second dose; 1 monkey had positive anti-human antibody titers before the first dose of rhuMAb β7. The 4 monkeys with the lowest trough concentrations had the highest ATA titers.

Following four weekly IV bolus doses of 25 mg/kg rhuMAb β7 to cynomolgus monkeys, the average serum concentration 15 minutes following the first dose was 917 µg/mL (range: 740-1070 µg/mL) compared with 15 minutes following the last dose, which was 1690 µg/mL (range: 1130-2250 µg/mL). The average trough serum concentrations before the second, third, and fourth doses were 325 µg/mL (range: 243-677 µg/mL), 535 µg/mL (range: 240-709 µg/mL), and 594 µg/mL (range: 194-776 µg/mL), respectively. There was also an ATA response at the 25 mg/kg dose level, with 5 of the 10 monkeys (3 of 4 recovery monkeys) in this dose group developing an ATA response that correlated with decreased serum concentrations of rhuMAb β7. ATAs were detected after the second dose in some monkeys; 1 monkey in this group also had positive anti-human antibody titers before the first dose of rhuMAb β7.

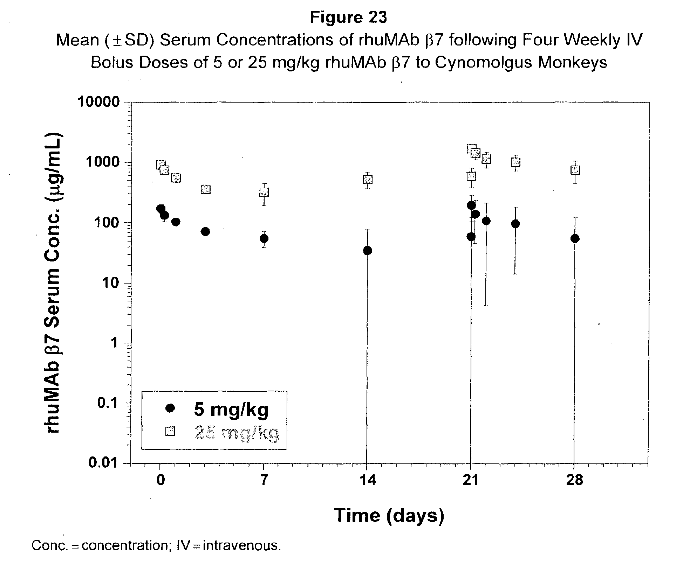

The serum concentrations over the 28 days during the in-life portion of the study following four weekly IV doses of 5 or 25 mg/kg are presented in Figure 23. The serum concentrations over the in-life portion (up to 7 days after the last dose) were compared by non-compartmental analysis because there was no recovery cohort in the 5 mg/kg dose group for comparison. The results indicated that the dose-normalized area under the serum concentration-time profiles from Day 0 to Day 7 after the first dose (AUC₀₋₇/dose) were similar for the 5 mg/kg and 25 mg/kg dose groups (113±17.0 and 119± 15.8 day • µg/mL/mg/kg, respectively; see Table 5). This suggests that rhuMAb β7 exhibits dose-proportional pharmacokinetics in cynomolgus monkeys at the dose range studied. The volume of distribution to the central compartment (*V*₁) was also similar following the first dose of 5 or 25 mg/kg (29.0±4.30 and 27.7±3.54 mL/kg, respectively; see Table 5). Following administration of 25 mg/kg, AUC₂₁₋₂₈ after the last dose was more than 2-fold greater compared with AUC₀₋₇ after the first dose (7024±1958 and 2987±394 day • µg/mL, respectively; see Table 2-5). *C*ₘₐₓ following administration of 25 mg/kg also increased approximately 2-fold after the last dose compared with after the first dose (1710±302 and 916 ± 111 µg/mL, respectively; see Table 5). Both of these findings suggest that there was accumulation of rhuMAb β7 following four weekly IV doses of 25 mg/kg to cynomolgus monkeys; however, such accumulation is expected in an antibody with a long half-life at these doses. The data after the last dose in the 5 mg/kg dose group could not be compared because ATAs affected rhuMAb β7 CL after the second dose.

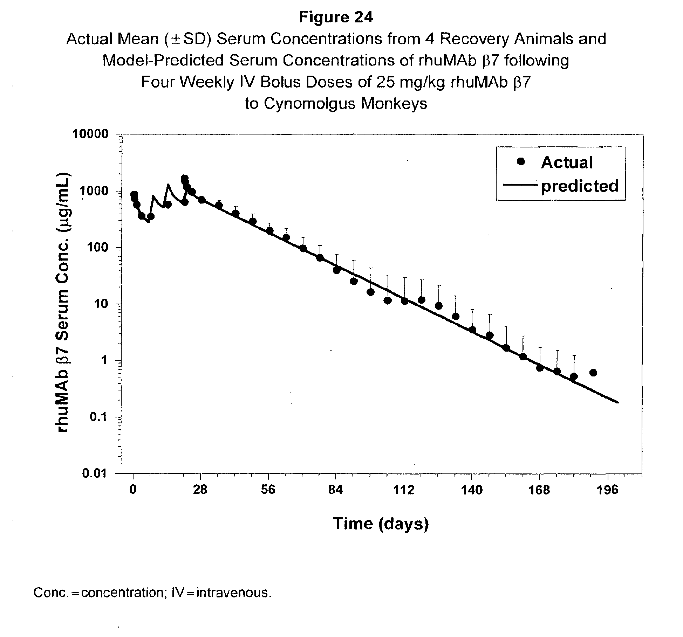

Mean data from the 4 monkeys from the 23-week recovery period following four weekly doses of 25 mg/kg were fit to a two-compartment model (see Figure 24). The serum concentration-time profile following the last dose was biphasic with a fast initial distribution phase followed by a slower elimination phase. Compartmental analysis estimated a slow CL of 2.93 mL/day/kg and a relatively long *t*_{1/2,β} of 14.5 days following four weekly IV bolus doses of 25 mg/kg rhuMAb β7 (see Tables 2-6). However, 3 of the 4 monkeys had ATAs, which probably contributed to the clearance of rhuMAb β7. *V*ₛₛ was estimated to be 56.9 mL/kg, which suggests that rhuMAb β7 remained mostly in the vascular compartment (see Tables 2-6). The total exposure (AUCₐₗₗ) following four weekly IV doses of 25 mg/kg was estimated as 33,400 day • µg/mL by non-compartmental analysis of the data from the recovery animals (see Tables 2-6).

**Table 2-5**

| Non-Compartmental PK Parameters of Individual Data (mean±SD) following the First or Last IV Bolus dose of rhuMAb β7 to Cynomolgus Monkeys | | | | | |
|---|---|---|---|---|---|
| Treatment Group | AUC₀₋₇ (day • µg/mL) | AUC₂₁₋₂₈ (day • g/mL) | AUC₀₋₇ or AUC₂₁₋₂₈/Dose (day • µg/mL/mg/kg) | Cₘₐₓ (µg/mL) | *V*₁ (mL/kg) |
| 5 mg/kg 1st dose (n=6) | 563±84.8 | NA | 113±17.0 | 175±24.0 | 29.0±4.30 |
| 25 mg/kg 1st dose (n=10) | 2990±394 | NA | 119±15.8 | 916±111 | 27.7±3.54 |
| 25 mg/kg last dose (n=10) | NA | 7020±1960 | 281±78.3 | 1710±302 | 15.1±3.03 |

| | | | | | |
|---|---|---|---|---|---|
| AUC₀₋₇=area under the serum concentration-time curve from Time=0 to PK Day 7; AUC₂₁₋₂₈=area under the serum concentration-time curve from Time=PK Day 21 to PK Day 28; *C*ₘₐₓ=maximum serum concentration following an IV bolus dose; IV=intravenous; NA=not applicable; PK=pharmacokinetic; *V*₁=volume of distribution to the central compartment (Dose/*C*ₘₐₓ). | | | | | |

**Table 2-6**

| Two-Compartment PK Parameters (%CV) of Mean Data from 4 Recovery Animals following Four Weekly IV Bolus doses of 25 mg/kg rhuMAb β7 to Cynomolgus Monkeys | | | |
|---|---|---|---|
| AUC (day • µg/mL) | CL (mL/day/kg) | *t*_{1/2,β} (days) | *V*ₛₛ (mL/kg) |
| 8530 (5.50) | 2.93 (5.51) | 14.5 (1.89) | 56.9 (7.08) |

| | | | |
|---|---|---|---|
| AUC=area under the serum concentration-time curve; CL=clearance; IV=intravenous; PK=pharmacokinetic; *t*_{1/2,}**p**beta half-life; *\*/ₛₛ=volume of distribution at steady state. | | | |

**Table 2-7**

| Two-Compartment Model Parameters (%CV) of Mean (±SD) Data from 4 Recovery Animals following Four Weekly IV Bolus Doses of 25 mg/kg rhuMAb β7 to Cynomolgus Monkeys | | | | | | |
|---|---|---|---|---|---|---|
| A (µg/mL) | B (µg/mL) | α (day⁻¹) | β (day⁻¹) | *k*₁₂ (day⁻¹) | *k*₂₁ (day⁻¹) | *k*₁₀ (day⁻¹) |
| 509 (26.4) | 378 (8.64) | 0.795 (57.5) | 0.0479 (1.90) | 0.372 (72.5) | 0.366 (51.4) | 0.104 (16.0) |

| | | | | | | |
|---|---|---|---|---|---|---|
| A, B=zero-time intercepts associated with the alpha and beta phases, respectively; α, β=macrorate constants alpha and beta, respectively; *k*₁₀=elimination rate from central compartment; *k*₁₂, *k*₂₁=intercompartmental rate constants. | | | | | | |

### FLOW CYTOMETRY ANALYSIS (IMMUNOPHENOTYPING AND PHARMACODYNAMICS)

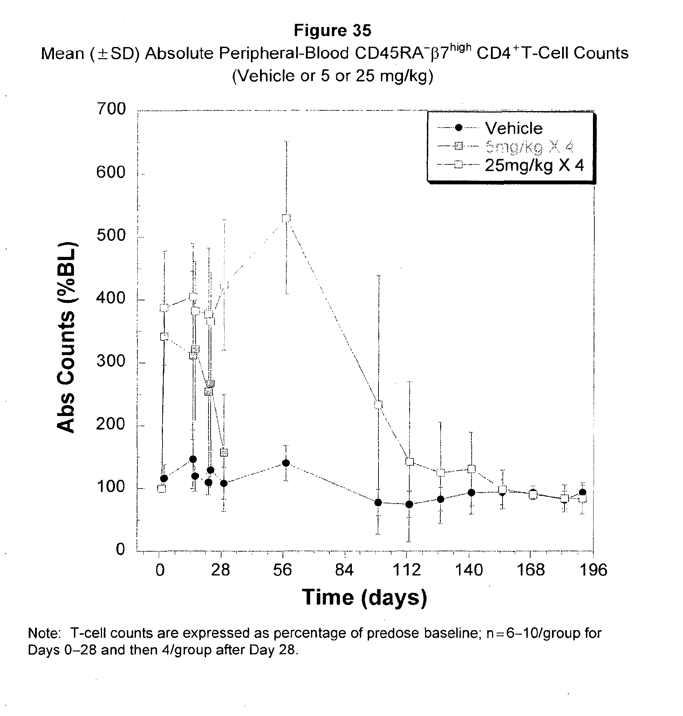

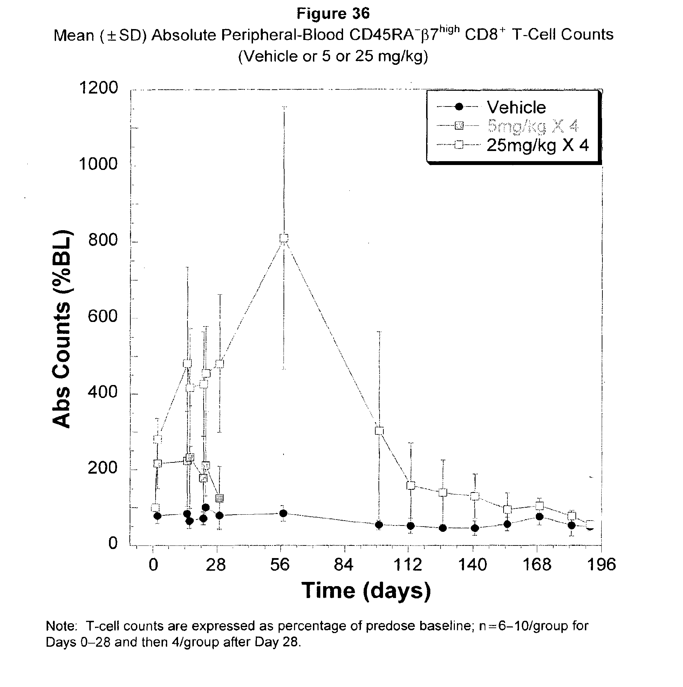

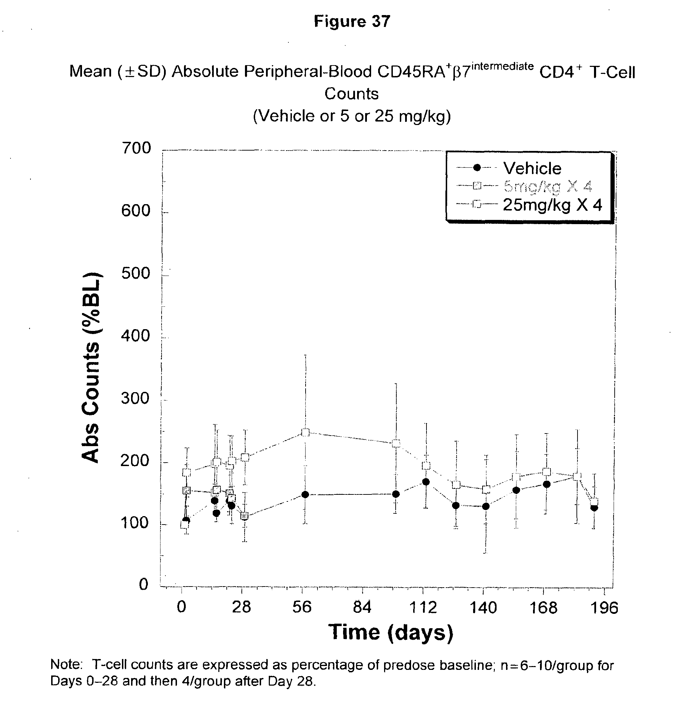

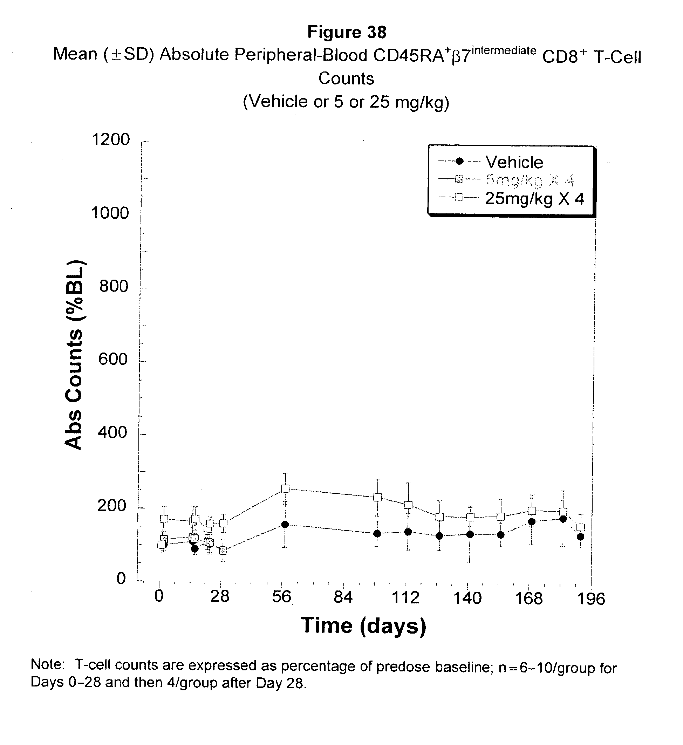

Group mean (± SD) counts for subsets of peripheral-blood CD4 and CD8 T cells are presented in Figures 35-38.

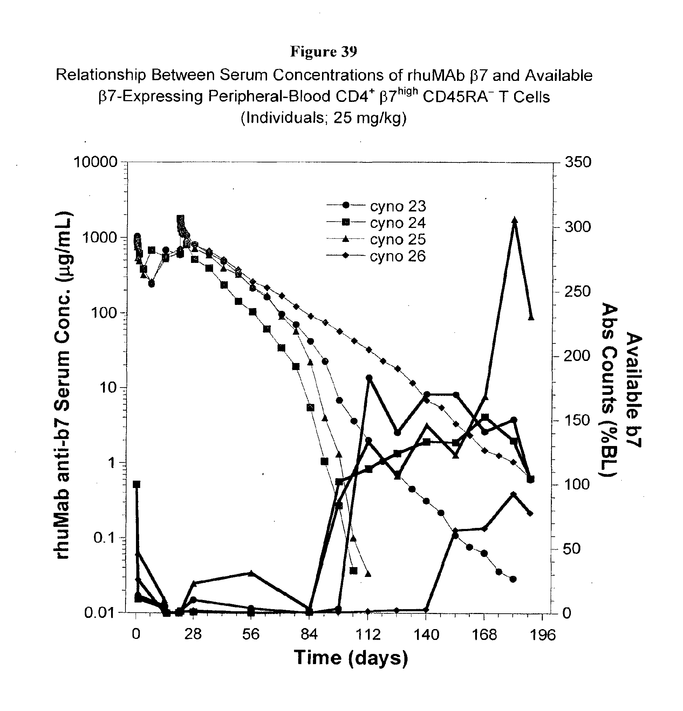

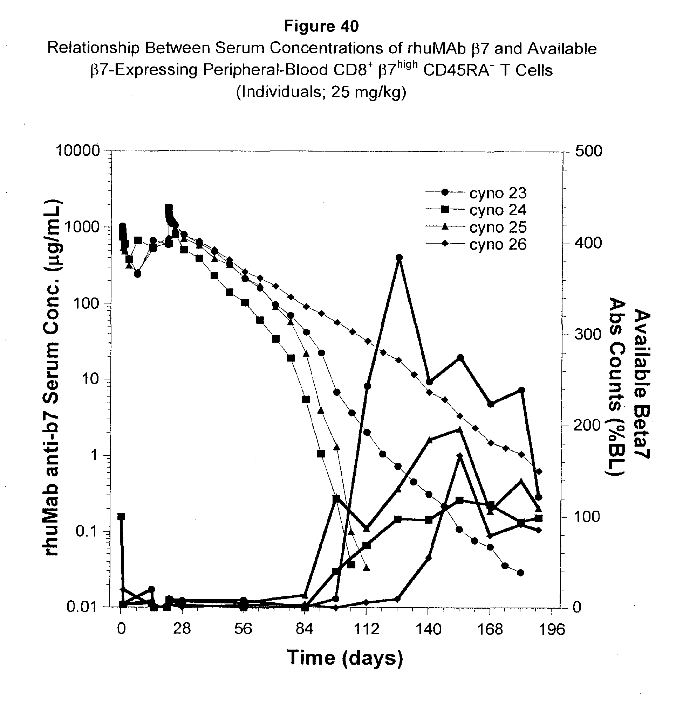

Figures 39-40 show the relationship between serum concentrations of rhuMAb β7 and available β7-expressing peripheral-blood CD4⁺ β7^{high} CD45RA⁻ T cells and CD8⁺ β7^{high} CD45RA- T cells in individual cynomolgus monkeys. Individual and group mean (± SD) absolute counts, β7 expression, and available β7 expression on subsets of CD4 and CD8 T cells are presented in Appendix E.

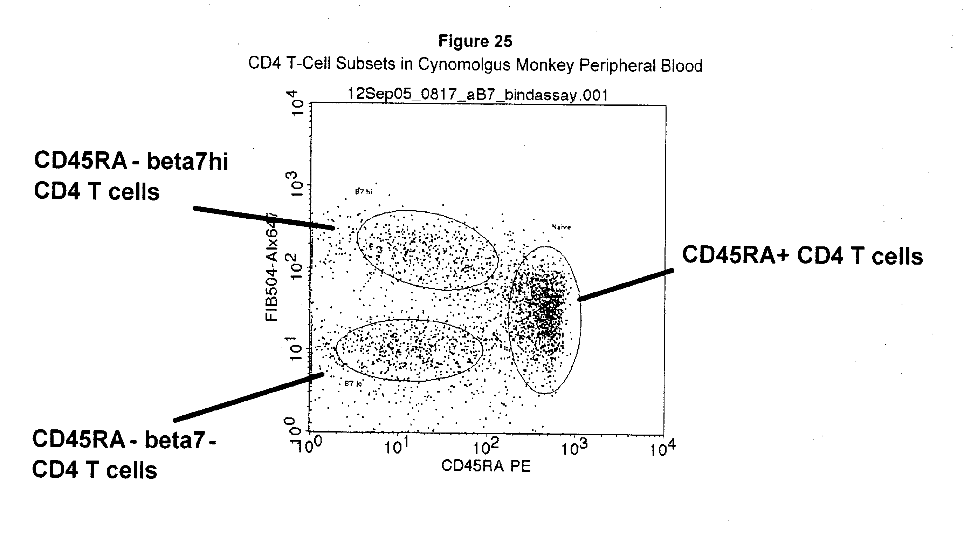

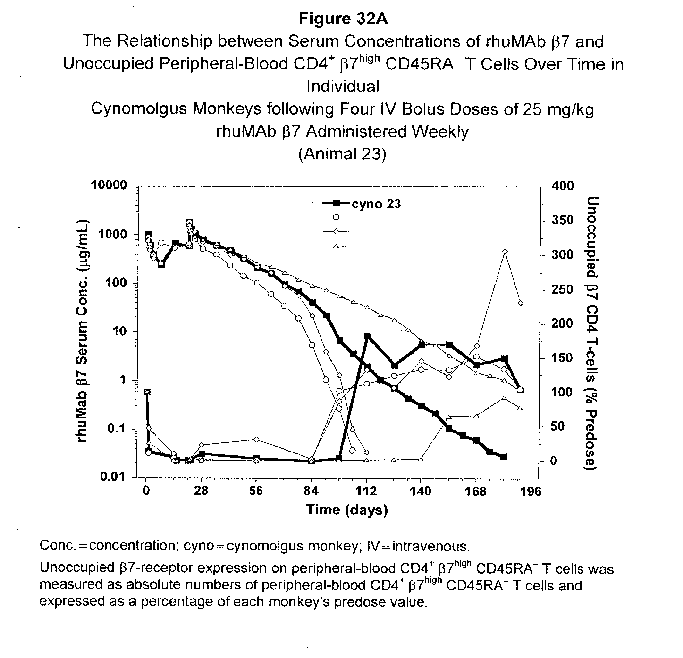

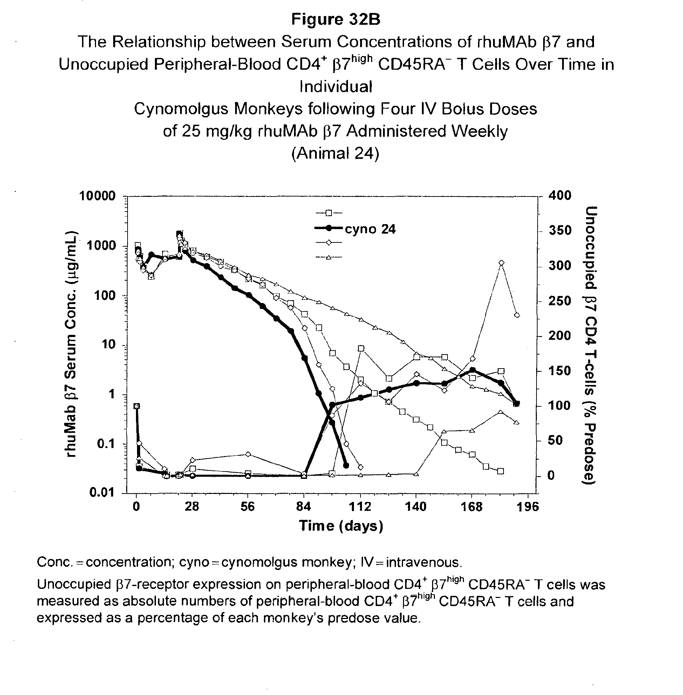

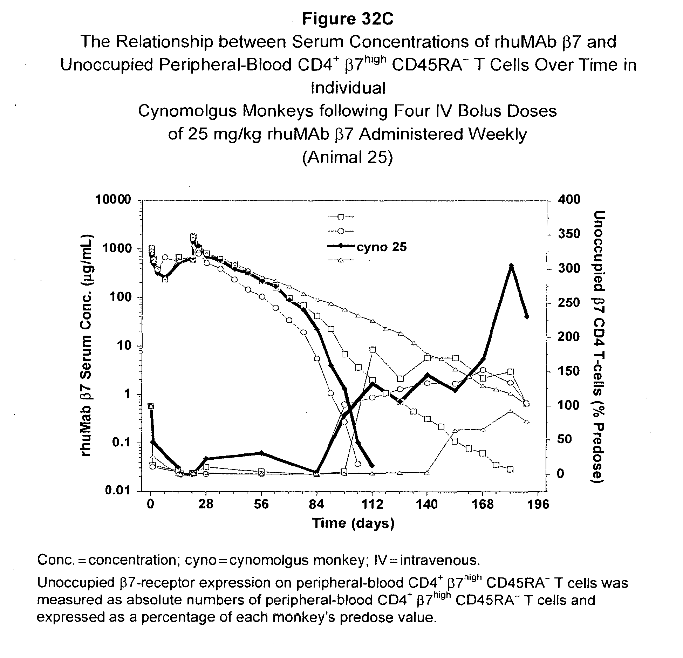

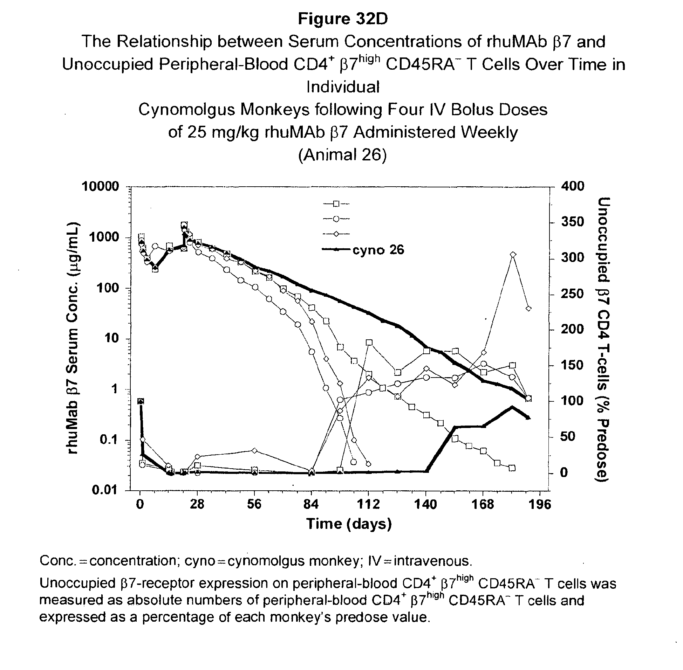

The occupation of β7 receptors in this study was analyzed by flow cytometry as available β7 on peripheral-blood CD4 or CD8 T-cell subsets defined as CD45RA⁺ or CD45RA⁻. CD45RA⁺ and CD45RA⁻ T cells, phenotypically similar to naive and memory/effector T cells in humans, express intermediate vs. high levels of β7 (see Figure 25 for representative FACS dot plot of T-cell subsets). CD45RA⁻β7^{high} CD4 T cells home preferentially to intestinal sites. The data from this assay indicated saturation of β7 following the first dose at both 5 and 25 mg/kg, and the return of free β7 that correlated with a decrease in serum concentrations of rhuMAb β7 (see Figures 32-33 in the PK/PD relationship section above). This saturation data suggested that a serum concentration of rhuMAb β7 above 1-10 µg/mL would likely maintain complete β7-receptor saturation on peripheral-blood T cells.

Total β7 expression levels on peripheral blood T cells were examined following administration of rhuMAb β7 using a non-competing antibody for detection of β7 by flow cytometry. There was no evidence of down-modulation of β7 on peripheral blood T cells in cynomolgus monkeys following IV doses of 5 or 25 mg/kg (data not shown). Total β7 expressing cells in peripheral blood were also examined following administration of rhuMAb β7 using the same non-competing antibody for detection of β7.

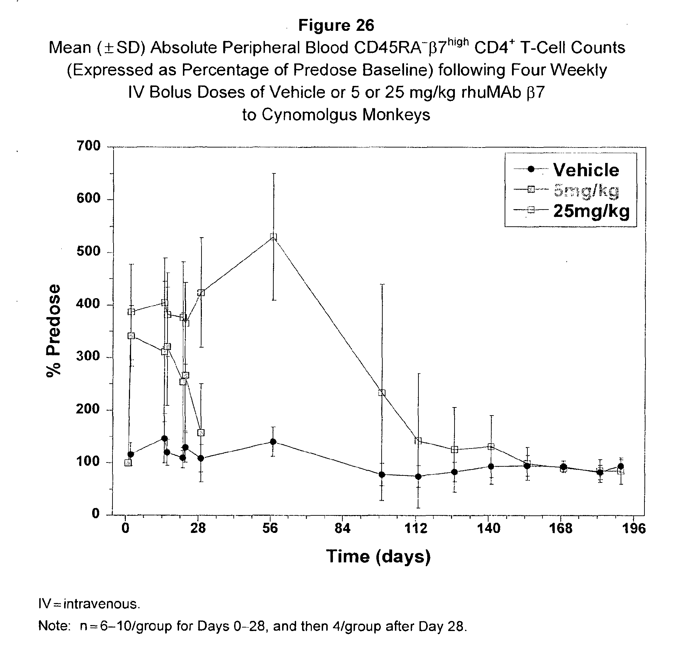

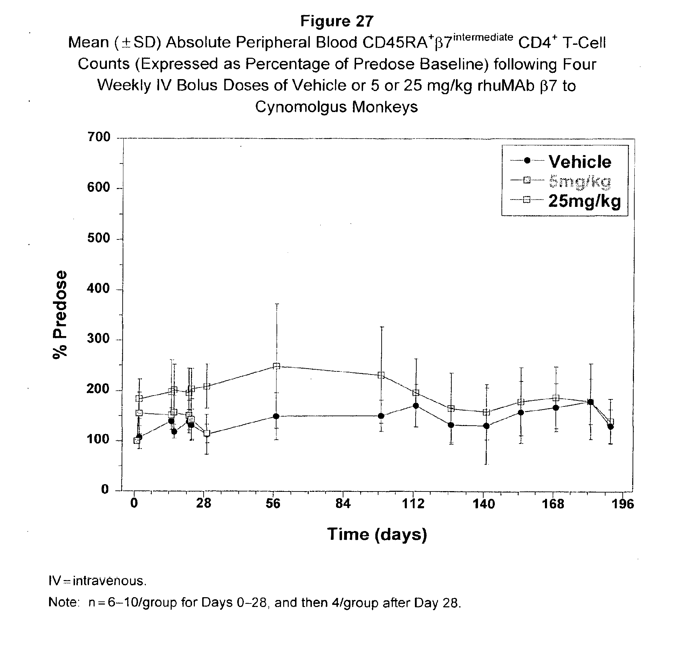

CD45RA⁻β7^{high} CD4⁺ T cells (phenotypically similar to gut-homing CD4⁺ cells in humans and mice) increased approximately 5-fold over baseline levels following four weekly doses of 25 mg/kg (see Figure 26). Likewise, CD45RA⁺β7^{intermediate} CD4⁺ T cells (phenotypically similar to human naive T cells) also increased following four weekly doses of 25 mg/kg (approximately 2.5-fold over baseline levels) (see Figure 27). There were no substantial changes in CD45RA-β7^{low} T cells or in T-cell subsets of vehicle-control animals (see Figures 26 and 27 ). Similar results were observed with CD45RA⁻β7^{high} and CD45RA⁺β7^{intermediate} CD8⁺ T cells. These results are consistent with inhibition of trafficking of β7-expressing lymphocytes to the gut, which is the proposed mechanism of action of this therapeutic antibody.

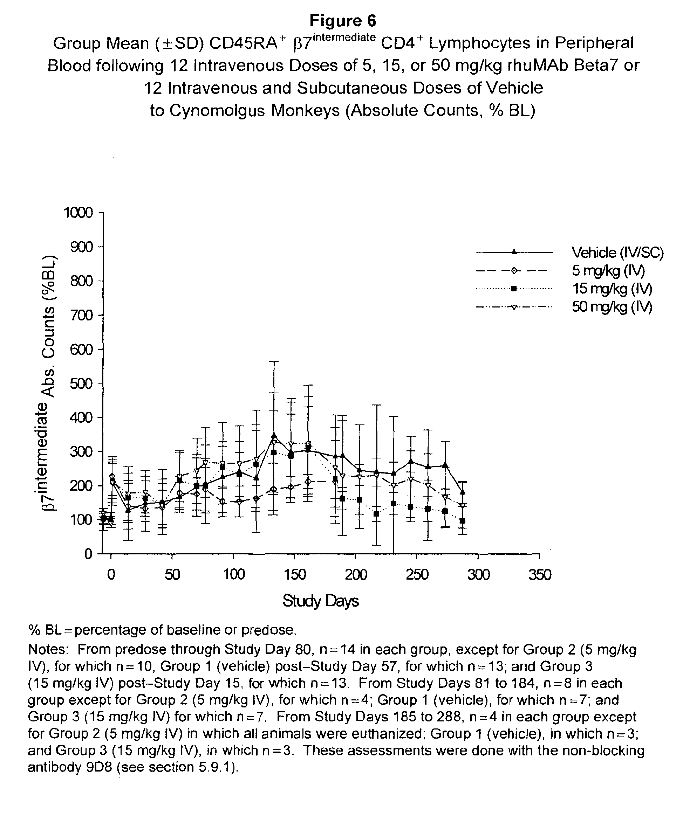

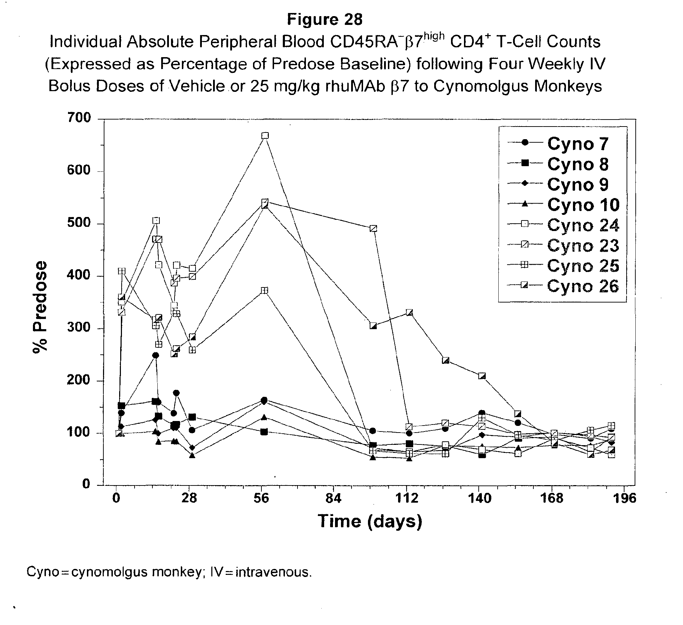

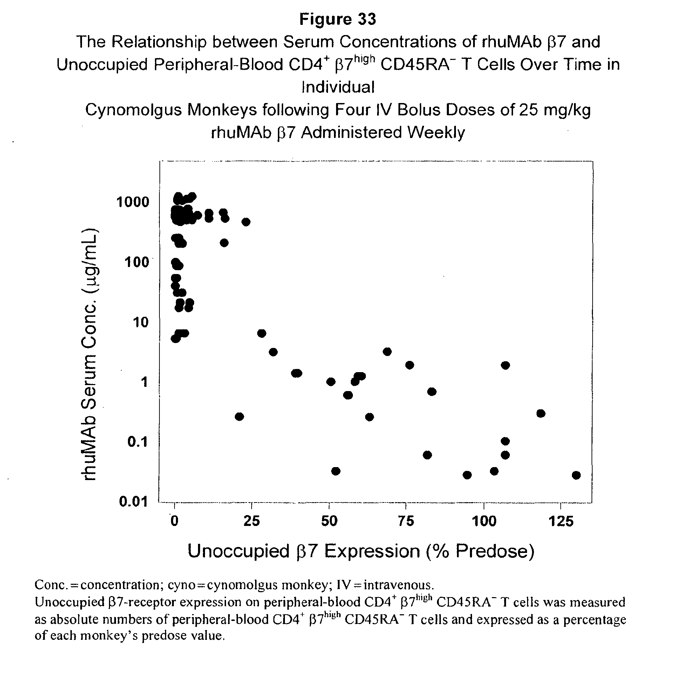

In support of this hypothesis, increased percentages of CD45RA⁻β7^{high} T cells in peripheral blood correlate with occupancy of the β7 receptor on blood T cells by rhuMAb β7. Likewise, loss of occupancy of β7 by rhuMAb β7 (detection of available β7 receptors) correlates with return to baseline of CD45RA⁻β7^{high} T cells in peripheral blood. For example, on Study Day 99, percentages of CD45RA⁻β7^{high} CD4⁺ T cells in the blood of Animals 24 and 25 decreased to baseline levels (see Figure 6). This decrease correlated with loss of occupancy of β7 by rhuMAb β7 on Study Day 99 in these particular monkeys (see Figures 32 and 33 in the PK/PD relationship section above). In contrast, Animals 23 and 26 exhibited saturated β7 receptors on peripheral-blood T cells on Study Day 99 (see Figures 32 and 33), and absolute numbers of CD45RA⁻β7^{high} CD4⁺ T cells in the blood remained elevated compared with predose levels (see Figure 28). Similar results were observed with the subsets of CD8⁺ T cells. Thus, occupancy of β7 receptors correlated with increased percentages of CD45RA⁻β7^{high} CD4⁺ T cells in the blood, consistent with inhibition of trafficking to intestinal sites. Increases in subsets of CD45RA⁻ and CD45RA⁺ peripheral-blood T cells likely contributed to the observed increase in circulating lymphocytes described below.

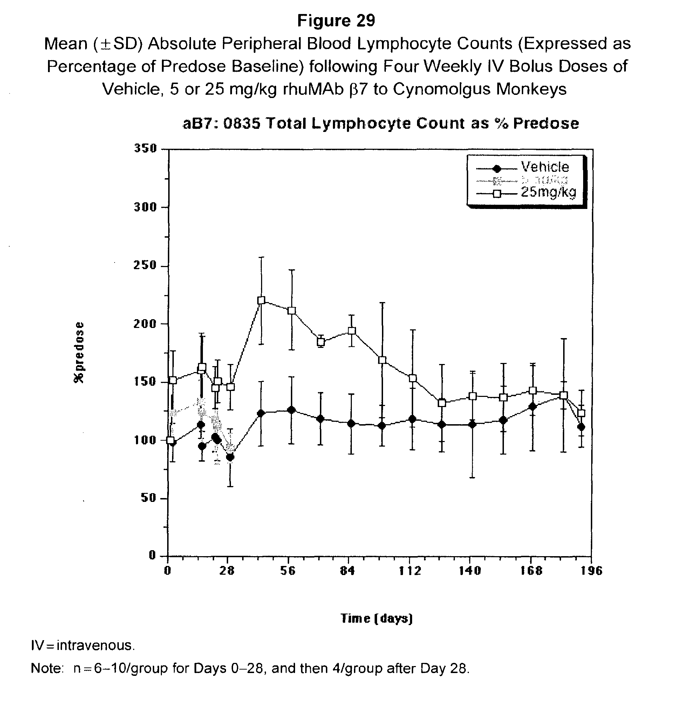

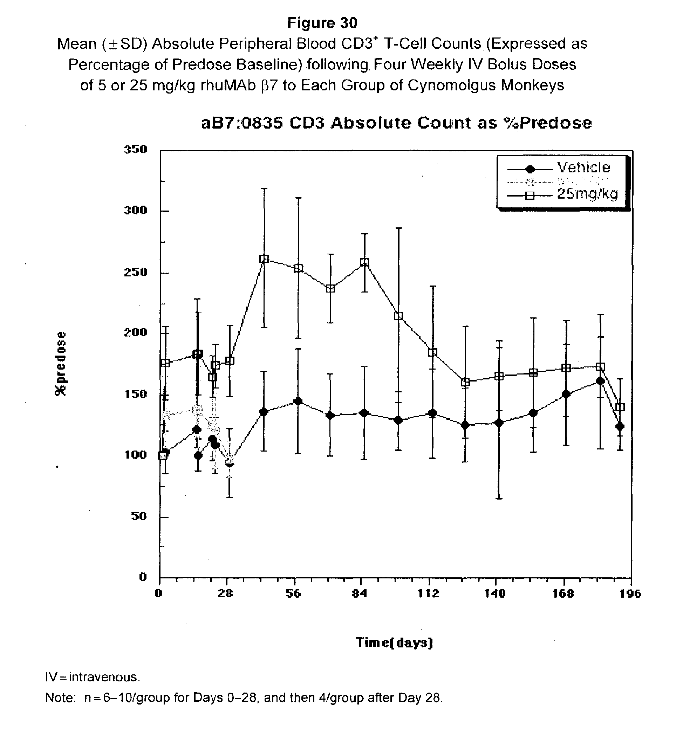

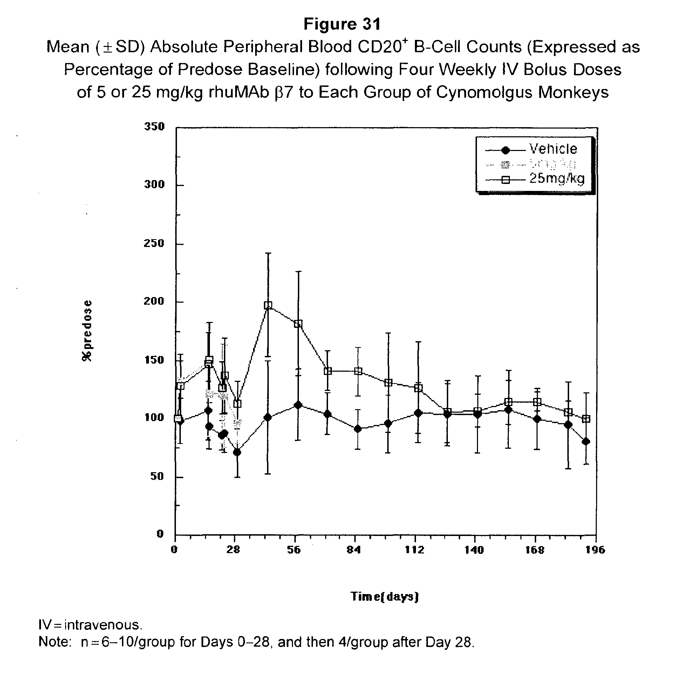

Weekly administration of 25 mg/kg rhuMAb β7 induced a mild increase in circulating lymphocytes in cynomolgus monkeys that was evident after the first dose and appeared to increase after the last dose. The cynomolgus monkeys in the 25 mg/kg dose group had an average increase of approximately 2.2-fold over baseline compared with an average increase of 1.3-fold for the vehicle control group (see Figure 29). The absolute lymphocyte counts in the 25 mg/kg dose group returned to predose or vehicle levels as the serum concentrations of rhuMAb β7 decreased, an effect that correlated with the loss of lymphocyte β7 receptor occupancy by rhuMAb β7. Therefore, the mild increase in circulating lymphocytes evident in this study appeared to be transient, related to administration of rhuMAb β7, and was most likely due to the inhibition of trafficking of β7-expressing lymphocytes to the gut as mentioned above. The increase in peripheral-blood lymphocyte counts was evident in both T cells and B cells, although the increase in T cells was more substantial and exhibited increased duration compared with B cells (see Figures 30 and 31).

### PK/PD RELATIONSHIPS

Integrin β7-preceptor saturation and the levels of circulating β7⁺ T cells were used as PD markers.

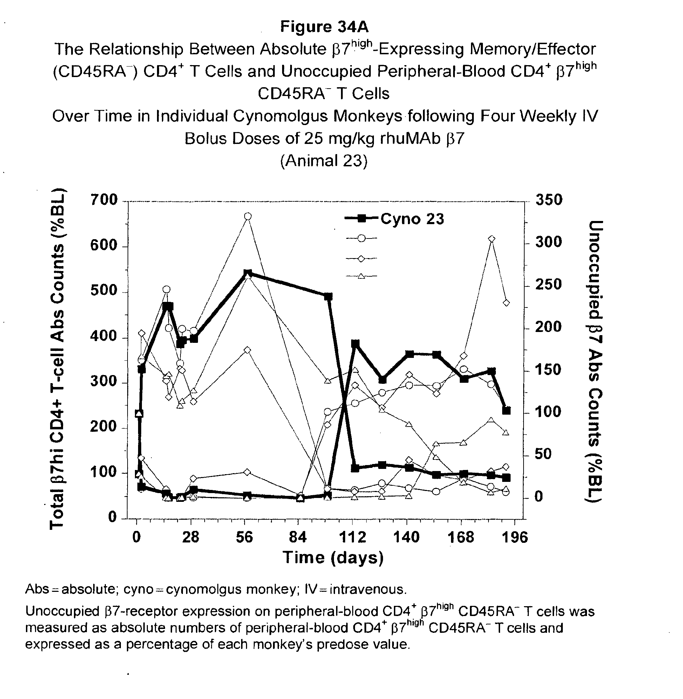

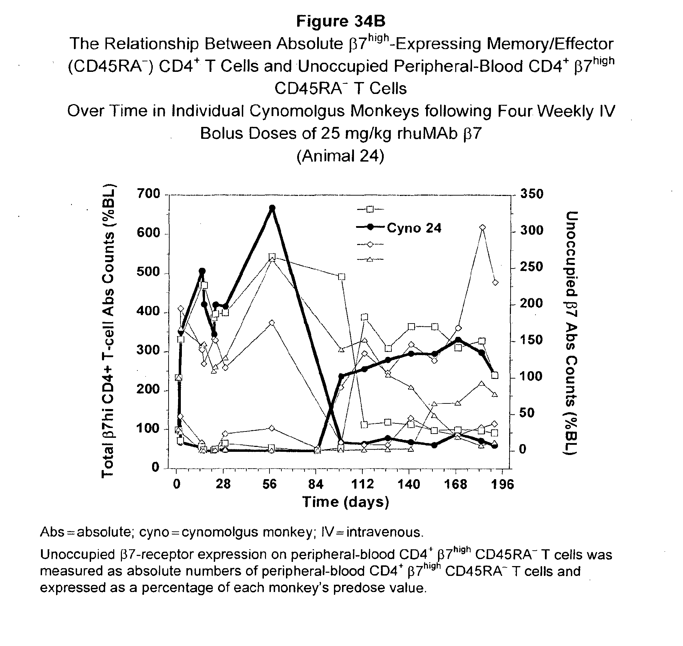

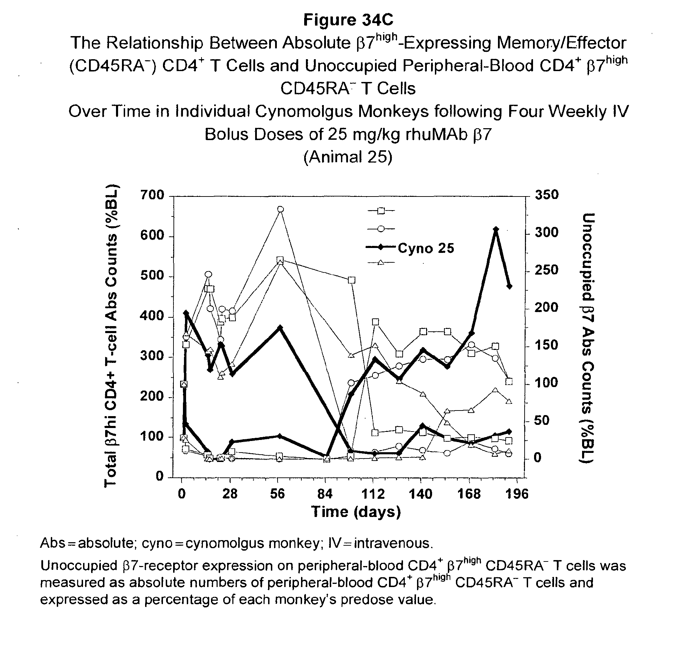

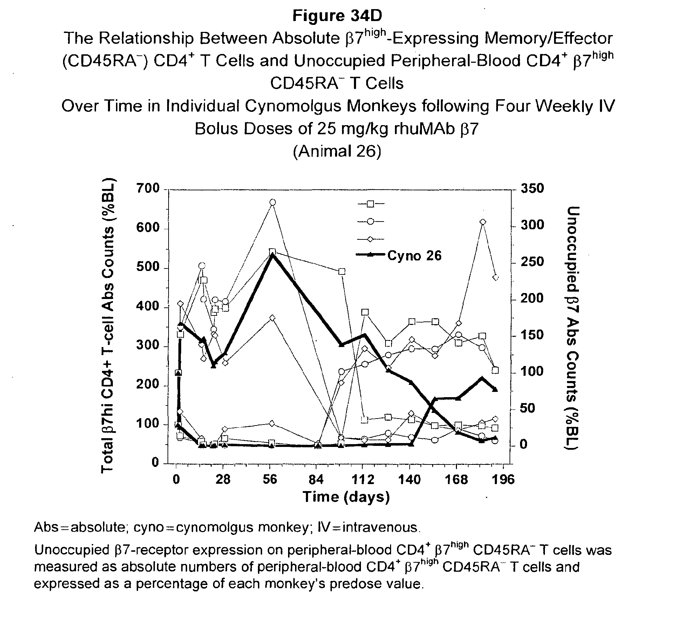

Following four weekly doses of 5 or 25 mg/kg rhuMAb β7 to cynomolgus monkeys, no evidence of down modulation of β7 expression on peripheral-blood T cells was observed. However, saturation of β7 on peripheral-blood T cells was observed following the first dose of 5 or 25 mg/kg rhuMAb β7, and desaturation (detection of available β7) appeared to correlate with a decrease in rhuMAb β7 level below approximately 10 µg/mL (see Figures 32A-D for profiles from Animals 23-26, respectively, and Figure 33). A correlation between occupation of β7 on peripheral blood CD45RA⁻β7^{hi} CD4 T cells and elevated absolute numbers of these cells was also observed following administration of rhuMAb β7 (see Figures 34A-D). These findings suggest that serum concentrations of rhuMAb β7 above 1-10 µg/mL maintain occupation of β7 on gut-homing CD4⁺ cells and block the homing of these T cells to the gut, which in turn may cause accumulation of these cells in the periphery.

### CONCLUSIONS

Following four weekly IV bolus doses of 5 or 25 mg/kg rhuMAb β7 to cynomolgus monkeys, the trough serum concentrations increased after the second dose except when ATAs were formed. AUC₀₋₇/dose following the first dose was similar for the 5 mg/kg and 25 mg/kg dose groups, suggesting that rhuMAb β7 exhibited dose-proportional pharmacokinetics in cynomolgus monkeys at the dose range studied. For the 25 mg/kg dose group, the AUC₀₋₇ and *C*ₘₐₓ increased approximately 2-fold after the last dose compared with the first dose. Both of these findings suggest accumulation of rhuMAb β7, as expected, following four weekly IV doses of 25 mg/kg to cynomolgus monkeys. Compartmental analysis of the data from the 25 mg/kg recovery animals estimated a slow CL of 2.93 mL/day/kg and a relatively long *t*_{1/2,}β of 14.5 days.

Integrin β7-receptor occupancy assays suggested that β7 receptors on peripheral blood T-cell subsets were fully saturated following the first of four weekly doses of 5 or 25 mg/kg rhuMAb β7 to cynomolgus monkeys. The detection of available β7 correlated with a decrease in serum concentrations of rhuMAb β7 and suggested that a serum concentration above approximately 10 µg/mL will maintain occupancy of β7 receptors on peripheral-blood T cells. CD45RA⁻β7^{high} T cells (phenotypically similar to gut-homing T cells in humans and mice) increased approximately 5-fold over baseline levels following four weekly doses of 25 mg/kg to cynomolgus monkeys. Likewise, CD45RA⁺β7^{intermediate} T cells (phenotypically similar to human naive T cells) also increased following four weekly doses of 25 mg/kg (approximately 2.5-fold over baseline levels). These findings are consistent with the mechanism of action of rhuMAb β7-namely, the inhibition of trafficking of β7-positive lymphocytes to the gut through inhibition of α4β7 binding to its ligands, which then leads to the accumulation of these cells in the peripheral circulation. These phenomena will be further explored in future studies in cynomolgus monkeys.

### APPENDIX 2F

### Study Summary Table

| **Test Article:** rhuMAb β7 |
|---|
| |
| **Type of Study:** Non-GLP, *in vivo,* multiple dose, safety and pharmacokinetics. |
| **Method:** The animals were randomized into one of three groups and received four weekly intravenous (IV) doses of the test material. Animals in Group 1 (5 males and 5 females) received rhuMAb β7 Vehicle at 1.25 mL/kg. Animals in Group 2 (3 males and 3 females) and Group 3 (5 males and 5 females) received rhuMAb β7 at 5 and 25 mg/kg, respectively. Three males and 3 females from each group had terminal necropsies 1 week after the last dose. Two males and 2 females from the groups administered vehicle or 25 mg/kg rhuMAb β7 were followed for a recovery period of approximately 23 weeks before a recovery necropsy. Serum samples were analyzed by quantitative ELISA to measure total rhuMAb β7 levels and by a bridging electrochemiluminescence assay to determine the level of antibody response against rhuMAb β7. Total occupied and unoccupied β7-expressing CD4⁺ and CD8⁺ T-cell subsets, occupancy of β7 on CD4⁺ and CD8⁺ T-cell subsets, and lymphocyte subsets (total B cells, T cells, and natural killer cells) were analyzed by flow cytometry. Pharmacokinetic data underwent non-compartmental and two-compartment analysis. |
| **Results:** Overall, the serum concentration-time profile of rhuMAb β7 following four weekly IV doses was biphasic, with a fast initial distribution phase followed by a slower elimination phase. Trough serum concentrations increased after the second dose except when anti-therapeutic antibodies (ATAs) were formed. The dose-normalized area under the serum concentration-time profile from Day 0 to Day 7 (AUC₀₋₇/dose)following the first dose was similar for the 5 mg/kg and 25 mg/kg dose groups, suggesting that rhuMAb β7 exhibits dose-proportional pharmacokinetics in cynomolgus monkeys at the dose range studied. For the 25 mg/kg dose group, the AUC₀₋₇ and maximum serum concentration (*C*ₘₐₓ) increased approximately 2-fold after the last dose, compared with the first dose. Both of these findings suggested that there was accumulation of rhuMAb β7 following four weekly IV doses of 25 mg/kg to cynomolgus monkeys; however, such accumulation is expected for an antibody with a long half-life at these doses. Compartmental analysis of the data from the 25 mg/kg recovery animals estimated a slow clearance of 2.93 mL/day/kg and a relatively long terminal half-life of 14.5 days. However, 3 of the 4 monkeys had ATAs that probably contributed to rhuMAb β7clearance. Integrin β7-preceptor occupancy assays suggested that β7 receptors on peripheral-blood T-cell subsets were fully saturated following the first of four weekly doses of 5 or 25 mg/kg rhuMAb β7 to cynomolgus monkeys. The detection of available β7 correlated with a decrease in serum concentrations of rhuMAb β7 and suggested that a serum concentration above approximately 1-10 µg/mL will maintain occupancy of β7 receptors on peripheral-blood T cells. CD45RA⁻β7^{high} T cells (phenotypically similar to gut-homing T cells in humans and mice) increased approximately 5-fold |
| over baseline levels following four weekly doses of 25 mg/kg to cynomolgus monkeys. Likewise, CD45RA⁺β7^{intermediate} T cells (phenotypically similar to human naïve T cells) also increased following four weekly doses of 25 mg/kg (approximately 2.5-fold over baseline levels). These findings are consistent with the mechanism of action of rhuMAb β7-namely, the inhibition of trafficking of β7-positive lymphocytes to the gut through inhibition of α4β7 binding to its ligands, which then leads to the accumulation of these cells in the peripheral circulation. |

## Claims

1. A method of determining the dosing of an integrin beta7 antagonist for treatment of a gastrointestinal inflammatory disorder in a patient, wherein said integrin beta7 antagonist is an anti-beta7 antibody, the method comprising adjusting the dose of the integrin beta7 antagonist based on a comparison of the amount of a biomarker in a sample obtained from the patient after or during treatment with a dose or dosing regimen of the integrin beta7 antagonist, to an amount of the biomarker in a sample obtained from the patient before the treatment, wherein a change in the amount of the biomarker after or during the treatment, as compared to before the treatment, is indicative of the efficacy of or responsiveness to the dose or dosing regimen of the integrin beta7 antagonist for treatment of the gastrointestinal disorder in the patient, and wherein the biomarker is selected from a group consisting of gut-homing lymphocytes in the patient's peripheral blood, integrin beta7 antagonist occupancy on gut-homing lymphocytes, and beta7 integrin receptors on gut-homing lymphocytes, wherein the gut-homing lymphocytes are a distinctive subgroup of lymphocytes identified as CD45RA⁻β7^{high}CD4⁺.

2. The method of claim 1, wherein said gastrointestinal inflammatory disorder is an inflammatory bowel disease.

3. The method of claim 2, wherein said inflammatory bowel disease is Crohn's disease (CD) or ulcerative colitis (UC).

4. The method of Claim 3, wherein said patient is a human.

5. The method of claim 2, wherein said integrin beta7 antagonist is a monoclonal anti-beta7 antibody.

6. The method of Claim 5, wherein said antibody is a chimeric, human or humanized antibody.

7. The method of Claim 1, wherein said antibody is an antibody fragment.

8. The method of Claim 5, wherein the antibody comprises six hypervariable regions (HVRs) selected from the group consisting of HVR-L1, HVR-L2, HVR-L3, HVR-H1, HVR-H2, and HVR-H3, wherein:
(i) HVR-L1 comprises amino acid sequence A1-A11, wherein A1-A11 is RASESVDTYLH (SEQ ID NO: 1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:67, or XaaYASQSIS (SEQ ID NO:68, where Xaa represents any amino acid) or a variant of SEQ ID NOs:2, 67 or 68 wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 wherein amino acid C8 is selected from the group consisting of N, V, W, Y, R, S, T, A, F, H, I L, M, and Y;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO:66); or comprises amino acid sequence F1-F11, wherein F1-F11 is AMTGSSGYFDF (SEQ ID NO:63), ARTGSSGYFDF (SEQ ID NO:64), or AQTGSSGYFDF (SEQ ID NO:65), or a variant of SEQ ID NOs:6, 63, 64, 65, or 66 wherein amino acid F2 is R, M, A, E, G, Q, S, and/or amino acid F11 is selected from the group consisting of F and Y.

9. The method of Claim 8, wherein said antibody comprises three heavy chain hypervariable region (HVR-H1-H3) sequences and three light chain hypervariable region (HVR-L1-L3) sequences, wherein
(i)HVR-L1 comprises SEQ ID NO: 7, SEQ ID NO:8 or SEQ ID NO:9;
(ii)HVR-L2 comprises SEQ ID NO: 2;
(iii)HVR-L3 comprises SEQ ID NO:3;
(iv)HVR-H1 comprises SEQ ID NO:4;
(v)HVR-H2 comprises SEQ ID NO:5; and
(vi)HVR-H3 comprises SEQ ID NO:6 or SEQ NO:63 or SEQ ID NO:64 or SEQ ID NO:66.

10. A method of Claim 1 wherein said sample is a peripheral blood sample of said patient.

## Patentansprüche

1. Verfahren zur Bestimmung der Dosierung eines Integrin-beta7-Antagonisten zur Behandlung einer entzündlichen Magen-Darm-Störung in einem Patienten, wobei der Integrin-beta7-Antagonist ein anti-beta7-Antikörper ist, wobei das Verfahren das Einstellen der Dosis des Integrin-beta7-Antagonisten basierend auf einem Vergleich der Menge eines Biomarkers in einer Probe umfasst, wobei die Probe von dem Patienten nach oder während der Behandlung mit einer Dosis oder einer Dosierungsanleitung des Integrin-beta7-Antagonisten erhalten wird, mit einer Menge des Biomarkers in einer Probe, die von dem Patienten vor der Behandlung erhalten wird, wobei eine Änderung der Menge des Biomarkers nach oder während der Behandlung im Vergleich zu vor der Behandlung die Wirksamkeit oder das Ansprechen auf die Dosis oder die Dosierungsanleitung des Integrin-beta7-Antagonisten bei der Behandlung der Magen-Darm-Störung in dem Patienten anzeigt, und wobei der Biomarker ausgewählt ist aus der Gruppe bestehend aus Darm-homing-Lymphozyten ("gut-homing lymphocytes") im peripheren Blut des Patienten, Integrin-beta7-Antagonisten, die auf Darm-homing-Lymphozyten besetzt sind, und beta7-Integrin-Rezeptoren auf Darm-homing-Lymphozyten, wobei die Darm-homing-Lymphozyten eine unterscheidbare Untergruppe der Lymphozyten sind, die als CD45RA⁻β7^{high}CD4⁺ bezeichnet werden.

2. Verfahren nach Anspruch 1, wobei die Magen-Darm-Störung eine entzündliche Darmerkrankung ist.

3. Verfahren nach Anspruch 2, wobei die entzündliche Darmerkrankung Morbus Crohn (CD) oder Colitis ulcerosa (UC) ist.

4. Verfahren nach Anspruch 3, wobei der Patient ein Mensch ist.

5. Verfahren nach Anspruch 2, wobei der Integrin-beta7-Antagonist ein monoclonaler anti-beta7 Antikörper ist.

6. Verfahren nach Anspruch 5, wobei der Antikörper ein chimärer, humaner oder humanisierter Antikörper ist.

7. Verfahren nach Anspruch 1, wobei der Antikörper ein Antikörperfragment ist.

8. Verfahren nach Anspruch 5, wobei der Antikörper sechs hypervariable Regionen (HVRs) umfasst, die ausgewählt sind aus der Gruppe bestehend aus HVR-L1, HVR-L2, HVR-L3, HVR-H1, HVR-H2 und HVR-H3, wobei
(i) HVR-L1 die Aminosäuresequenz A1-A11 umfasst, wobei A1-A11 RASESVDTYLH (SEQ ID NO:1); RASESVDSLLH (SEQ ID NO:7), RASESVDTLLH (SEQ ID NO:8) oder RASESVDDLLH (SEQ ID NO:9) oder eine Variante von SEQ ID NOs:1, 7, 8 oder 9 ist, wobei Aminosäure A2 ausgewählt ist aus der Gruppe bestehend aus A, G, S, T und V, und/oder Aminosäure A3 ausgewählt ist aus der Gruppe bestehend aus S, G, I, K, N, P, Q, R und T, und/oder A4 ausgewählt ist aus der Gruppe bestehend aus E, V, Q, A, D, G, H, I, K, L, N und R, und/oder Aminosäure A5 ausgewählt ist aus der Gruppe bestehend aus S, Y, A, D, G, H, I, K, N, P, R, T und V, und/oder Aminosäure A6 ausgewählt ist aus der Gruppe bestehend aus V, R, I, A, G, K, L, M und Q, und/oder Aminosäure A7 ausgewählt ist aus der Gruppe bestehend aus D, V, S, A, E, G, H, I, K, L, N, P, S und T, und/oder Aminosäure A8 ausgewählt ist aus der Gruppe bestehend aus D, G, N, E, T, P und S, und/oder Aminosäure A9 ausgewählt ist aus der Gruppe bestehend aus L, Y, I und M und/oder Aminosäure A10 ausgewählt ist aus der Gruppe bestehend aus L, A, I, M und V, und/oder Aminosäure A11 ausgewählt ist aus der Gruppe bestehend aus H, Y, F und S;
(ii) HVR-L2 die Aminosäuresequenz B1-B8 umfasst, wobei B1-B8 KYASQSIS (SEQ ID NO:2), RYASQSIS (SEQ ID NO:67) oder XaaYASQSIS (SEQ ID NO:68, wobei Xaa eine beliebige Aminosäure darstellt) oder eine Variante von SEQ ID NOs: 2, 67 oder 68 ist, wobei Aminosäure B1 ausgewählt ist aus der Gruppe bestehend aus K, R, N, V, A, F, Q, H, P, I, L, Y und Xaa (wobei Xaa eine beliebige Aminosäure darstellt), und/oder Aminosäure B4 ausgewählt ist aus der Gruppe bestehend aus S und D, und/oder Aminosäure B5 ausgewählt ist aus der Gruppe bestehend aus Q und S, und/oder Aminosäure B6 ausgewählt ist aus der Gruppe bestehend aus S, D, L und R, und/oder Aminosäure B7 ausgewählt ist aus der Gruppe bestehend aus I, V, E und K;
(iii) HVR-L3 die Aminosäuresequenz C1-C9 umfasst, wobei C1-C9 QQGNSLPNT (SEQ ID NO:3) oder eine Variante von SEQ ID NO:3 ist, wobei Aminosäure C8 ausgewählt ist aus der Gruppe bestehend aus N, V, W, Y, R, S, T, A, F, H, I, L, M und Y;
(iv) HVR-H1 die Aminosäuresequenz D1-D10 umfasst, wobei D1-D10 GFFITNNYWG (SEQ ID NO:4) ist;
(v) HVR-H2 die Aminosäuresequenz E1-E17 umfasst, wobei E1-E17 GYISYSGSTSYNPSLKS (SEQ ID NO:5) oder eine Variante von SEQ ID NO:5 ist, wobei Aminosäure E2 ausgewählt ist aus der Gruppe bestehend aus Y, F, V und D, und/oder Aminosäure E6 ausgewählt ist aus der Gruppe bestehend aus S und G, und/oder Aminosäure E10 ausgewählt ist aus der Gruppe bestehend aus S und Y, und/oder Aminosäure E12 ausgewählt ist aus der Gruppe bestehend aus N, T, A und D, und/oder Aminosäure 13 ausgewählt ist aus der Gruppe bestehend aus P, H, D und A, und/oder Aminosäure E15 ausgewählt ist aus der Gruppe bestehend aus L und V, und/oder Aminosäure E17 ausgewählt ist aus der Gruppe bestehend aus S und G; und
(vi) HVR-H3 die Aminosäuresequenz F2-F11 umfasst, wobei F2-F11 MTGSSGYFDF (SEQ ID NO:6) oder RTGSSGYFDF (SEQ ID NO:66) ist; oder Aminosäuresequenz F1-F11 umfasst, wobei F1-F11 AMTGSSGYFDF (SEQ ID NO:63), ARTGSSGYFDF (SEQ ID NO:64), oder AQTGSSGYFDF (SEQ ID NO:65) oder eine Variante von SEQ ID NOs: 6, 63, 64, 65 oder 66 umfasst, wobei Aminosäure F2 R, M, A, E, G, Q, S ist, und/oder Aminosäure F11 ausgewählt ist aus der Gruppe bestehend aus F und Y.

9. Verfahren nach Anspruch 8, wobei der Antikörper drei Sequenzen der hypervariablen Region der schweren Kette (HVR-H1-H3) und drei Sequenzen der hypervariablen Region der leichten Kette (HVR-L1-L3) umfasst, wobei
(i) HVR-L1 SEQ ID NO:7, SEQ ID NO:8 oder SEQ ID NO:9 umfasst;
(ii) HVR-L2 SEQ ID NO:2 umfasst;
(iii) HVR-L3 SEQ ID NO:3 umfasst;
(iv) HVR-H1 SEQ ID NO:4 umfasst;
(v) HVR-H2 SEQ ID NO:5 umfasst; und
(vi) HVR-H3 SEQ ID NO:6 oder SEQ ID NO:63 oder SEQ ID NO:64 oder SEQ ID NO:66 umfasst.

10. Verfahren nach Anspruch 1, wobei die Probe eine Probe des peripheren Bluts des Patienten ist.

## Revendications

1. Méthode de détermination du dosage d'un antagoniste d'intégrine bêta-7 pour le traitement d'un trouble gastro-intestinal inflammatoire chez un patient, dans laquelle ledit antagoniste d'intégrine bêta-7 est un anticorps anti-bêta-7, la méthode comprenant l'ajustement de la dose de l'antagoniste d'intégrine bêta-7 basé sur une comparaison de la quantité d'un biomarqueur dans un échantillon provenant du patient après ou pendant le traitement avec une dose ou selon une posologie de l'antagoniste d'intégrine bêta-7, à une quantité du biomarqueur dans un échantillon provenant du patient avant le traitement, une variation de la quantité du biomarqueur après ou pendant le traitement, comparée à avant le traitement, indiquant l'efficacité de la dose ou de la posologie de l'antagoniste d'intégrine bêta-7 ou la réactivité à ladite dose ou posologie pour le traitement d'un trouble gastro-intestinal chez le patient, et dans laquelle le biomarqueur est choisi dans un groupe constitué par les lymphocytes de domiciliation intestinale dans le sang périphérique du patient, l'occupation de l'antagoniste d'intégrine bêta-7 sur les lymphocytes de domiciliation intestinale, et les récepteurs d'intégrine bêta-7 sur les lymphocytes de domiciliation intestinale, lesdits lymphocytes de domiciliation intestinale étant un sous-groupe distinct de lymphocytes identifiés comme étant des CD45RA⁻β7^{high}CD4⁺.

2. Méthode selon la revendication 1, dans laquelle ledit trouble gastro-intestinal inflammatoire est une maladie inflammatoire de l'intestin.

3. Méthode selon la revendication 2, dans laquelle ladite maladie inflammatoire de l'intestin est la maladie de Crohn (CD) ou la rectocolite hémorragique (UC).

4. Méthode selon la revendication 3, dans laquelle ledit patient est l'homme.

5. Méthode selon la revendication 2, dans laquelle ledit antagoniste d'intégrine bêta-7 est un anticorps monoclonal anti-bêta-7.

6. Méthode selon la revendication 5, dans laquelle ledit anticorps est un anticorps chimère, humain ou humanisé.

7. Méthode selon la revendication 1, dans laquelle ledit anticorps est un fragment d'anticorps.

8. Méthode selon la revendication 5, dans laquelle l'anticorps comprend six régions hypervariables (HVR) choisies dans le groupe constitué par HVR-L1, HVR-L2, HVR-L3, HVR-H1, HVR-H2, et HVR-H3, où :
(i) HVR-L1 comprend la séquence d'acides aminés A1-A11, dans laquelle A1-A11 est RASESVDTYLH (SEQ ID N° 1) ; RASESVDSLLH (SEQ ID N° 7), RASESVDTLLH (SEQ ID N° 8), ou RASESVDDLLH (SEQ ID N° 9) ou un variant des SEQ ID N° 1, 7, 8 ou 9 dans lequel l'acide aminé A2 est choisi dans le groupe constitué par A, G, S, T, et V et/ou l'acide aminé A3 est choisi dans le groupe constitué par S, G, I, K, N, P, Q, R, et T, et/ou l'acide aminé A4 est choisi dans le groupe constitué par E, V, Q, A, D, G, H, I, K, L, N, et R, et/ou l'acide aminé A5 est choisi dans le groupe constitué par S, Y, A, D, G, H, I, K, N, P, R, T, et V, et/ou l'acide aminé A6 est choisi dans le groupe constitué par V, R, I, A, G, K, L, M, et Q, et/ou l'acide aminé A7 est choisi dans le groupe constitué par D, V, S, A, E, G, H, I, K, L, N, P, S, et T, et/ou l'acide aminé A8 est choisi dans le groupe constitué par D, G, N, E, T, P et S, et/ou l'acide aminé A9 est choisi dans le groupe constitué par L, Y, I et M, et/ou l'acide aminé A10 est choisi dans le groupe constitué par L, A, I, M, et V et/ou l'acide aminé A11 est choisi dans le groupe constitué par H, Y, F, et S ;
(ii) HVR-L2 comprend la séquence d'acides aminés B1-B8, dans laquelle B1-B8 est KYASQSIS (SEQ ID N° 2), RYASQSIS (SEQ ID N° 67, ou XaaYASQSIS (SEQ ID N° 68, où Xaa représente un acide aminé quelconque) ou un variant des SEQ ID N° 2, 67 ou 68 dans lequel l'acide aminé B1 est choisi dans le groupe constitué par K, R, N, V, A, F, Q, H, P, I, L, Y et Xaa (où Xaa représente un acide aminé quelconque), et/ou l'acide aminé B4 est choisi dans le groupe constitué par S et D, et/ou l'acide aminé B5 est choisi dans le groupe constitué par Q et S, et/ou l'acide aminé B6 est choisi dans le groupe constitué par S, D, L, et R, et/ou l'acide aminé B7 est choisi dans le groupe constitué par I, V, E, et K ;
(iii) HVR-L3 comprend la séquence d'acides aminés C1-C9, dans laquelle C1-C9 est QQGNSLPNT (SEQ ID N° 3) ou un variant de la SEQ ID N° 3 dans lequel l'acide aminé C8 est choisi dans le groupe constitué par N, V, W, Y, R, S, T, A, F, H, I, L, M, et Y ;
(iv) HVR-H1 comprend la séquence d'acides aminés D1-D10 dans laquelle D1-D10 est GFFITNNYWG (SEQ ID N° 4) ;
(v) HVR-H2 comprend la séquence d'acides aminés E1-E17, dans laquelle E1-E17 est GYISYSGSTSYNPSLKS (SEQ ID N° 5), ou un variant de la SEQ ID N° 5 dans lequel l'acide aminé E2 est choisi dans le groupe constitué par Y, F, V, et D, et/ou l'acide aminé E6 est choisi dans le groupe constitué par S et G, et/ou l'acide aminé E10 est choisi dans le groupe constitué par S et Y, et/ou l'acide aminé E12 est choisi dans le groupe constitué par N, T, A, et D, et/ou l'acide aminé 13 est choisi dans le groupe constitué par P, H, D, et A, et/ou l'acide aminé E15 est choisi dans le groupe constitué par L et V, et/ou l'acide aminé E17 est choisi dans le groupe constitué par S et G ; et
(vi) HVR-H3 comprend la séquence d'acides aminés F2-F11, dans laquelle F2-F11 est MTGSSGYFDF (SEQ ID N° 6) ou RTGSSGYFDF (SEQ ID N° 66) ; ou comprend la séquence d'acides aminés F1-F11, dans laquelle F1-F11 est AMTGSSGYFDF (SEQ ID N° 63), ARTGSSGYFDF (SEQ ID N° 64), ou AQTGSSGYFDF (SEQ ID N° 65), ou un variant des SEQ ID N° 6, 63, 64, 65, ou 66 dans lequel l'acide aminé F2 est R, M, A, E, G, Q, S, et/ou l'acide aminé F11 est choisi dans le groupe constitué par F et Y.

9. Méthode selon la revendication 8, dans laquelle ledit anticorps comprend trois séquences de régions hypervariables de chaînes lourdes (HVR-H1-H3) et trois séquences de régions hypervariables de chaînes légères (HVR-L1-L3), dans laquelle :
(i) HVR-L1 comprend la SEQ ID N° 7, la SEQ ID N° 8 ou la SEQ ID N° 9 ;
(ii) HVR-L2 comprend la SEQ ID N° 2 ;
(iii) HVR-L3 comprend la SEQ ID N° 3 ;
(iv) HVR-H1 comprend la SEQ ID N° 4 ;
(v) HVR-H2 comprend la SEQ ID N° 5 ; et
(vi) HVR-H3 comprend la SEQ ID N° 6 ou la SEQ ID N° 63 ou la SEQ ID N° 64 ou la SEQ ID N° 66.

10. Méthode selon la revendication 1, dans laquelle ledit échantillon est un échantillon de sang périphérique dudit patient.
